# EUROPEAN PATENT APPLICATION

(11) **EP 4 353 712 A1**
(43) Date of publication of application: **17.04.2024**
(21) Application number: 22306531.9
(22) Date of filing: 11.10.2022
(51) Int. Cl.: C07D 215/40, C07D 217/02, C07D 237/30, C07D 241/42, C07D 401/12, A61P 29/00, A61K 31/4709, A61K 31/4725, A61K 31/498, A61K 31/502

(54) **NEW N-HETEROARYLBENZAMIDES DERIVATIVES AS FLT3 INHIBITORS**

(71) Applicant: Biodol Therapeutics, 34830 Clapiers (FR); Institut National de la Santé et de la Recherche Médicale (INSERM), 75013 Paris (FR); Centre National de la Recherche Scientifique, 75016 Paris (FR); UNIVERSITE DE MONTPELLIER, 34090 Montpellier (FR); Université de Strasbourg, 67000 Strasbourg (FR)
(72) Inventor: The designation of the inventor has not yet been filed
(74) Representative: Cabinet Nony

(57) **Abstract**

The present invention relates to a compound of formula (I) or any of its acceptable salts (I) 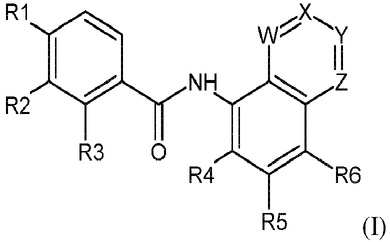 wherein W, X, Y and Z each independently represents =CH- or -N-, provided that at most two of W, X, Y and Z both represent a -N- group, R1 represents a linear or cyclic nitrogen containing (C₄-C₈)alkyl group, said group being a non aromatic (C₄-C₈)alkyl group that can be linear or cyclic comprising 4 to 8 carbon atoms that contains at least one nitrogen atom, that interrupts said alkyl chain, said group being optionally interrupted by one or two oxygen atoms and optionally substituted by a (C₁-C₄)alkyl group or by a (C₃-C₆)cycloalkyl group, R2 represents a hydrogen atom, a halogen atom, a (C₁-C₄)alkyl group, a (C₁-C₄)alkoxy group, a (C₁-C₄)fluoroalkyl group, a -CO-(C₁-C₄)alkyl group, a -CONH₂ group, a SO₂-NH₂ group or a cyano group, R3 represents a hydrogen atom, a halogen atom, a (C₁-C₄)alkyl group, a (C₁-C₄)alkoxy group, a (C₁-C₄)fluoroalkyl group, a cyano group, a (C₁-C₄)alkylsulfonyl group or a SO₂-NH₂ group, and R4, R5 and R6 independently represent a hydrogen atom, a halogen atom, a -COOH group, a (C₁-C₄)fluoroalkyl group, a (C₁-C4)alkylsulfonyl group or a (C₁-C₄)alkoxy group.

The present invention further related to a pharmaceutical composition comprising it and to its use in the prevention and/or the treatment of pain.

## Description

### FIELD OF THE INVENTION

The present invention relates to the prevention and/or treatment of pain, and more particularly provides new N-heteroarylbenzamides derivatives as FLT3-inhibitors.

### BACKGROUND OF THE INVENTION

The current treatment of chronic pain (duration > 3 months), especially neuropathic pain, is essentially symptomatic and unsatisfactory for most patients. Indeed, a number of pain medications are available on the pharmaceutical market, most of which have been initially marketed for other indications, e.g. epilepsy and depression. These medications are however only partially efficacious against neuropathic pain: only a limited number of patients achieve a 50% reduction of their pain symptoms and it is still today unfortunately observed that some neuropathic pain syndromes are completely refractory to these medications, thus affecting considerably the quality of life of these patients. Moreover, the existing medications often generate a large variety of adverse effects that limit their use in patients: they can elicit dizziness, somnolence, tiredness, constipation, dry mouth, nausea, vomiting, and weight gain.

Peri-operative and post-operative pain is another pain condition that is unsatisfactorily treated. Post-operative pain essentially treated with opioids like morphine, which remain the unsurpassed treatment for the management of acute pain. However, their use for chronic pain is seriously limited by undesirable side-effects such as constipation, nausea, vomiting, sedation and respiratory depression and there is a risk of abuse, addiction and number of opioid-associated deaths, as well. Most importantly, acute or chronic administration of an opioid can also produce tolerance to its analgesic effects, which requires increasing the dose of opioid and exacerbation of the aforementioned side-effects, and pain hypersensitivity referred to as opioid-induced hyperalgesia (OIH) and latent pain sensitization, which cannot be overcome by increasing the dose of the opioid and leaves the patient without adequate treatment of its pain and a considerable degradation of his quality of life.

Thus, there is a need to find new strategies for treating pain.

One of the new strategies to combat pain consists in inhibiting FLT3 (Rivat et al. Nat. Commun., 2018, 9, 1042)

Indeed, WO2011/083124 discloses the use of an FLT3 receptor antagonist for the treatment of pain disorders. FLT3 (fms-related tyrosine kinase 3) is a member of the class III receptor tyrosine kinase (RTK) family, which contains an extracellular domain (ECD) interacting with its ligand FL, and an intracellular domain containing a kinase domain responsible for auto-phosphorylation. Upon activation, FLT3 dimerizes and auto-phosphorylates tyrosine residues present in its intracellular domain. FLT3 receptor antagonist according to WO2011/083124 encompasses several types of molecules that inhibit FLT3 activation, e.g. small organic molecules acting at either the intra- or extracellular FLT3 sites, antibodies against FLT3 or its ligand FL or inhibitors of FLT3 expression. WO2011/083124 cites a large list of pain disorders that encompasses acute pain, chronic pain, neuropathic pain, inflammatory pain, low back pain, post-operative pain, cancer pain, vascular headache such as migraine, fibromyalgia, hyperalgesia such as mechanical and thermal hyperalgesia, allodynia such as thermal and mechanical allodynia, peripheral sensitization of pain mechanisms and central sensitization of pain mechanisms.

More recently some FLT3 receptor antagonists, which inhibit the interaction between FLT3 and FL, were described in WO2016/016370**.** According to WO2016/016370, antagonists inhibiting the interaction between FLT3 and FL can be used to treat various pain disorders, including acute pain, chronic pain, neuropathic pain, inflammatory pain, iatrogenic pain including cancer pain, infectious pain including herpetic pain visceral pain, central pain, dysfunctioning pain including fibromyalgia, nociceptive pain including postsurgical pain, and mixed pain types involving the viscera, gastrointestinal tract, cranial structures, musculoskeletal system, spine, urogenital system, cardiovascular system and CNS, including cancer pain, back and orofacial pain.

Even more recently, WO2018/211018 describes the use of FLT3 antagonists in combination with opioids. Such combinations enhance opioid analgesia, reduce tolerance to opioid analgesia and inhibit opioid-induced hyperalgesia. Thus, FLT3 antagonists can be used to improve analgesia in pain disorders requiring treatment with opioids, such as inflammatory and post-operative pain.

### SUMMARY OF THE INVENTION

It has now been found that compounds as defined in formula (I) hereinafter are useful in the prevention and/or treatment of pain conditions or disorders.

Herein is therefore provided a compound of formula (I) as defined herein after or any of its acceptable salts, a process for manufacturing it or a pharmaceutical composition comprising a compound of formula (I) as defined below, alone or in combination with an opioid. Said opioid may be selected among alfentanil, allylprodine, alphaprodine, anileridine, benzylmorphine, bezitramide, buprenorphine, butorphanol, clonitazene, codeine, cyclazocine, desomorphine, dextromoramide, dextropropoxyphene, dezocine,diampromide, diamorphone, dihydrocodeine, dihydromorphine, dimenoxadol, dimepheptanol, dimethylthiambutene, dioxaphetylbuturate, dipipanone, eptazocine, ethoheptazine, ethylmethylthiambutene. ethylmorphine, etonitazene fentanyl, heroin, hydrocodone, hydromorphone, hydroxypethideine, isomethadone, ketobemidone, levallorphan, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propiram, propoxyphene, remifentanyl, sufentonil, tapentadol, tilidine and tramadol.

The present invention therefore relates to compounds of formula (I), as defined below or any of its acceptable salts, alone or in combination with an opioid, as defined hereinabove, for use in the prevention and/or treatment of pain.

In particular, the prevention and/or treatment of pain extends to the conditions related thereto.

The present invention further relates to the use of a compound of formula (I) as defined below or any of its acceptable salts, alone or in combination with an opioid, as defined hereinabove, for the manufacture of a medicament for the prevention and/or treatment of pain.

The present invention further relates to a method of prevention and/or treatment of pain comprising a step of administering to a patient in need thereof a compound of formula (I) as defined below or any of its acceptable salts, alone or in combination with an opioid, as defined hereinabove, or of a pharmaceutical composition comprising it.

### DEFINITIONS

As used herein, the term "patient" refers to either an animal, such as a valuable animal for breeding, company or preservation purposes, or preferably a human or a human child, which is afflicted with, or has the potential to be afflicted with, one or more diseases and conditions described herein.

In particular, as used in the present application, the term "patient" refers to a mammal such as a rodent, cat, dog, primate or human, preferably said patient is a human .

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means reversing, alleviating, inhibiting the progress of, or preventing pain condition as described herein, in particular in the paragraph "PAIN CONDITIONS".

As used herein, an "effective amount" refers to an amount of a compound of the present invention which is effective in preventing, reducing, eliminating, treating or controlling the pain. The term "controlling" is intended to refer to all processes wherein there may be a slowing, interrupting, arresting, or stopping of the progression of the pain, but does not necessarily indicate a total elimination of all manifestation of pain.

As used herein, « preventing » also encompasses « reducing the likelihood of occurrence » or « reducing the likelihood of reoccurrence ».

The term "prophylaxis-effective amount" refers to a concentration of compound of this invention that is effective in inhibiting, preventing, decreasing the likelihood of a pain condition.

Likewise, the term "treatment-effective amount" refers to a concentration of compound that is effective in treating a pain condition as described herein.

The term "preventing", as used herein, means reducing the risk of onset or slowing the occurrence of a given phenomenon, namely in the present invention, a pain condition as described herein.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, the term *"FLT3"* or *"FLT3 receptor"* (Fms-related tyrosine kinase 3), also known as CD135, Ly72, Flk-2, Flt-3 or B230315G04, are used interchangeably and have their general meaning in the art. The FLT3 receptor can be from any animal species, but typically is a mammalian (e.g., human and non-human primate) FLT3 receptor, particularly a human FLT3 receptor. The naturally occurring human *Flt3* gene has a nucleotide sequence as shown in Genbank Accession number NM_004119.2 and the naturally occurring human FLT3 protein has an aminoacid sequence as shown in Genbank Accession number NP_004110.2. The murine nucleotide and amino acid sequences have also been described (Genbank Accession numbers NM_010229.2 and NP_034359.2).

As used herein, the term the terms "FL" or "FLT3-Ligand" are used interchangeably and have their general meaning in the art. They refer to the cytokine which is a natural ligand of the FLT3 receptor. FL can be from any source, but typically is a mammalian (e.g., human and non-human primate) FL, particularly a human FL.

The term *"FLT3 inhibitor"* refers to any compound which inhibits or downregulates the biological activity associated with activation of the FLT3 receptor by FL in the subject, including any of the downstream biological effects otherwise resulting from the binding to FLT3 receptor with FL. Such a FLT3 inhibitor can act by occupying the FL-binding site or a portion thereof, or a nearby cavity (allosteric site), thereby making FLT3 receptor inaccessible to its natural ligand, FL, so that its normal biological activity is prevented or reduced. The term FLT3 receptor inhibitor includes also any agent able to interact with FL, the natural ligand of FLT3.

The compound of formula (I) according to the present invention are FLT3 inhibitors, and more particularly are inhibitor of the interaction between FLT3 and FL. Said compounds that inhibit the interaction between FLT3 and FL encompass those compounds that bind either to the FLT3 receptor, FL or both, provided that the binding of the said compounds of interest prevents the interaction between FLT3 receptor and FL.

As used herein, the term *"analgesic effect"* refers to a clinical effect which results from the use of a substance that produces analgesia. The term *"analgesia"* refers to loss of sensitivity to pain without loss of consciousness.

The terms "between... and..." and "ranging from... to..." should be understood as being inclusive of the limits, unless otherwise specified.

### DETAILED DESCRIPTION OF THE INVENTION

The inventors have in particular found that the compounds of formula (I) are endowed with an inhibition activity of FLT3.

Herein is provided a compound of formula (I) or any of its acceptable salts (I) 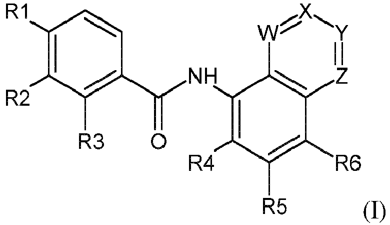 wherein
W, X, Y and Z each independently represents =CH- or -N-, provided that at most two of W, X, Y and Z both represent a -N- group,
R1 represents a linear or cyclic nitrogen containing (C₄-C₈)alkyl group, said group being a non aromatic (C₄-C₈)alkyl group that can be linear or cyclic comprising 4 to 8 carbon atoms that contains at least one nitrogen atom, that interrupts said alkyl chain, said group being optionally interrupted by one or two oxygen atoms and optionally substituted by a (C₁-C₄)alkyl group or by a (C₃-C₆)cycloalkyl group, wherein R1 comprises at least one primary, secondary or tertiary amine, in particular one or two secondary or tertiary amine, R2 represents a hydrogen atom, a halogen atom, a (C₁-C₄)alkyl group, a (C₁-C₄)alkoxy group, a (C₁-C₄)fluoroalkyl group, a -CO-(C₁-C₄)alkyl group, a -CONH₂ group, a SO₂-NH₂ group or a cyano group,
R3 represents a hydrogen atom, a halogen atom, a (C₁-C₄)alkyl group, a (C₁-C₄)alkoxy group, a (C₁-C₄)fluoroalkyl group, a cyano group, a (C₁-C₄)alkylsulfonyl group or a SO₂-NH₂ group, and
R4, R5 and R6 independently represent a hydrogen atom, a halogen atom, a -COOH group, a (C₁-C₄)fluoroalkyl group, a (C₁-C₄)alkylsulfonyl group or a (C₁-C₄)alkoxy group.

Herein is further provided a pharmaceutical composition comprising at least one new compound as defined above or any of its pharmaceutically acceptable salts, or at least any of compounds (1) to (57) as defined above or any of its pharmaceutically acceptable salts, and also at least one pharmaceutically acceptable excipient.

Herein is further provided a pharmaceutical composition comprising at least one new compound as defined above or any of its pharmaceutically acceptable salts, or at least any of compounds (1) to (57) as defined above or any of its pharmaceutically acceptable salts, an opioid and also at least one pharmaceutically acceptable excipient, wherein said opioid may be selected among alfentanil, allylprodine, alphaprodine, anileridine, benzylmorphine, bezitramide, buprenorphine, butorphanol, clonitazene, codeine, cyclazocine, desomorphine, dextromoramide, dextropropoxyphene, dezocine,diampromide, diamorphone, dihydrocodeine, dihydromorphine, dimenoxadol, dimepheptanol, dimethylthiambutene, dioxaphetylbuturate, dipipanone, eptazocine, ethoheptazine, ethylmethylthiambutene. ethylmorphine, etonitazene fentanyl, heroin, hydrocodone, hydromorphone, hydroxypethideine, isomethadone, ketobemidone, levallorphan, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propiram, propoxyphene, remifentanyl, sufentonil, tapentadol, tilidine and tramadol.

Herein is further provided a compound of formula (I) as defined above or any of its pharmaceutically acceptable salts, and any of compounds (1) to (57) as defined herein after or any of its pharmaceutically acceptable salts, alone or in combination with an opioid, in particular as defined hereinabove, for use as a medicament.

Herein is further provided a compound of formula (I) as defined above or any of its pharmaceutically acceptable salts, and any of compounds (1) to (57) as defined herein after or any of its pharmaceutically acceptable salts, alone or in combination with an opioid, in particular as defined hereinabove, for use in the prevention and/or treatment of pain.

The compounds of the invention may exist in the form of free bases or of addition salts with pharmaceutically acceptable acids.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include benzenesulfonate, ethanesulfonate, fumarate, methanesulfonate, p-toluenesulfonate and the like.

Suitable physiologically acceptable acid addition salts of compounds of formula (I) include hydrobromide, tartrate, citrate, trifluoroacetate, ascorbate, hydrochloride, triflate, maleate, mesylate, formate, acetate and fumarate.

The compounds of formula (I) and or salts thereof may form solvates or hydrates and the invention includes all such solvates and hydrates.

The terms "hydrates" and "solvates" simply mean that the compounds (I) according to the invention can be in the form of a hydrate or solvate, *i*.*e*. combined or associated with one or more water or solvent molecules. This is only a chemical characteristic of such compounds, which can be applied for all organic compounds of this type.

The compounds of formula (I) and any of compounds (1) to (57) as defined hereinafer can comprise one or more asymmetric carbon atoms. They can thus exist in the form of enantiomers or of diastereoisomers. These enantiomers, diastereoisomers and their mixtures, including the racemic mixtures, are encompassed within the scope of the present invention

The compounds of formula (I) and any of compounds (1) to (57) as defined herein after may be under amorphous or crystalline forms, which are encompassed within the scope of the present invention as well.

In the context of the present invention, the term:
- "halogen" is understood to mean chlorine, fluorine, bromine, or iodine, and in particular denotes chlorine, fluorine or bromine,
- "(C₁-Cₓ)alkyl" as used herein respectively refers to C₁-Cₓ normal, secondary or tertiary saturated hydrocarbon, in particular a (C₁-C₃)alkyl or a (C₁-C₅)alkyl. Examples are, but are not limited to, methyl, ethyl, 1-propyl, 2-propyl, butyl, pentyl,
- "cycloalkyl group" as used herein refers to a monocyclic alkyl group comprising, unless otherwise mentioned, from 3 to 7 carbon atoms, saturated or partially unsaturated and unsubstituted or substituted. By way of examples, mention may be made of, but not limited to: cyclopropyl, cyclobutyl, cyclopentyl, cyclobutenyl, cyclopentenyl, cyclohexyl, cyclohexenyl, cycloheptyl, cycloheptenyl, groups and the like, in particular a cyclopentyl, a cyclohexyl, a cycloheptyl, a cycloheptenyl, or a cyclohexenyl;
- "fluoroalkyl group" as used herein refers to an alkyl group as previously defined where the alkyl group is substituted with at least one fluorine atom. In other terms, at least one hydrogen atom of the alkyl group is replaced by a fluorine atom. By way of example, mention may be made of CH₂F, CHF₂, CH₂CHF₂, -CH₂CH₂F and the like. When all the hydrogen atoms belonging to the alkyl group are replaced by fluorine atoms, the fluoroalkyl group can be named perfluoroalkyl group. By way of example, mention may be made of trifluoromethyl group or trifluoroethyl group and the like;
- "alkoxy group" as used herein refers to an -O-alkyl group where the alkyl group is as previously defined. By way of examples, mention may be made of, but not limited to: methoxy, ethoxy, propoxy, isopropoxy, linear, secondary or tertiary butoxy, isobutoxy, pentoxy or hexoxy groups, and the like;
- "(C₁-C₄)alkylsulfonyl group" as used herein refers to a -SO₂-alkyl group where the alkyl group is as previously defined. By way of examples, mention may be made of, but not limited to -SO₂CH₃, -SO₂CH₂CH₃ and the like;
- "linear or cyclic nitrogen containing (C₄-C₈)alkyl group" means a non-aromatic (C₄-C₈)alkyl group that can be linear or cyclic comprising 4 to 8 carbon atoms that contains at least one nitrogen atom, in particular one or two nitrogen atoms, that interrupts said alkyl chain. When cyclic, it can be a monocyclic group, a monocyclic group linked to the rest of the structure by a (C₁-C₃)alkylene group, a bicyclic group, a bridged bicyclic group, a spiro bicyclic group or a fused bicyclic group. Said group may be interrupted by one or two oxygen atoms and optionally substituted by a (C₁-C₄)alkyl group or by a (C₃-C₆)cycloalkyl group. By way of example, mention may be made of (dimethylamino)ethoxy, 1-methylpiperidinyl, 1-methylpyrrolidinyl, piperazinyl, 4-methylpiperazinyl, 4-isopropylpiperazinyl, 4-cyclobutylpiperazinyl, 4-methyl-1,4-diazepane, 1-amino-4-methylpiperidine, 3-hydroxymethyl-1-methylpyrrolidine, octahydropyrrolo[3,4-c]pyrrolyl, diazaspiro[3.5]nonanyl, 2-methyl-2,5-diazabicyclo[2.2.1]heptane and 7-methyl-2,7-diazaspiro[3.5]nonane
- "protonable amine" as used herein is meant, a sp3 carbon atom-bound amine which is likely protonated at pH 7.4, including a primary, secondary or tertiary aliphatic amine, in particular a secondary or tertiary aliphatic amine.

According to one embodiment, a compound of formula (I) as defined above or any of its pharmaceutically acceptable salt, is provided, wherein W is a -N= and X, Y and Z are =CH- or Y is a -N= and W, X and Z are =CH- or X is a -N= and W, Y and Z are =CH- or Z is a -N= and W, X and Y are =CH- or W and Z are -N= and X and Y are =CH- or X and Y are =-N and W and Z are =CH-.

According to one embodiment, a compound of formula (I) as defined above or any of its pharmaceutically acceptable salt, is provided, wherein R1 is selected from, 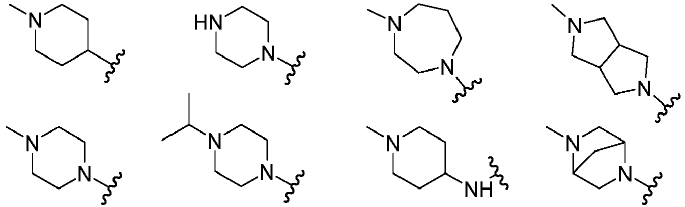 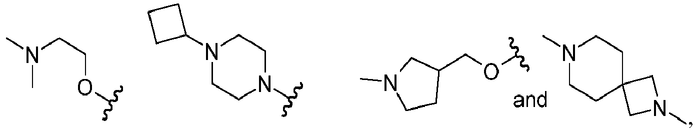 and is particular selected from 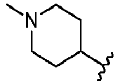 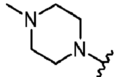 and

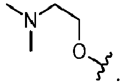

According to one embodiment, a compound of formula (I) as defined above or any of its pharmaceutically acceptable salt, is provided, wherein R2 represents a hydrogen atom, a halogen atom, a (C₁-C₄)alkyl group, a (C₁-C₄)alkoxy group, a (C₁-C₄)fluoroalkyl group or a cyano group, and in particular a hydrogen atom, a fluorine, a methyl group, a methoxy group, a trifluoro methyl group or a cyano group.

According to one embodiment, a compound of formula (I) as defined above or any of its pharmaceutically acceptable salt, is provided, wherein R3 represents a hydrogen atom, a halogen atom, a (C₁-C₄)alkyl group, a (C₁-C₄)alkoxy group, a (C₁-C₄)fluoroalkyl group or a (C₁-C₄)alkylsulfonyl group, and in particular a hydrogen atom, a fluorine, a methyl group, a methoxy group, a trifluoromethyl group or a SO₂-CH₃ group.

According to one embodiment, a compound of formula (I) as defined above or any of its pharmaceutically acceptable salt, is provided, wherein R4, R5 and R6 independently represent a hydrogen atom, a halogen atom, a (C₁-C₄)fluoroalkyl group or a (C₁-C₄)alkoxy group, and in particular a hydrogen atom, a fluorine or chlorine atom, a trifluoromethyl group or a methoxy group.

According to one embodiment, a compound of formula (I) as defined above or any of its pharmaceutically acceptable salt, is provided, wherein
R2 represents a hydrogen atom, a halogen atom, a (C₁-C₄)alkyl group, a (C₁-C₄)alkoxy group, a (C₁-C₄)fluoroalkyl group, a -CO-(C₁-C₄)alkyl group or a cyano group, in particular a hydrogen atom, a halogen atom, a (C₁-C₄)alkyl group, a (C₁-C₄)alkoxy group, a (C₁-C₄)fluoroalkyl group or a cyano group,
R3 represents a hydrogen atom, a halogen atom, a (C₁-C₄)alkyl group, a (C₁-C₄)alkoxy group, a (C₁-C₄)fluoroalkyl group, a cyano group or a (C₁-C₄)alkylsulfonyl group, in particular a hydrogen atom, a halogen atom, a (C₁-C₄)alkyl group, a (C₁-C₄)alkoxy group, a (C₁-C₄)fluoroalkyl group or a (C₁-C₄)alkylsulfonyl group,
R4, R5 and R6 independently represent a hydrogen atom, a halogen atom, a (C₁-C₄)fluoroalkyl group, a -COOH group, a (C₁-C₄)alkylsulfonyl group or a (C₁-C₄)alkoxy group, in particular a hydrogen atom, a halogen atom, a (C₁-C₄)fluoroalkyl group or a (C₁-C₄)alkoxy group.

According to one embodiment, the linear or cyclic nitrogen containing (C₄-C₈)alkyl group comprises one protonable amine. According to another embodiment, the linear or cyclic nitrogen containing (C₄-C₈)alkyl group comprises two protonable amine. In one embodiment, the linear or cyclic nitrogen containing (C₄-C₈)alkyl group comprises one protonable amine is cyclic. In one embodiment, the linear or cyclic nitrogen containing (C₄-C₈)alkyl group comprises one protonable amine is linear. In one embodiment, the linear or cyclic nitrogen containing (C₄-C₈)alkyl group is interrupted by one oxygen atom.

According to one embodiment, a compound of formula (I) as defined above or any of its pharmaceutically acceptable salt, is provided, wherein R6 represent a halogen atom, a COOH group, a (C₁-C₄)fluoroalkyl group, a (C₁-C₄)alkylsulfonyl group or a (C₁-C₄)alkoxy group.

According to a preferred embodiment of the present invention, the compound of formula (I) or a pharmaceutically acceptable salt thereof, in particular hydrochloride salt thereof, characterized in that said compound is selected from the following compounds:
- (1) N-(5-fluoroquinolin-8-yl)-4-(1-methylpiperidin-4-yl)benzamide,
- (2) N-(8-fluoroisoquinolin-5-yl)-4-(1-methylpiperidin-4-yl)benzamide,
- (3) N-(5-fluoroisoquinolin-8-yl)-4-(1-methylpiperidin-4-yl)benzamide,
- (4) N-(8-fluoroquinolin-5-yl)-4-(1-methylpiperidin-4-yl)benzamide,
- (5) N-(8-fluoroquinoxalin-5-yl)-4-(1-methylpiperidin-4-yl)benzamide,
- (6) 4-(1-methylpiperidin-4-yl)-N-(quinolin-5-yl)benzamide,
- (7) N-(isoquinolin-5-yl)-4-(l-methylpiperidin-4-yl)benzamide,
- (8) N-(isoquinolin-8-yl)-4-(1-methylpiperidin-4-yl)benzamide,
- (9) 4-(1-methylpiperidin-4-yl)-N-(quinolin-8-yl)benzamide,
- (10) 4-[2-(dimethylamino)ethoxy]-N-(isoquinolin-8-yl)benzamide,
- (11) 4-[2-(dimethylamino)ethoxy]-N-(quinolin-8-yl)benzamide,
- (12) 2-fluoro-4-(1-methylpiperidin-4-yl)-N-(quinolin-8-yl)benzamide,
- (13) 3-fluoro-N-(isoquinolin-5-yl)-4-(1-methylpiperidin-4-yl)benzamide,
- (14) 4-(2-(dimethylamino)ethoxy)-N-(isoquinolin-5-yl)benzamide,
- (15) 4-(2-(dimethylamino)ethoxy)-N-(quinolin-5-yl)benzamide,
- (16) 2-methoxy-4-(1-methylpiperidin-4-yl)-N-(quinolin-8-yl)benzamide,
- (17) N-(isoquinolin-5-yl)-3-methoxy-4-(1-methylpiperidin-4-yl)benzamide,
- (18) 4-(1-methylpiperidin-4-yl)-N-(quinolin-8-yl)-2-(trifluoromethyl)benzamide,
- (19) 4-[2-(dimethylamino)ethoxy]-N-(phthalazin-5-yl)benzamide,
- (20) 4-(2-(dimethylamino)ethoxy)-N-(8-fluoroquinolin-5-yl)benzamide,
- (21) 3-cyano-N-(isoquinolin-5-yl)-4-(1-methylpiperidin-4-yl)benzamide,
- (22) 4-(2-(dimethylamino)ethoxy)-N-(5-fluoroisoquinolin-8-yl)benzamide,
- (23) 4-(2-(dimethylamino)ethoxy)-N-(8-fluoroquinoxalin-5-yl)benzamide,
- (24) 3-fluoro-N-(isoquinolin-5-yl)-4-(4-methylpiperazin-1-yl)benzamide,
- (25) N-(isoquinolin-5-yl)-2-methanesulfonyl-4-(1-methylpiperidin-4-yl)benzamide,
- (26) 4-[2-(dimethylamino)ethoxy]-N-(5-fluoroquinolin-8-yl)benzamide,
- (27) 4-(1-methylpiperidin-4-yl)-N-(quinolin-5-yl)-3-(trifluoromethyl)benzamide,
- (28) 4-(1-methylpiperidin-4-yl)-N-(quinoxalin-5-yl)benzamide,
- (29) 3-methyl-4-(4-methylpiperazin-1-yl)-N-(quinolin-5-yl)benzamide,
- (30) N-(8-chloroquinolin-5-yl)-4-(1-methylpiperidin-4-yl)benzamide,
- (31) 4-[2-(dimethylamino)ethoxy]-N-(quinoxalin-5-yl)benzamide,
- (32) N-(8-chloroquinolin-5-yl)-4-[2-(dimethylamino)ethoxy]benzamide,
- (33) 4-[2-(dimethylamino)ethoxy]-N-(8-fluoroisoquinolin-5-yl)benzamide,
- (34) 4-[2-(dimethylamino)ethoxy]-N-(7-methoxyisoquinolin-5-yl)benzamide,
- (35) 4-(4-methylpiperazin-1-yl)-N-(quinolin-8-yl)benzamide,
- (36) 3-cyano-4-(2-(dimethylamino)ethoxy)-N-(isoquinolin-8-yl)benzamide,
- (37) N-(8-fluoroquinoxalin-5-yl)-4-(4-methylpiperazin-1-yl)benzamide,
- (38) N-(isoquinolin-8-yl)-4-(4-methylpiperazin-1-yl)benzamide,
- (39) N-(isoquinolin-5-yl)-4-(4-methylpiperazin-1-yl)benzamide,
- (40) 4-(4-methylpiperazin-1-yl)-N-(quinolin-5-yl)benzamide,
- (41) 4-(4-methylpiperazin-1-yl)-N-(quinoxalin-5-yl)benzamide,
- (42) N-(5-fluoroquinolin-8-yl)-4-(4-methylpiperazin-1-yl)benzamide,
- (43) 4-(2-(dimethylamino)ethoxy)-3-methyl-N-(quinolin-8-yl)benzamide,
- (44) N-(8-fluoroquinolin-5-yl)-4-(4-methylpiperazin-1-yl)benzamide,
- (45) N-(isoquinolin-5-yl)-2-methyl-4-(4-methylpiperazin-1-yl)benzamide,
- (46) N-(8-chloroquinolin-5-yl)-4-(4-methylpiperazin-1-yl)benzamide,
- (47) 4-(2-(dimethylamino)ethoxy)-2-methyl-N-(quinolin-5-yl)benzamide,
- (48) 4-(2-(dimethylamino)ethoxy)-N-(isoquinolin-5-yl)-3-(trifluoromethyl)benzamide,
- (49) 4-(2-(dimethylamino)ethoxy)-N-(quinolin-5-yl)-2-(trifluoromethyl)benzamide,
- (50) 3-methoxy-4-(4-methylpiperazin-1-yl)-N-(quinolin-5-yl)benzamide,
- (51) N-(8-chloroisoquinolin-5-yl)-4-(4-methylpiperazin-1-yl)benzamide hydrochloride,
- (52) 4-(4-methylpiperazin-1-yl)-N-[5-(trifluoromethyl)isoquinolin-8-yl]benzamide hydrochloride,
- (53) 3-fluoro-N-(5-fluoroisoquinolin-8-yl)-4-{5-methyloctahydropyrrolo[3,4-c]pyrrol-2-yl}benzamide,
- (54) N-(8-methoxyisoquinolin-5-yl)-3-methyl-4-{7-methyl-2,7-diazaspiro[3.5]nonan-2-yl}benzamide, and
- (55) 3-methyl-4-[(1-methylpyrrolidin-3-yl)methoxy]-N-(quinolin-8-yl)benzamide,
- (56) 2-fluoro-4-(4-methylpiperazin-1-yl)-N-(quinolin-5-yl)benzamide, and
- (57) 2-fluoro-N-(isoquinolin-8-yl)-4-[4-(propan-2-yl)piperazin-1-yl]benzamide.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

As used herein, the terms below have the following definitions unless otherwise mentioned throughout the instant specification:
Carboxyl activating agent: a carboxyl activating agent that can be used as additive to perform coupling between carboxylic acids and amines. This agent is used to create *in situ* an activated ester leaving group, enhancing or allowing the reaction. By way of examples, mention may be made of, but not limited to: N-(3-Dimethylaminopropyl)-N'-ethylcarbodiimide (EDCI) and N,N'-Diisopropylcarbodiimide (DIC or dicyclohexylcarbodiimide (DCC).

Racemization suppressor agent: A racemization suppressor agent that can be used in addition to carboxyl activation agent in order to suppress the racemization that can occur while coupling between carboxylic acids and amines. By way of examples, mention may be made of, but not limited to: 1-Hydroxybenzotriazole (HOBt), 1-hydroxy-7-aza-benzotriazole (HOAt) and Ethyl cyanohydroxyiminoacetate (for example commercialized under the name of Oxyma^{®} by Merck Millipore).

Alkali metals: By way of examples, mention may be made of, but not limited to: lithium (Li), sodium (Na), potassium (K), and cesium (Cs).

Chlorinating agent: chemical reagents that can be used to add chlorine atoms to other chemicals, in particular the chlorinating can be used to convert a carboxylic acid or a carboxylate to an acyl chloride. By way of examples, mention may be made of, but not limited to: SOCl₂, PCl₃, PCl₅, oxalyl chloride and propionyl chloride.

The following reactions may be implemented for obtaining a compound of formula (I) as set out in scheme **1** herein after:

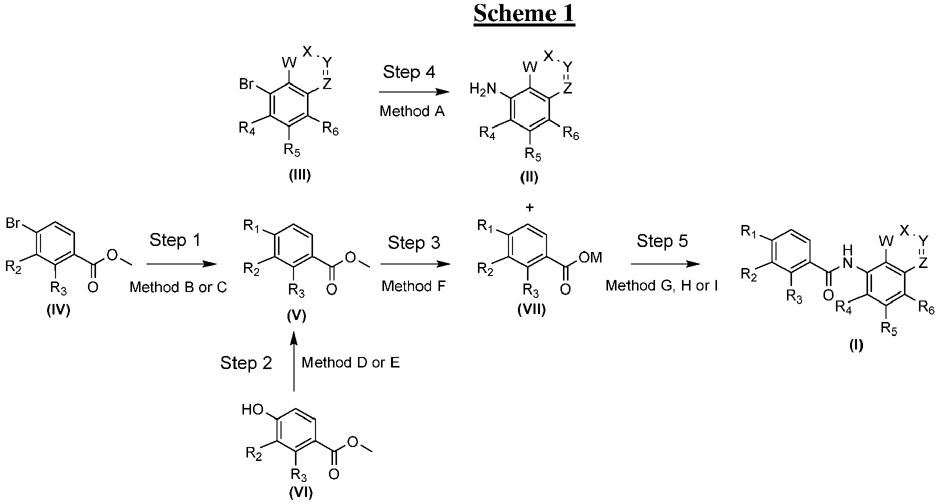

According to scheme 1, in which W, X, Y, Z, R1, R2, R3, R4, R5 and R6 are as defined above and M represents an alkali metal or a hydrogen, compound (IV) can be converted in STEP 1 to compound (V), when R1 represents a cyclic nitrogen connected by a carbon atom to the central phenyl ring, by treatment with a boronic acid or ester derivative in the presence of a palladium catalyst, for example palladium acetate (Pd(OAc)₂), a phosphine ligand such as 2-Dicyclohexylphosphino-2',6'-diisopropoxybiphenyl (RuPhos), and a base such as K₂CO₃ in solution in a mixture of solvent for example, toluene and water, by heating up to 80°C. The resulting compound can then be used in a hydrogenation reaction using palladium supported on coal Pd/C as a catalyst, a polar protic solvent for example, MeOH or EtOH, under an H₂ atmosphere (Method B: adapted from Miyaura et al. Tetrahedron Letters, 1979, 20, 3437-3440). Alternative way to prepare compound (V) when R1 represents a cyclic nitrogen connected by a nitrogen atom to the central phenyl ring, from compound (IV) can be done using a Buchwald-Hartwig reaction with an amine, in the presence of a palladium catalyst, for example tris(dibenzylideneacetone)dipalladium(0) (Pd₂(dba)₃), a phosphine ligand such as (9,9-dimethyl-9H-xanthene-4,5-diyl)bis(diphenylphosphane) (Xantphos), and a base such as Cs₂CO₃ in solution for example in dioxane by heating up to reflux of solvent (Method C: adapted from Guram et al., Angew. Chem. Int. Ed Engl. 1995, 34, 1348-1350).

Compound (VI) can be converted in STEP 2 to compound (V), by nucleophilic substitution using a chloride derivative and a base such as K₂CO₃ in solution for example in DMF by heating up to 80°C (Method D). Alternative way to prepare compound (V) from compound (VI) can be done by a Mitsunobu coupling using an alcohol derivative in presence of triphenylphosphine (PPh₃) and diisopropyl azodicarboxylate (DIAD) in solution for example in THF by stirring at 0°C (Method E: adapted from Maghar et al. J. Chem. Soc. Perkin Trans. 1975, 1, 461-463).

Compound (V) can be converted in STEP 3 to compound (VII), by saponification using a base such as LiOH, NaOH, KOH, RbOH or CsOH and water in solution in a mixture of solvent for example, MeOH and THF (Method F: adapted from Chan et al. J. Org. Chem., 2007, 72, 8863-8869). Optionally, the resulting carboxylate derivative can be treated with an acid to obtain the corresponding carboxylic derivative.

Compound (III) can be converted in STEP 4 to compound (II), by treatment with sodium azide (NaN3), copper catalyst such as iodide (CuI), and a base such as trans-N,N'-Dimethylcyclohexane-1,2-diamine (TMDCA) in solution for example in DMSO by heating up to 105°C (Method A: adapted from Klapars et al. J. Am. Chem. Soc. 2001, 123, 7727-7729).

Compounds (II) and (VII) can be converted together in STEP 5 to compound (I), by a peptide coupling using a carboxyl activating agent for peptide synthesis such as N-(3-Dimethylaminopropyl)-N'-ethylcarbodiimide (EDCI) or N,N'-Diisopropylcarbodiimide (DIC), a racemization suppressor agent such as 1-Hydroxybenzotriazole (HOBt) or Ethyl cyanohydroxyiminoacetate (for example commercialized under the name of Oxyma^{®} by Merck Millipore) and an organic base such as triethylamine (Et3N) or N,N-Diisopropylethylamine (DIPEA) in solution for example in DMF, optionally by heating up to 33°C (Method G: adapted from Schotten. Ber. Deutsch. Chem. Gesell., 1884, 17, 2544; Method I: adapted from Subirós-Funosas et al. Chem. Eur. J., 2009, 15, 9394-9403). Alternative way to prepare compound (I) from compounds (VII) and (II) can be done, first, by treatment of (VII) by a chlorinating agent such as SOCl₂, PCl₃ or PCl₅, and optionally an organic base such as triethylamine (Et₃N), in an aprotic solvent such as THF, in order to form the corresponding acyl chloride derivative. Then said acyl chloride derivative may be reacted with compound (II) in the presence of an organic base such as triethylamine (Et3N) in solution for example in DMF (Method H: adapted from Baumann. *Ber. Deutsch. Chem. Gesell,* **1886,** 19,3212)

Alternatively, the present invention relates to a synthesis process for manufacturing a compound of formula (I) as defined above or anyone of its pharmaceutically acceptable salts, comprising at least the following step:
- reacting a compound of formula (II): 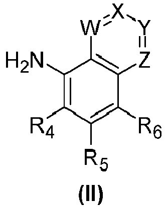 wherein R4, R5, R6, W, X, Y and Z are as defined hereinabove,
   with a compound of formula (VII): 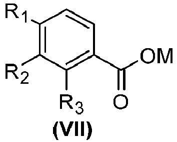 wherein R1, R2, R3 are as defined as defined hereinabove and M represents an alkali metal or a hydrogen, in order to obtain a compound of formula (I),
   (i) by a peptide synthesis using a carboxyl activating agent, in particular N-(3-Dimethylaminopropyl)-N'-ethylcarbodiimide (EDCI) or N,N'-Diisopropylcarbodiimide (DIC) with a racemization suppressor agent, in particular 1-Hydroxybenzotriazole (HOBt) or ethyl cyanohydroxyiminoacetate, and with an organic base in an aprotic polar solvent; or
   (ii) first, by treating a compound of formula (VII) separately in presence of a chlorinating agent, in particular SOCh, and optionally an organic base in an aprotic solvent in order to form the corresponding acyl chloride derivative and then, by reacting said acyl chloride derivative with a compound of formula (II) by nucleophilic substitution using an organic base in an aprotic solvent.

All reactions were carried out under usual atmosphere unless otherwise stated. Chemicals and solvents were purchased from several suppliers and were used without further purification.

Analytical TLC were performed using silica gel plates Merck 60F254 and plates were visualized by exposure to ultraviolet light. Compounds were purified on silica gel VWR (particle size 0.040-0.063nm) or using Buchi flash chromatography (normal phase column: Buchi Silica 40 µm irregular, 4 or 12g).

HPLC were performed using Agilent 1260 Infinity II using the following parameters: Flow rate of 1.5 mL/min, column temperature: 40°C (InfinityLab Poroshell 120 EC-C18, 4.6 × 100mm, 2.7 micron), solvent system: A (0.05% of TFA in H2O) and B (Acetonitrile), t = 0 min to 1 min: 95% of A and 5% of B then t = 1 min to t = 7 min: 5% to 60% of B, t = 7 min to t = 8.5 min: 60 % to 100% of B, t = 8.5 min to t = 9 min: 100% and finally t = 9 min to t = 9.5 min: 100% to 5%.¹H, ¹⁹F and ¹³C NMR spectra were recorded on Bruker Avance Spectrometer operating at 400 or 500 MHz, 376 MHz and 101 or 125 MHz, respectively. All chemical shift values δ and coupling constants J are quoted in ppm and in Hz, respectively, multiplicity (s = singulet, d = doublet, t = triplet, q = quartet, quin = quintet, sex = sextet m = multiplet, br = broad). All spectra were evaluated using MestreNova 14.1.1 (MestreLab Research SSL).

Analytical RP-HPLC-MS was performed using a LC 1200 Agilent with quadrupole-time-of-flight (QTOF) (Agilent Accurate Mass QToF 6520) with a Zorbax Agilent C18-column (C18,50 mmx2.1mm;1.8 µm) using the following parameters: 1) The solvent system: A (acetonitrile + 0.5% formic acid) and B (H2O+ 0.05% formic acid). 2) Gradient: t = 0 to 8 min: 98 to 0% of B; t = 8 to 12.5 min: 0% of B; t = 12.5 to 12.6 min: 0 to 98% of B and t = 12.6 to 13 min: 98% of B. 3) Flow rate of 0.5 mL/min. 4) Column temperature: 40°C. 5) DAD scan from 190 nm to 700 nm. 6) Ionization mode: ESI+.

The chemical structures and spectroscopic data of some compounds of formula (I) of the invention are illustrated respectively in the following Table I and Table II.

**Table I**

| **Compound No** | **Structure** |
|---|---|
| (1) | 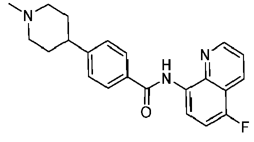 |
| (2) | 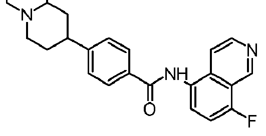 |
| (3) | 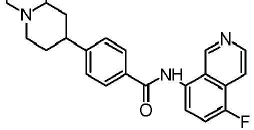 |
| (4) | 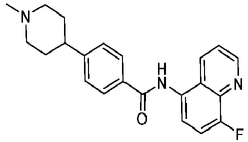 |
| (5) | 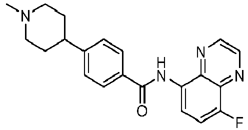 |
| (6) | 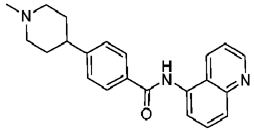 |
| (7) | 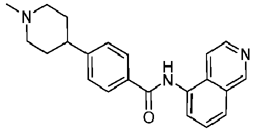 |
| (8) | 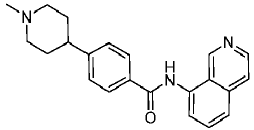 |
| (9) | 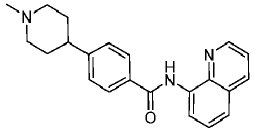 |
| (10) | 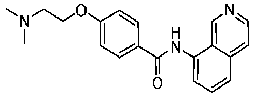 |
| (11) | 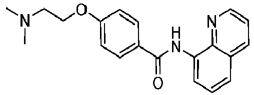 |
| (12) | 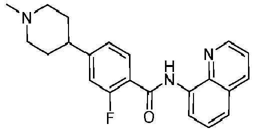 |
| (13) | 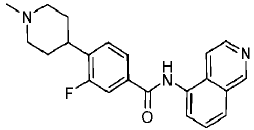 |
| (14) | 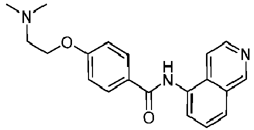 |
| (15) | 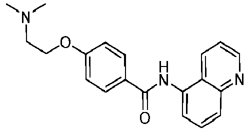 |
| (16) | 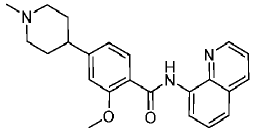 |
| (17) | 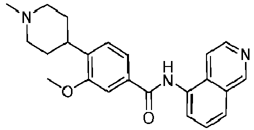 |
| (18) | 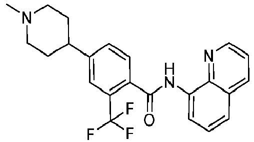 |
| (19) | 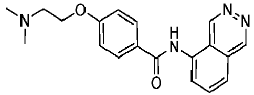 |
| (20) | 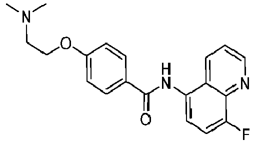 |
| (21) | 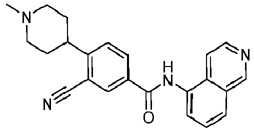 |
| (22) | 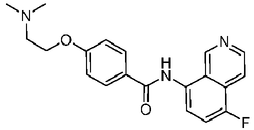 |
| (23) | 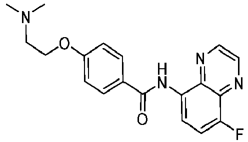 |
| (24) | 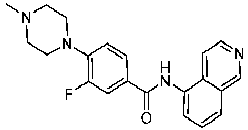 |
| (25) | 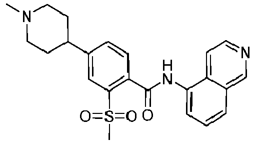 |
| (26) | 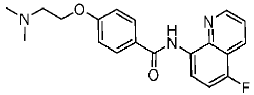 |
| (27) | 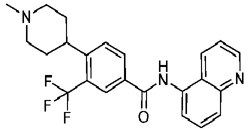 |
| (28) | 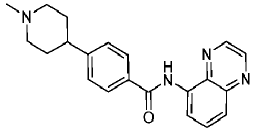 |
| (29) | 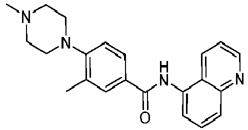 |
| (30) | 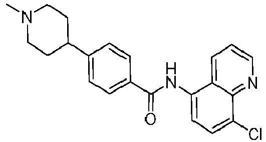 |
| (31) | 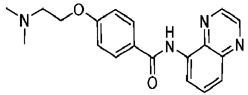 |
| (32) | 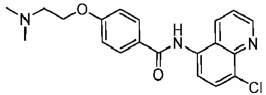 |
| (33) | 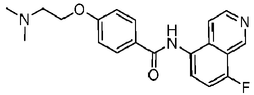 |
| (34) | 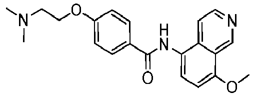 |
| (35) | 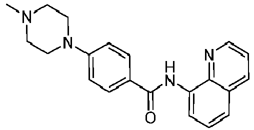 |
| (36) | 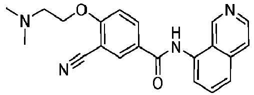 |
| (37) | 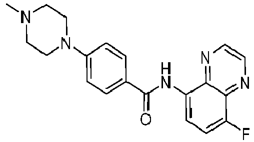 |
| (38) | 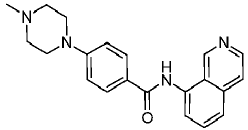 |
| (39) | 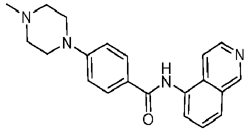 |
| (40) | 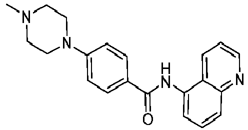 |
| (41) | 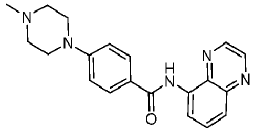 |
| (42) | 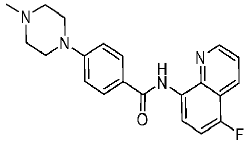 |
| (43) | 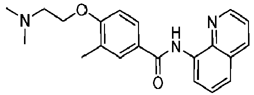 |
| (44) | 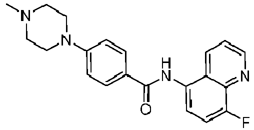 |
| (45) | 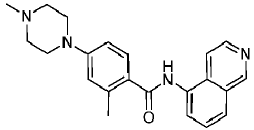 |
| (46) | 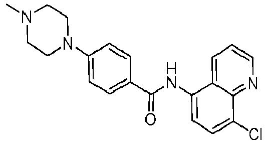 |
| (47) | 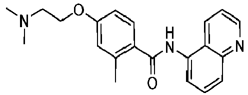 |
| (48) | 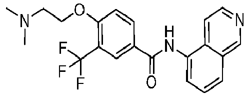 |
| (49) | 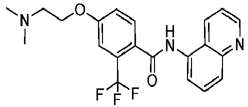 |
| (50) | 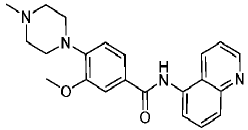 |
| (51) | 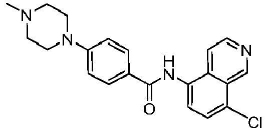 |
| (52) | 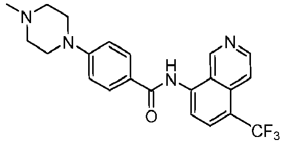 |
| (53) | 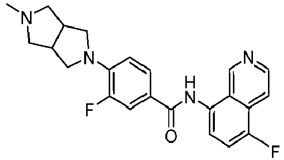 |
| (54) | 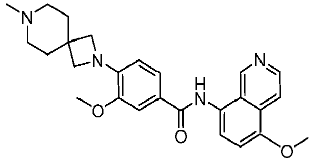 |
| (55) | 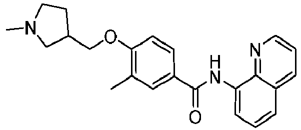 |
| (56) | 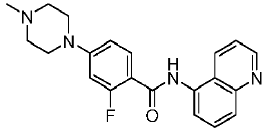 |
| (57) | 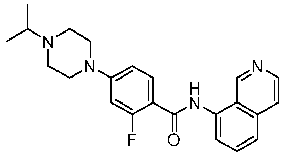 |

**Table II**

| **No** | **General methods** | **NMR** |
|---|---|---|
| (1) | B, F, G | **NMR¹H (400 MHz, MeOD) :** δ 8.96 (dd, *J* = 4.3, 1.6 Hz, 1H), 8.71 (dd, *J* = 8.7, 5.4 Hz, 1H), 8.51 (dd, *J* = 8.5, 1.7 Hz, 1H), 7.96 (d, *J* = 8.3 Hz, 2H), 7.66 (dd, *J* = 8.5, 4.3 Hz, 1H), 7.46 (d, *J* = 8.2 Hz, 2H), 7.31 (dd, *J* = 9.9, 8.6 Hz, 1H), 3.04 (bd, *J* = 11.7 Hz, 2H), 2.67 (tt, *J* = 11.6, 4.3 Hz, 1H), 2.36 (s, 3H), 2.21 (td, *J* = 11.8, 3.1 Hz, 2H), 1.95-1.76 (m, 4H). |
| | | **NMR¹³C (101 MHz, MeOD) :** δ = 167.11 (CO), 154.69 (d, *J* = 251.0 Hz, C_{q}), 152.00 (C_{q}), 151.11 (CH_{Ar}), 140.52 (d, *J* = 3.3 Hz, C_{q}), 133.84 (C_{q}), 132.19 (d, *J* = 4.2 Hz, C_{q}), 130.77 (d, *J* = 4.0 Hz, CH_{Ar}), 128.56 (2CH_{Ar}), 128.50 (2CH_{Ar}), 123.47 (d, *J* = 2.6 Hz, CH_{Ar}), 120.09 (d, *J* = 18.2 Hz C_{q}), 117.66 (d, *J* = 8.0 Hz, CH_{Ar}), 111.20 (d, *J* = 20.1 Hz, CH_{Ar}), 56.93 (2CH₂), 46.33 (CH₃), 42.79 (CH), 33.78 (2CH₂). |
| | | **NMR¹⁹F (376 MHz, MeOD, decoupled) :** δ = -130.34. |
| | | **HRMS (ESI):** m/z calculated for C₂₂H₂₃FN₃O⁺, [M+H]⁺ 364.1820, found 364.1830. |
| (2) | A, H | **NMR¹H (400 MHz, MeOD) :** δ = 9.50 (s, 1H), 8.59 (d, *J* = 6.1 Hz, 1H), 8.03 (d, *J* = 8.3 Hz, 2H), 7.91-7.85 (m, 2H), 7.48-2.44 (m, 3H), 3.10 (d, *J* = 11.7 Hz, 2H), 2.72 (tt, *J* = 11.6, 4.1 Hz, 1H), 2.41 (s, 3H), 2.30 (td, *J* = 11.9, 3.0 Hz, 2H), 1.99-1.80 (m, 4H). |
| | | **NMR¹³C (101 MHz, MeOD) :** δ = 169.77 (CO), 158.86 (d, *J* = 255.2 Hz, C_{q}), 151.66 (C_{q}), 146.94 (d, *J* = 5.2 Hz, CH_{Ar}), 144.40 (CH_{Ar}), 134.98 (d, *J* = 3.7 Hz, C_{q}), 133.25 (C_{q}), 130.66 (d, *J* = 8.6 Hz, CH_{Ar}), 130.58 (C_{q}), 129.24 (2CH_{Ar}), 128.30 (2CH_{Ar}), 120.65 (d, *J* = 16.5 Hz, C_{q}), 117.91 (d, *J* = 2.2 Hz, CH_{Ar}), 112.41 (d, *J* = 20.5 Hz, CH_{Ar}), 56.77 (2CH₂), 45.97 (CH₃), 42.46 (CH), 33.47 (2CH₂). |
| | | **NMR¹⁹F (376 MHz, MeOD, decoupled) :** δ = -126.4. |
| | | **HRMS (ESI):** m/z calculated for C₂₂H₂₃FN₃O⁺, [M+H]⁺ 364.1820, found 364.1821. |
| (3) | A, G | **NMR ¹H (400 MHz, MeOD) :** δ 9.39 (s, 1H), 8.40 (d, *J* = 6.0 Hz, 1H), 7.80 (bd, *J* = 6.0 Hz, 1H), 7.24 (dd, *J* = 10.1, 8.4 Hz, 1H), 6.79 (dd, *J* = 8.4, 4.1 Hz, 1H). |
| | | **NMR ¹³C (101 MHz, MeOD) :** δ = 150.72 (d, *J* = 240.0 Hz, C_{q}), 148.39 (d, *J* = 2.5 Hz, CH_{Ar}), 143.74 (d, *J* = 3.0 Hz, C_{q}), 142.76 (d, *J* = 1.8 Hz, CH_{Ar}), 127.68 (d, *J* = 18.5 Hz, C_{q}), 119.81 (d, *J* = 3.9 Hz, C_{q}), 116.97 (d, *J* = 20.2 Hz, CH_{Ar}), 114.70 (d, *J* = 3.7 Hz, CH_{Ar}), 110.53 (d, *J* = 6.5 Hz, CH_{Ar}). |
| | | **NMR ¹⁹F (376 MHz, MeOD, decoupled):** δ = -141.11. |
| | | **HRMS (ESI):** m/z calculated for C₂₂H₂₃FN₃O⁺, [M+H]⁺ 364.1820, found 364.1823. |
| (4) | G | **NMR ¹H (400 MHz, MeOD) :** δ 8.94 (dd, *J* = 4.3, 1.6 Hz, 1H), 8.44 (dt, *J =* 8.7, 1.6 Hz, 1H), 8.10 - 8.04 (m, 2H), 7.70 - 7.63 (m, 2H), 7.58 (dd, *J* = 10.4, 8.3 Hz, 1H), 7.52 - 7.46 (m, 2H), 3.60 (d, *J =* 12.5 Hz, 2H), 3.19 - 3.09 (m, 2H), 3.02 (tt, *J* = 12.1, 3.7 Hz, 1H), 2.91 (s, 3H), 2.22 - 2.13 (m, 2H), 2.04 (qd, *J* = 13.1, 3.8 Hz, 2H). |
| | | **NMR¹³C (126 MHz, MeOD) :** δ 169.64 (CO), 157.58 (d, *J* = 255.1 Hz, CF), 151.69 (CH_{Ar}), 149.53 (C_{q}), 139.13 (d, *J* = 12.7 Hz, C_{q}), 134.21 (d, *J* = 2.3 Hz, CH_{Ar}), 133.84 (C_{q}), 131.03 (d, *J* = 4.9 Hz, C_{q}), 129.45 (2CH_{Ar}), 128.22 (2CH_{Ar}), 127.92 (d, *J* = 2.0 Hz, C_{q}), 126.07 (d, *J* = 8.0 Hz, CH_{Ar}), 123.58 (CH_{Ar}), 114.43 (d, *J* = 20.3 Hz, CH_{Ar}), 55.79 (2CH₂), 43.93 (CH₃), 40.36 (CH), 31.61 (2CH₂). |
| | | **NMR¹⁹F (376 MHz, MeOD) :** δ -128.46. |
| | | **HRMS (ESI):** m/z calculated for C₂₂H₂₃FN₃O⁺ [M+H]⁺: 364.1820; found 364.1829. |
| (5) | A, G | **NMR ¹H (400 MHz, MeOD):** δ 9.04 (d, *J* = 1.8 Hz, 1H), 8.97 (d, *J* = 1.8 Hz, 1H), 8.16 (dd, *J* = 8.5, 5.0 Hz, 1H), 7.54 (dd, *J* = 9.6, 8.5 Hz, 1H). |
| | | **NMR ¹³C (101 MHz, MeOD):** δ = 158.18 (d, *J* = 260.8 Hz, C_{q}), 148.07 (CH_{Ar}), 147.15 (d, *J* = 2.9 Hz, CH_{Ar}), 142.26 (C_{q}), 135.52 (d, *J* = 13.3 Hz, C_{q}), 134.25 (d, *J* = 8.0 Hz, CH_{Ar}), 119.51 (d, *J* = 4.8 Hz, C_{q}), 115.08 (d, *J* = 20.0 Hz, CH_{Ar}). |
| | | **NMR ¹⁹F (376 MHz, MeOD, decoupled):** δ = -126.86. |
| | | **HRMS (ESI):** m/z calculated for C₂₁H₂₂FN₄O⁺, [M+H]⁺ 365.1772, found 365.1782. |
| (6) | G | **NMR¹H (400 MHz, MeOD) :** δ 8.88 (dd, *J* = 4.3, 1.7 Hz, 1H), 8.45 (bd, *J* = 8.3 Hz, 1H), 8.07-7.98 (m, 3H), 7.83 (dd, *J* = 8.5, 7.5 Hz, 1H), 7.72 (dd, *J* = 7.4, 1.1 Hz, 1H), 7.57 (dd, *J* = 8.6, 4.3 Hz, 1H), 7.46 (d, *J* = 8.4 Hz, 2H), 3.08 (dt, *J* = 12.7, 3.3 Hz, 2H), 2.71 (tt, *J* = 11.6, 4.2 Hz, 1H), 2.40 (s, 3H), 2.28 (td, *J* = 11.9, 3.1 Hz, 2H), 1.97-1.78 (m, 4H). |
| | | **NMR ¹³C (101 MHz, MeOD) :** δ 169.76 (CO), 151.81 (C_{q}), 151.43 (CH_{Ar}), 149.31 (C_{q}), 135.26 (C_{q}), 134.11 (CH_{Ar}), 133.36 (C_{q}), 130.70 (CH_{Ar}), 129.22 (2CH_{Ar}), 128.29 (2CH_{Ar}), 128.09 (CH_{Ar}), 126.39 (C_{q}), 125.74 (CH_{Ar}), 122.50 (CH_{Ar}), 56.87 (2CH₂), 46.19 (CH₃), 42.67 (CH), 33.68 (2CH₂). |
| | | **HRMS (ESI):** m/z calculated for C₂₂H₂₄N₃O⁺, [M+H]⁺ 346.1914, found 346.1917. |
| (7) | G | **NMR¹H (400 MHz, MeOD) :** δ 9.29 (d, *J* = 1.0 Hz, 1H), 8.47 (d, *J* = 6.1 Hz, 1H), 8.08 (dt, *J* = 8.3, 1.0 Hz, 1H), 8.04 (d, *J* = 8.4 Hz, 2H), 7.93 (dd, *J* = 7.4, 1.1 Hz, 1H), 7.89 (dt, *J* = 6.0, 1.0 Hz, 1H), 7.75 (dd, *J* = 8.2, 7.4 Hz, 1H), 7.47 (d, *J* = 8.2 Hz, 2H), 3.08 (dt, *J* = 12.7, 3.3 Hz, 2H), 2.71 (tt, *J* = 11.6, 4.2 Hz, 1H), 2.40 (s, 3H), 2.28 (td, *J* = 11.9, 3.1 Hz, 2H), 1.98-1.79 (m, 4H). |
| | | **NMR ¹³C (101 MHz, MeOD) :** δ 169.66 (CO), 153.56 (CH_{Ar}), 151.84 (C_{q}), 143.07 (CH_{Ar}), 134.26 (C_{q}), 133.74 (C_{q}), 133.39 (CH_{Ar}), 130.83 (C_{q}), 129.68 (CH_{Ar}), 129.23 (2CH_{Ar}), 128.76 (CH_{Ar}), 128.30 (2CH_{Ar}), 127.77 (C_{q}), 117.93 (CH_{Ar}), 56.89 (2CH₂), 46.21 (CH₃), 42.71 (CH), 33.70 (2CH₂). |
| | | **HRMS (ESI):** m/z calculated for C₂₂H₂₄N₃O⁺ [M+H]⁺: 346.1914; found 346.1919. |
| (8) | G | **NMR¹H (400 MHz, MeOD) :** δ 9.41 -9.28 (m, 1H), 8.48 (d, *J* = 5.8 Hz, 1H), 8.07 - 8.01 (m, 2H), 7.94 - 7.87 (m, 2H), 7.85 (dd, *J* = 8.2, 7.3 Hz, 1H), 7.78 (dd, J= 7.3, 1.3 Hz, 1H), 7.51 - 7.44 (m, 2H), 3.08 - 3.01 (m, 2H), 2.74 - 2.64 (m, 1H), 2.35 (s, 3H), 2.21 (td, *J* = 11.8, 3.1 Hz, 2H), 1.96 - 1.79 (m, 4H). |
| | | **NMR ¹³C (101 MHz, MeOD) :** δ 169.86 (CO), 152.12 (C_{q}), 149.26 (CH_{Ar}), 142.98 (CH_{Ar}), 138.43 (C_{q}), 135.99 (C_{q}), 133.23 (C_{q}), 132.26 (CH_{Ar}), 129.21 (2CH_{Ar}), 128.33 (2CH_{Ar}), 126.55 (2CH_{Ar}), 125.80 (C_{q}), 122.47(CH_{Ar}), 56.99 (2CH₂), 46.42 (CH3), 42.93 (CH), 33.89 (2CH₂). |
| | | **HRMS (ESI):** m/z calculated for C₂₂H₂₄N₃O⁺ [M+H]⁺: 346.1914; found 346.1914. |
| (9) | G | **NMR¹H (400 MHz, MeOD) :** δ 8.92 (dd, *J* = 4.2, 1.7 Hz, 1H), 8.78 (dd, *J =* 7.5, 1.5 Hz, 1H), 8.35 (dd, *J* = 8.3, 1.7 Hz, 1H), 8.06 - 7.94 (m, 2H), 7.67 (dd, *J* = 8.3, 1.4 Hz, 1H), 7.64 - 7.57 (m, 2H), 7.51 - 7.46 (m, 2H), 3.08 - 3.01 (m, 2H), 2.69 (tt, *J* = 11.6, 4.3 Hz, 1H), 2.36 (s, 3H), 2.22 (td, *J* = 11.8, 3.0 Hz, 2H), 1.96 - 1.78 (m, 4H). |
| | | **NMR ¹³C (126 MHz, MeOD) :** δ 167.28 (CO), 152.02 (C_{q}), 150.10 (CH_{Ar}), 140.20 (C_{q}), 137.83 (CH_{Ar}), 135.48 (C_{q}), 134.02 (C_{q}), 129.67 (C_{q}), 128.59 (2CH_{Ar}), 128.52 (2CH_{Ar}), 128.15 (CH_{Ar}), 123.57 (CH_{Ar}), 123.22 (CH_{Ar}), 117.94 (CH_{Ar}), 56.95 (2CH₂), 46.35 (CH₃), 42.83 (CH), 33.81 (2CH₂). |
| | | **HRMS (ESI):** m/z calculated for C₂₂H₂₄N₃O⁺ [M+H]⁺: 346.1914; found 346.1923. |
| (10) | D, F, G | **NMR ¹H (400 MHz, MeOD) :** δ 9.32 (t, *J* = 1.0 Hz, 1H), 8.45 (d, *J* = 5.8 Hz, 1H), 8.11 - 8.01 (m, 2H), 7.89 - 7.83 (m, 2H), 7.81 (dd, *J* = 8.2, 7.2 Hz, 1H), 7.74 (dd, *J* = 7.3, 1.3 Hz, 1H), 7.19 - 7.04 (m, 2H), 4.21 (t, *J* = 5.4 Hz, 2H), 2.81 (t, *J* = 5.4 Hz, 2H), 2.36 (s, 6H). |
| | | **NMR ¹³C (126 MHz, MeOD) :** δ 169.43 (CO), 163.52 (C_{q}), 149.29 (CH_{Ar}), 142.92 (CH_{Ar}), 138.36 (C_{q}), 136.10 (C_{q}), 132.21 (CH_{Ar}), 130.94 (2CH_{Ar}), 127.43 (C_{q}), 126.46 (CH_{Ar}), 126.40 (CH_{Ar}), 125.79 (C_{q}), 122.43 (CH_{Ar}), 115.53 (2CH_{Ar}), 66.95 (CH₂), 58.98 (CH₂), 45.87 (2CH₃). |
| | | **HRMS (ESI):** m/z calculated for C₂₀H₂₂N₃O₂⁺ [M+H]⁺: 336.1707; found 336.1703. |
| (11) | D, F, G | **NMR ¹H (400 MHz, MeOD) :** δ 8.92 (dd, *J* = 4.2, 1.7 Hz, 1H), 8.77 (dd, *J =* 7.4, 1.5 Hz, 1H), 8.34 (dd, *J* = 8.3, 1.7 Hz, 1H), 8.08 - 7.96 (m, 2H), 7.68 - 7.60 (m, 2H), 7.60 - 7.57 (m, 1H), 7.20 - 7.07 (m, 2H), 4.23 (t, *J* = 5.4 Hz, 2H), 2.84 (t, *J* = 5.4 Hz, 2H), 2.39 (s, 6H). |
| | | **NMR ¹³C (126 MHz, MeOD) :** δ 166.95 (CO), 163.48 (C_{q}), 150.02 (CH_{Ar}), 140.17 (C_{q}), 137.80 (CH_{Ar}), 135.57 (C_{q}), 130.22 (2CH_{Ar}), 129.65 (C_{q}), 128.27 (C_{q}), 128.14 (CH_{Ar}), 123.37 (CH_{Ar}), 123.18 (CH_{Ar}), 117.81 (CH_{Ar}), 115.80 (2CH_{Ar}), 66.96 (CH₂), 58.98 (CH₂), 45.85 (2CH₃). |
| | | **HRMS (ESI):** m/z calculated for C₂₀H₂₁N₃NaO₂⁺ [M+Na]⁺: 358.1526; found 358.1524. |
| (12) | B, F, G | **NMR¹H** (400 **MHz, MeOD) :** δ 8.89 (dd, *J* = 4.2, 1.7 Hz, 1H), 8.85 (dd, *J* = 7.6, 1.4 Hz, 1H), 8.32 (dd, *J* = 8.3, 1.7 Hz, 1H), 8.06 (t, *J* = 8.2 Hz, 1H), 7.66 (dd, *J* = 8.3, 1.4 Hz, 1H), 7.63 - 7.47 (m, 2H), 7.34 - 7.18 (m, 2H), 3.01 (dt, *J* = 12.6, 3.3 Hz, 2H), 2.68 (tt, *J* = 11.8, 3.9 Hz, 1H), 2.34 (s, 3H), 2.17 (td, *J* = 12.7, 11.9, 3.4 Hz, 2H), 1.96 - 1.87 (m, 2H), 1.80 (dtd, *J* = 13.4, 12.1, 3.8 Hz, 2H). |
| | | **NMR¹³C (126 MHz, MeOD) :** δ 163.24 (CO), 163.01 - 161.04 (d, *J* = 248.30 Hz, CF), 155.07 - 155.01 (d, *J* = 8.50 Hz, C_{q}), 150.10 (CH_{Ar}), 140.01 (C_{q}), 137.63 (CH_{Ar}), 135.77 (C_{q}), 132.78 - 132.76 (d, *J* = 2.40 Hz, CH_{Ar}), 129.58 (C_{q}), 128.12 (CH_{Ar}), 124.78 - 124.76 (d, *J* = 2.73 Hz, CH_{Ar}), 123.69 (CH_{Ar}), 123.19 (CH_{Ar}), 120.75 - 120.65 (d, *J* = 12.32 Hz, C_{q}), 118.20 (CH_{Ar}), 115.86 - 115.67 (d, *J* = 24.64 Hz, CH_{Ar}), 56.79 (2CH₂), 46.37 (CH₃), 42.58 (CH), 33.59 (2CH₂). |
| | | **NMR ¹⁹F (376 MHz, MeOD) :** δ -114.04. |
| | | **HRMS (ESI):** m/z calculated for C₂₂H₂₃FN₃O⁺ [M+H]⁺: 364.1820; found 364.1827. |
| (13) | B, F, G | **NMR¹H (400 MHz, MeOD) :** δ 9.27 (d, *J* = 1.0 Hz, 1H), 8.45 (d, *J* = 6.1 Hz, 1H), 8.05 (dt, *J* = 8.3, 1.1 Hz, 1H), 7.95 - 7.82 (m, 3H), 7.79 - 7.67 (m, 2H), 7.50 (t, *J* = 7.7 Hz, 1H), 3.08 - 3.00 (m, 2H), 2.97 (ddt, *J* = 10.4, 8.2, 3.5 Hz, 1H), 2.35 (s, 3H), 2.27 - 2.14 (m, 2H), 1.88 (td, *J* = 9.1, 7.9, 3.5 Hz, 4H). |
| | | **NMR ¹³C (126 MHz, MeOD) :** δ 168.12 (d, *J* = 2.3 Hz, CO), 161.90 (d, *J* = 245.5 Hz, CF), 153.53 (CH_{Ar}), 143.09 (CH_{Ar}), 138.16 (d, *J* = 14.7 Hz, C_{q}), 135.23 (d, *J* = 7.3 Hz, C_{q}), 133.97 (C_{q}), 133.57 (C_{q}), 130.75 (C_{q}), 129.61 (CH_{Ar}), 129.35 (d, *J* = 4.9 Hz, CH_{Ar}), 128.70 (CH_{Ar}), 127.84 (CH_{Ar}), 124.95 (d, *J* = 3.3 Hz, CH_{Ar}), 117.83 (CH_{Ar}), 115.96 (d, *J* = 25.3 Hz, CH_{Ar}), 56.88 (2CH₂), 46.38 (CH₃), 36.19 (d, *J* = 1.9 Hz, CH), 32.44 (2CH₂). |
| | | **NMR¹⁹F (376 MHz, MeOD) :** δ -119.74. |
| | | **HRMS (ESI):** m/z calculated for C₂₂H₂₃FN₃O⁺ [M+H]⁺: 364.1820; found 364.1817. |
| (14) | D, F, G | **NMR¹H (400 MHz, MeOD) :** δ 9.28 (d, *J* = 1.0 Hz, 1H), 8.46 (d, *J* = 6.1 Hz, 1H), 8.12-8.02 (m, 3H), 7.90 (dd, *J* = 7.5, 1.1 Hz, 1H), 7.87 (dt, *J* = 6.0, 1.0 Hz, 1H), 7.73 (dd, *J* = 8.3, 7.4 Hz, 1H), 7.16-7.08 (m, 2H), 4.22 (t, *J* = 5.4 Hz, 2H), 2.85 (t, *J* = 5.4 Hz, 2H), 2.39 (s, 6H). |
| | | **NMR ¹³C (101 MHz, MeOD) :** δ 169.28 (CO), 163.46 (C_{q}), 153.52 (CH_{Ar}), 143.00 (CH_{Ar}), 134.39 (C_{q}), 133.77 (C_{q}), 130.94 (2CH_{Ar}), 130.81 (C_{q}), 129.64 (CH_{Ar}), 128.74 (CH_{Ar}), 127.65 (CH_{Ar}), 127.57 (C_{q}), 117.98 (CH_{Ar}), 115.53 (2CH_{Ar}), 66.83 (CH₂), 58.95 (CH₂), 45.81 (2CH₃). |
| | | **HRMS (ESI):** m/z calculated for C₂₀H₂₂N₃O₂⁺, [M+H]⁺ 336.1707, found 336.1707. |
| (15) | D, F, G | **NMR¹H (400 MHz, MeOD) :** δ 8.86 (dd, *J* = 4.3, 1.6 Hz, 1H), 8.43 (bd, *J =* 8.5, 1H), 8.05 (d, *J* = 8.9 Hz, 2H), 7.99 (dt, *J* = 8.5, 1.1 Hz, 1H), 7.80 (dd, *J* = 8.5, 7.4 Hz, 1H), 7.68 (dd, *J* = 7.4, 1.2 Hz, 1H), 7.54 (dd, *J* = 8.6, 4.3 Hz, 1H), 7.10 (d, *J* = 8.9 Hz, 2H), 4.22 (t, *J* = 5.4 Hz, 2H), 2.88 (t, *J* = 5.4 Hz, 2H), 2.41 (s, 6H). |
| | | **NMR ¹³C (101 MHz, MeOD) :** δ 169.36 (CO), 163.37 (C_{q}), 151.38 (CH_{Ar}), 149.28 (C_{q}), 135.39 (C_{q}), 134.14 (CH_{Ar}), 130.93 (2CH_{Ar}), 130.68 (CH_{Ar}), 127.98 (CH_{Ar}), 127.59 (C_{q}), 126.42 (C_{q}), 125.69 (CH_{Ar}), 122.43 (CH_{Ar}), 115.52 (2CH_{Ar}), 66.64 (CH₂), 58.87 (CH₂), 45.71 (2CH₃). |
| | | **HRMS (ESI):** m/z calculated for C₂₀H₂₂N₃O₂⁺, [M+H]⁺ 336.1707, found 336.1709. |
| (16) | B, F, G | **NMR¹H (400 MHz, MeOD) :** δ 8.81 (t, *J* = 1.8 Hz, 1H), 8.80-8.77 (m, 1H), 8.17 (dd, *J* = 8.3, 1.7 Hz, 1H), 8.05 (d, *J* = 8.0 Hz, 1H), 7.55-7.46 (m, 2H), 7.43 (dd, *J* = 8.3, 4.2 Hz, 1H), 6.96 (d, *J* = 1.5 Hz, 1H), 6.93 (dd, *J* = 8.2, 1.6 Hz, 1H), 4.11 (s, 3H), 2.97 (dt, *J* = 12.6, 3.1 Hz, 2H), 2.54 (tt, *J* = 11.6, 4.2 Hz, 1H), 2.31 (s, 3H), 2.12 (td, *J* = 11.8, 3.0 Hz, 2H), 1.88-1.69 (m, 4H). |
| | | **NMR ¹³C (101 MHz, MeOD) :** δ 165.17 (CO), 159.41 (C_{q}), 153.79 (C_{q}), 149.86 (CH_{Ar}), 140.31 (C_{q}), 137.34 (CH_{Ar}), 136.57 (C_{q}), 133.01 (CH_{Ar}), 129.54 (C_{q}), 128.10 (CH_{Ar}), 123.08 (CH_{Ar}), 122.91 (CH_{Ar}), 120.70 (C_{q}), 120.56 (CH_{Ar}), 118.04 (CH_{Ar}), 111.57 (CH_{Ar}), 56.81 (2CH₂), 56.72 (CH₃), 46.22 (CH₃), 42.84 (CH), 33.54 (2CH₂). |
| | | **HRMS (ESI):** m/z calculated for C₂₃H₂₆N₃O₂⁺, [M+H]⁺ 376.2020, found 376.2030. |
| (17) | B, F, G | **NMR¹H (400 MHz, MeOD) :** δ 9.27 (s, 1H), 8.45 (d, *J* = 6.0 Hz, 1H), 8.05 (d, *J* = 8.2 Hz, 1H), 7.91 (dd, *J* = 7.5, 1.1 Hz, 1H), 7.87 (d, *J* = 6.0 Hz, 1H), 7.73 (t, *J* = 7.8 Hz, 1H), 7.68 (dd, *J* = 7.9, 1.7 Hz, 1H), 7.62 (d, *J* = 1.8 Hz, 1H), 7.37 (d, *J* = 7.9 Hz, 1H), 3.93 (s, 3H), 3.13-3.00 (m, 3H), 2.38 (s, 3H), 2.26 (td, *J* = 11.4, 4.0 Hz, 2H), 1.91-1.75 (m, 4H). |
| | | **NMR ¹³C (101 MHz, MeOD) :** δ 169.54 (CO), 158.58 (C_{q}), 153.53 (CH_{Ar}), 143.04 (CH_{Ar}), 139.57 (C_{q}), 134.25 (CH_{Ar}), 134.23 (CH_{Ar}), 133.70 (C_{q}), 130.78 (C_{q}), 129.68 (C_{q}), 128.73 (CH_{Ar}), 127.77 (CH_{Ar}), 127.75 (CH_{Ar}), 121.25 (CH_{Ar}), 117.95 (C_{q}), 110.98 (CH_{Ar}), 57.12 (2CH₂), 56.10 (CH₃), 46.23 (CH₃), 35.95 (CH), 32.20 (2CH₂). |
| | | **HRMS (ESI):** m/z calculated for C₂₃H₂₆N₃O₂⁺, [M+H]⁺ 376.2020, found 376.2022. |
| (18) | B, F, G | **NMR¹H (400 MHz, MeOD) :** δ 8.81 (dd, *J* = 4.2, 1.7 Hz, 1H), 8.74 (dd, *J* = 7.6, 1.4 Hz, 1H), 8.32 (dd, *J* = 8.3, 1.7 Hz, 1H), 7.77 - 7.72 (m, 2H), 7.72 - 7.65 (m, 2H), 7.61 (dd, *J* = 8.3, 7.6 Hz, 1H), 7.55 (dd, *J* = 8.3, 4.3 Hz, 1H), 3.05 (dq, *J =* 11.5, 2.5, 1.7 Hz, 2H), 2.76 (tt, *J =* 12.0, 4.0 Hz, 1H), 2.37 (s, 3H), 2.24 (td, *J* = 12.0, 2.7 Hz, 2H), 1.98 - 1.90 (m, 2H), 1.84 (dtd, *J* = 13.2, 12.0, 3.8 Hz, 2H). |
| | | **NMR ¹³C (101 MHz, MeOD) :** δ 168.09 (C=O), 150.38 (C_{q}), 150.16 (CH_{Ar}), 139.97 (C_{q}), 137.75 (CH_{Ar}), 135.37 (C_{q}), 135.19 (C_{q}), 135.18 (C_{q}), 132.05 (CH_{Ar}), 130.01 (CH_{Ar}), 129.63 (C_{q}), 128.78 - 128.47 (d, *J* = 31.51 Hz, C-CF₃) , 128.03 (CH_{Ar}), 126.58 - 123.86 (d, *J* = 272.89 Hz, CF₃), 126.30 - 126.26 (d, *J* = 4.79 Hz, CH_{Ar}), 124.15 (CH_{Ar}), 123.20 (CH_{Ar}), 118.37 (CH_{Ar}), 56.76 (2CH₂), 46.26 (CH₃), 42.51 (CH), 33.62 (2CH₂). |
| | | **NMR¹⁹F (376 MHz, MeOD) :** δ -60.08. |
| | | **HRMS (ESI):** m/z calculated for C₂₃H₂₂F₃N₃NaO⁺ [M+Na]⁺: 436.1607; found 436.1623. |
| (19) | D, H | **NMR¹H (500 MHz, MeOD) :** δ 9.63 (s, 2H), 8.15 (dd, *J* = 6.5, 2.3 Hz, 1H), 8.12 - 8.05 (m, 4H), 7.15 (d, *J* = 9.0 Hz, 2H), 4.26 (t, *J* = 5.4 Hz, 2H), 2.93 (t, *J* = 5.4 Hz, 2H), 2.46 (s, 6H). |
| | | **NMR ¹³C (126 MHz, MeOD) :** δ 169.25 (CO), 163.56 (C_{q}), 152.55 (CH_{Ar}), 148.84 (CH_{Ar}), 135.58 (C_{q}), 134.76 (CH_{Ar}), 131.10 (2CH_{Ar}), 131.06 (CH_{Ar}), 128.84 (C_{q}), 127.29 (C_{q}), 125.89 (CH_{Ar}), 123.57 (C_{q}), 115.61 (2CH_{Ar}), 66.54 (CH₂), 58.84 (CH₂), 45.63 (2CH₃). |
| | | **HRMS (ESI):** m/z calculated for C₁₉H₂₁N₄O₂⁺ [M+H]⁺: 337.1659; found 337.1660. |
| (20) | D, H | **NMR¹H (400 MHz, MeOD) :** δ 8.91 (dd, *J* = 4.2, 1.5 Hz, 1H), 8.42 (dt, *J* = 8.7, 1.6 Hz, 1H), 8.04 (d, *J* = 8.8 Hz, 2H), 7.67-7.58 (m, 2H), 7.54 (dd, *J* = 10.4, 8.3 Hz, 1H), 7.10 (d, *J* = 8.9 Hz, 2H), 4.22 (t, *J* = 5.4 Hz, 2H), 2.85 (t, *J* = 5.4 Hz, 2H), 2.39 (s, 6H). |
| | | **NMR ¹³C (101 MHz, MeOD) :** δ = 169.47 (CO), 163.46 (C_{q}), 157.46 (d, *J* = 255.0 Hz, C_{q}), 151.60 (d, *J* = 1.6 Hz, CH_{Ar}), 139.08 (d, *J* = 12.9 Hz, C_{q}), 134.27 (d, *J* = 2.4 Hz, CH_{Ar}), 131.25 (d, *J* = 4.8 Hz, CH_{Ar}), 130.91 (2CH_{Ar}), 127.94 (d, *J* = 2.2 Hz, C_{q}), 127.37 (C_{q}), 125.95 (d, *J* = 7.9 Hz, C_{q}), 123.48 (CH_{Ar}), 115.52 (2CH_{Ar}), 114.39 (d, *J* = 20.2 Hz, CH_{Ar}), 66.79 (CH₂), 58.93 (CH₂), 45.79 (2CH₃). |
| | | **NMR ¹⁹F (376 MHz, MeOD, decoupled):** δ = -128.6 |
| | | **HRMS (ESI):** m/z calculated for C₂₀H₂₁FN₃O₂⁺ [M+H]⁺: 354.1612; found 354.1622. |
| (21) | B, H | **NMR ¹H (400 MHz, MeOD)** : δ 9.20 (s, 1H), 8.38 (d, *J* = 6.0 Hz, 1H), 8.32 (s, 1H), 8.24 (dd, *J* = 8.3, 2.0 Hz, 1H), 8.00 (dd, *J* = 8.2, 1.1 Hz, 1H), 7.86 (dd, *J* = 7.5, 1.3 Hz, 1H), 7.81 (d, *J* = 6.0 Hz, 1H), 7.68-7.62 (m, 2H), 3.17-3.00 (m, 3H), 2.40 (s, 3H), 2.39-2.30 (m, 2H), 1.98-1.77 (m, 4H). |
| | | **NMR ¹³C (101 MHz, MeOD) :** δ 167.34 (CO), 153.72 (C_{q}), 153.60 (CH_{Ar}), 143.19 (CH_{Ar}), 134.64 (C_{q}), 133.82 (Cq), 133.80 (CH_{Ar}), 133.76 (CH_{Ar}), 133.55 (C_{q}), 130.80 (C_{q}), 129.64 (CH_{Ar}), 128.75 (CH_{Ar}), 128.38 (CH_{Ar}), 128.02 (CH_{Ar}), 118.10 (C_{q}), 117.83 (Cq), 113.75 (CH_{Ar}), 56.46 (2CH₂), 45.81 (CH₃), 41.12 (CH), 32.54 (2CH₂). |
| | | **HRMS (ESI):** m/z calculated for C₂₃H₂₃N₄O⁺, [M+H]⁺ : 371.1866; found 371.1864. |
| (22) | A, D, H | **NMR ¹H (400 MHz, MeOD)** : δ 9.33 (s, 1H), 8.58 (d, *J* = 5.9 Hz, 1H), 8.07 (d, *J* = 8.8 Hz, 2H), 8.02 (dd, *J* = 5.9, 1.0 Hz, 1H), 7.70 (dd, *J* = 8.3, 4.7 Hz, 1H), 7.57 (dd, *J* = 9.8, 8.3 Hz, 1H), 7.13 (d, *J* = 8.9 Hz, 2H), 4.24 (t, *J* = 5.4 Hz, 2H), 2.89 (t, *J* = 5.4 Hz, 2H), 2.42 (s, 6H). |
| | | **NMR ¹³C (101 MHz, MeOD)** : δ = 169.59 (CO), 163.52 (C_{q}), 157.14 (d, *J* = 253.1 Hz, C_{q}), 149.53 (d, *J* = 2.2 Hz, CH_{Ar}), 143.67 (d, *J* = 1.6 Hz, CH_{Ar}), 132.31 (d, *J* = 4.6 Hz, C_{q}), 130.96 (2CH_{Ar}), 128.26 (d, *J* = 18.9 Hz, C_{q}), 127.34 (C_{q}), 126.95 (d, *J* = 7.9 Hz, CH_{Ar}), 126.60 (C_{q}), 115.59 (2CH_{Ar}), 115.56 (d, *J* = 20.6 Hz, CH_{Ar}), 114.94 (d, *J* = 3.7 Hz, CH_{Ar}), 66.71 (CH₂), 58.91 (CH₂), 45.73 (2CH₃). |
| | | **NMR ¹⁹F (376 MHz, MeOD, decoupled)** : δ = -126.6. |
| | | **HRMS (ESI):** m/z calculated for C₂₀H₂₁FN₃O₂⁺, [M+H]⁺ 354.1612, found 354.1614. |
| (23) | A, D, H | **NMR ¹H (400 MHz, MeOD)** : δ 8.97 (d, *J* = 1.8 Hz, 1H), 8.94 (d, *J* = 1.9 Hz, 1H), 8.69 (dd, J= 8.7, 4.8 Hz, 1H), 7.95 (d, *J* = 8.8 Hz, 2H), 7.56 (dd, *J* = 10.2, 8.8 Hz, 1H), 7.08 (d, *J* = 8.8 Hz, 2H), 4.20 (t, *J* = 5.4 Hz, 2H), 2.83 (t, *J* = 5.4 Hz, 2H), 2.38 (s, 6H). |
| | | **NMR ¹³C (101 MHz, MeOD)** : δ = 166.89 (CO), 163.52 (C_{q}), 153.92 (d, *J* = 255.8 Hz, C_{q}), 146.97 (d, *J* = 2.6 Hz, CH_{Ar}), 145.69 (CH_{Ar}), 135.83 (C_{q}), 134.24 (d, *J* =14.2 Hz, C_{q}), 132.46 (d, *J* =4.6 Hz, C_{q}), 130.29 (2CH_{Ar}), 127.75 (C_{q}), 118.52 (d, *J* = 7.4 Hz, CH_{Ar}), 115.74 (2CH_{Ar}), 115.49 (d, *J* = 19.2 Hz, CH_{Ar}), 66.89 (CH₂), 58.94 (CH₂), 45.82 (2CH₃). |
| | | **NMR ¹⁹F (376 MHz, MeOD, decoupled):** δ = -133.3. |
| | | **HRMS (ESI):** m/z calculated for G₁₉H₂₀FN₄O₂⁺, [M+H]⁺ 355.1565, found 355.1574. |
| (24) | C, F, G | **NMR ¹H (400 MHz, MeOD)** : δ 9.29 (d, *J* = 1.0 Hz, 1H), 8.47 (d, *J* = 6.1 Hz, 1H), 8.08 (dt, *J* = 8.3, 1.0 Hz, 1H), 7.97 - 7.83 (m, 3H), 7.83 - 7.63 (m, 2H), 7.17 (t, *J* = 8.6 Hz, 1H), 3.28 (t, *J* = 5.0 Hz, 4H), 2.67 (t, *J* = 4.9 Hz, 4H), 2.38 (s, 3H). |
| | | **NMR ¹³C (126 MHz, MeOD)** : δ 168.21 (CO), 155.99 (d, *J* = 245.7 Hz, CF), 153.54 (CH_{Ar}), 144.60 (d, *J* = 8.2 Hz, C_{q}), 143.05 (CH_{Ar}), 134.21 (C_{q}), 133.74 (C_{q}), 130.81 (C_{q}), 129.68 (CH_{Ar}), 128.74 (CH_{Ar}), 128.49 (d, *J* = 6.9 Hz, C_{q}), 127.77 (CH_{Ar}), 125.85 (d, *J* = 3.0 Hz, CH_{Ar}), 119.61 (d, *J* = 3.4 Hz, CH_{Ar}), 117.94 (CH_{Ar}), 116.84 (d, *J* = 23.3 Hz, CH_{Ar}), 55.91 (2CH₂), 50.63 (d, *J* = 4.3 Hz, 2CH₂), 46.12 (CH₃). |
| | | **NMR ¹⁹F (376 MHz, MeOD) :** δ -122.95. |
| | | **HRMS (ESI):** m/z calculated for C₂₁H₂₂FN₄O⁺ [M+H]⁺: 365.1772; found 365.1769. |
| (25) | B, F, G | **NMR ¹H (400 MHz, CDCl₃)** : δ 9.43 (s, 1H), 9.19 (s, 1H), 8.49 (d, *J* = 6.0 Hz, 1H), 8.14 (d, *J* = 7.4 Hz, 1H), 7.94 - 7.88 (m, 2H), 7.85 (d, *J* = 8.2 Hz, 1H), 7.73 (d, *J* = 7.7 Hz, 1H), 7.62 (t, *J* = 7.9 Hz, 1H), 7.53 (dd, *J* = 7.8, 1.8 Hz, 1H), 3.33 (s, 3H), 2.98 (d, *J* = 11.3 Hz, 2H), 2.58 (tt, *J* = 12.1, 4.2 Hz, 1H), 2.27 (s, 3H), 2.09 (td, *J* = 11.8, 2.7 Hz, 2H), 1.79 - 1.71 (m, 2H), 1.71 - 1.58 (m, 2H). |
| | | **NMR ¹³C (126 MHz, MeOD):** δ 170.16 (CO), 153.39 (CH_{Ar}), 150.18 (C_{q}), 143.08 (CH_{Ar}), 139.82 (Cq), 136.59 (Cq), 133.56 (Cq), 133.45 (Cq), 133.43 (CH_{Ar}), 130.73 (Cq), 130.37 (CH_{Ar}), 129.38 (CH_{Ar}), 129.29 (CH_{Ar}), 128.73 (CH_{Ar}), 127.81 (CH_{Ar}), 118.06 (CH_{Ar}), 56.59 (2CH₂), 45.95 (CH₃), 45.73 (CH₃), 42.18 (CH), 33.33 (2CH₂). |
| | | **HRMS (ESI):** m/z calculated for C₂₃H₂₆N₃O₃S⁺ [M+H]⁺: 424.1689; found 424.1686. |
| (26) | D, F, G | **NMR ¹H (500 MHz, MeOD)** : δ 8.96 (dd, *J* = 4.3, 1.7 Hz, 1H), 8.69 (dd, *J* = 8.6, 5.4 Hz, 1H), 8.50 (dd, *J* = 8.4, 1.7 Hz, 1H), 8.01 (d, *J* = 8.8 Hz, 2H), 7.66 (dd, *J* = 8.5, 4.2 Hz, 1H), 7.30 (dd, *J* = 9.9, 8.6 Hz, 1H), 7.20 - 7.12 (m, 2H), 4.35 (t, *J* = 5.2 Hz, 2H), 3.24 (t, *J* = 5.2 Hz, 2H), 2.69 (s, 6H). |
| | | **NMR ¹³C (126 MHz, MeOD)** : δ 166.69 (CO), 162.84 (C_{q}), 154.60 (d, *J* = 250.6 Hz, CF), 151.05 (CH_{Ar}), 140.50 (d, *J* = 3.1 Hz, C_{q}), 132.23 (d, *J* = 4.1 Hz, C_{q}), 130.76 (d, *J* = 3.9 Hz, CH_{Ar}), 130.26 (2CH_{Ar}), 128.65 (C_{q}), 123.44 (d, *J* = 2.7 Hz, CH_{Ar}), 120.07 (d, *J* = 18.2 Hz, C_{q}), 117.60 (d, *J* = 7.9 Hz, CH_{Ar}), 115.88 (2CH_{Ar}), 111.18 (d, *J* = 20.0 Hz, CH_{Ar}), 65.21 (CH₂), 58.35 (CH₂), 44.97 (2CH₃). |
| | | **¹⁹F NMR (376 MHz, MeOD)** : δ -130.44. |
| | | **HRMS (ESI):** m/z calculated for C₂₀H₂₁FN₃O₂⁺ [M+H]⁺: 354.1612; found 354.1625. |
| (27) | B, F, G | **NMR ¹H (500 MHz, MeOD)** : δ 8.87 (dd, *J* = 4.3, 1.6 Hz, 1H), 8.44 (dt, *J =* 8.5, 1.3 Hz, 1H), 8.38 (d, *J* = 1.9 Hz, 1H), 8.30 (dd, *J* = 8.2, 2.0 Hz, 1H), 8.01 (dt, *J* = 8.5, 1.1 Hz, 1H), 7.80 (td, *J* = 8.7, 7.6 Hz, 2H), 7.72 (dd, *J* = 7.5, 1.2 Hz, 1H), 7.55 (dd, *J* = 8.6, 4.3 Hz, 1H), 3.12 - 2.97 (m, 3H, CH2+CH), 2.38 (s, 3H), 2.31 - 2.15 (m, 2H), 1.95 (qd, J = 12.5, 3.7 Hz, 2H), 1.88 - 1.73 (m, 2H). |
| | | **NMR ¹³C (126 MHz, MeOD) :** δ 168.06 (CO), 151.46 (CH_{Ar}), 150.33 (C_{q}), 149.27 (C_{q}), 134.92 (C_{q}), 134.01 (CH_{Ar}), 133.68 (C_{q}), 132.67 (CH_{Ar}), 130.65 (CH_{Ar}), 130.06 (CH_{Ar}), 129.47 (q, *J* = 29.8 Hz, C_{q}-CF), 128.27 (CH_{Ar}), 129.11 - 124.48 (q, *J* = 274.46 Hz, CF3), 126.65 (q, *J* = 6.0 Hz, CH_{Ar}), 126.25 (CH_{Ar}), 125.72 (C_{q}), 122.54 (CH_{Ar}), 56.89 (2CH₂), 46.34 (CH₃), 39.14 (CH), 33.95 (2CH₂). |
| | | **NMR ¹⁹F (376 MHz, MeOD):** δ -60.32. |
| | | **HRMS (ESI):** m/z calculated for C₂₃H₂₃F₃N₃O⁺ [M+H]⁺: 414.1788; found 414.1785. |
| (28) | G | **NMR ¹H (400 MHz, MeOD)** : δ 8.96 (d, *J* = 1.9 Hz, 1H), 8.91 (d, *J* = 1.9 Hz, 1H), 8.83 (dd, *J* = 7.3, 1.8 Hz, 1H), 8.12 - 7.95 (m, 2H), 7.91 - 7.81 (m, 2H), 7.53 - 7.40 (m, 2H), 3.11 - 2.98 (m, 2H), 2.69 (tt, *J* = 11.6, 4.2 Hz, 1H), 2.37 (s, 3H), 2.24 (td, *J* = 11.9, 3.1 Hz, 2H), 1.99 - 1.62 (m, 4H). |
| | | **NMR ¹³C (101 MHz, MeOD):** δ 167.33 (CO), 151.99 (C_{q}), 147.07 (CH_{Ar}), 144.80 (CH_{Ar}), 143.88 (C_{q}), 135.95 (C_{q}), 135.31 (C_{q}), 133.75 (C_{q}), 132.05 (CH_{Ar}), 128.64 (2CH_{Ar}), 128.58 (2CH_{Ar}), 124.34 (CH_{Ar}), 118.82 (CH_{Ar}), 56.85 (2CH₂), 46.16 (CH₃), 42.64 (CH), 33.62 (2CH₂). |
| | | **HRMS (ESI):** m/z calculated for C₂₁H₂₃N₄O⁺ [M+H]⁺: 347.1866; found 347.1888. |
| (29) | C, H | **NMR ¹H (400 MHz, MeOD)** : δ 8.87 (dd, *J* = 4.3, 1.6 Hz, 1H), 8.43 (ddd, *J* = 8.6, 1.6, 0.9 Hz, 1H), 8.00 (dt, *J* = 8.6, 1.0 Hz, 1H), 7.94-7.87 (m, 2H), 7.82 (dd, *J* = 8.5, 7.4 Hz, 1H), 7.69 (dd, *J* = 7.4, 1.1 Hz, 1H), 7.56 (dd, *J* = 8.6, 4.3 Hz, 1H), 7.17 (d, *J* = 8.1 Hz, 1H), 3.06 (bt, *J* = 4.7 Hz, 4H), 2.69 (s, 4H), 2.40 (s, 6H). |
| | | **NMR ¹³C (101 MHz, MeOD):** δ 169.78 (CO), 156.38 (C_{q}), 151.39 (CH_{Ar}), 149.31 (C_{q}), 135.41 (C_{q}), 134.16 (CH_{Ar}), 133.53 (CH_{Ar}), 131.94 (C_{q}), 130.69 (CH_{Ar}), 129.33 (C_{q}), 127.98 (CH_{Ar}), 127.75 (CH_{Ar}), 126.44 (C_{q}), 125.71 (CH_{Ar}), 122.44 (CH_{Ar}), 119.74 (CH_{Ar}), 56.32 (2CH₂), 51.97 (2CH₂), 46.10 (CH₃), 18.51 (CH₃). |
| | | **HRMS (ESI):** m/z calculated for C₂₂H₂₅N₄O⁺, [M+H]⁺ : 361.2023, found 361.2023. |
| (30) | G | **NMR ¹H (400 MHz, MeOD)** : δ 8.96 (dd, *J* = 4.2, 1.6 Hz, 1H), 8.46 (dd, *J =* 8.6, 1.6 Hz, 1H), 8.13 - 7.98 (m, 2H), 7.94 (d, *J* = 8.1 Hz, 1H), 7.67 (d, *J* = 8.1 Hz, 1H), 7.63 (dd, *J* = 8.6, 4.2 Hz, 1H), 7.44 (d, *J* = 8.4 Hz, 2H), 3.26 (dt, *J* = 12.8, 2.7 Hz, 4H), 2.80 (tt, *J* = 11.6, 4.3 Hz, 1H), 2.65 - 2.46 (m, 5H ; CH₂ + CH₃), 2.14 - 1.81 (m, 4H). |
| | | **NMR ¹³C (101 MHz, MeOD) :** δ 169.70 (CO), 152.09 (C_{q}), 151.99 (CH_{Ar}), 145.35 (C_{q}), 134.53 (CH_{Ar}), 134.50 (C_{q}), 133.16 (C_{q}), 132.23 (C_{q}), 130.56 (CH_{Ar}), 129.22 (CH_{Ar}), 128.30 (CH_{Ar}), 127.62 (C_{q}), 125.69 (CH_{Ar}), 123.27 (CH_{Ar}), 56.91 (2CH₂), 46.28 (CH3), 42.77 (CH), 33.75 (2CH₂). |
| | | HRMS (ESI): m/z calculated for C₂₂H₂₃ClN₃O⁺, [M+H]⁺ : 380.1524, found 380.1544. |
| (31) | D, F, G | **NMR ¹H (500 MHz, MeOD)** : δ 8.93 (d, *J* = 1.9 Hz, 1H), 8.88 (d, *J* = 1.9 Hz, 1H), 8.77 (dd, *J* = 7.5, 1.5 Hz, 1H), 8.04 - 7.95 (m, 2H), 7.86 - 7.71 (m, 2H), 7.19 - 7.03 (m, 2H), 4.21 (t, *J* = 5.4 Hz, 2H), 2.82 (t, *J* = 5.4 Hz, 2H), 2.37 (s, 6H). |
| | | **NMR ¹³C (126 MHz, MeOD) :** δ 166.93 (CO), 163.56 (C_{q}), 147.00 (CH_{Ar}), 144.70 (CH_{Ar}), 143.83 (C_{q}), 136.02 (C_{q}), 135.22 (C_{q}), 132.04 (CH_{Ar}), 130.31 (CH_{Ar}), 127.88 (C_{q}), 124.08 (CH_{Ar}), 118.61 (CH_{Ar}), 115.78 (CH_{Ar}), 66.94 (CH₂), 58.95 (CH₂), 45.84 (2CH₃). |
| | | **HRMS (ESI):** m/z calculated for C₁₉H₂₁N₄O₂⁺, [M+H]⁺ : 337.1659, found 337.1671. |
| (32) | D, F, G | **NMR ¹H (500 MHz, MeOD)** : δ 8.95 (dd, *J* = 4.2, 1.6 Hz, 1H), 8.44 (dd, *J* = 8.6, 1.7 Hz, 1H), 8.03 (d, *J* = 8.8 Hz, 2H), 7.91 (d, *J* = 8.1 Hz, 1H), 7.64 (d, *J* = 8.1 Hz, 1H), 7.60 (dd, *J* = 8.6, 4.2 Hz, 1H), 7.09 (d, *J* = 8.9 Hz, 2H), 4.21 (t, *J* = 5.4 Hz, 2H), 2.84 (t, *J* = 5.4 Hz, 2H), 2.39 (s, 6H). |
| | | **NMR ¹³C (126 MHz, MeOD)** : δ 169.27 (CO), 163.46 (C_{q}), 152.02 (CH_{Ar}), 145.28 (C_{q}), 134.63 (C_{q}), 134.50 (CH_{Ar}), 132.06 (C_{q}), 130.93 (2CH_{Ar}), 130.52 (CH_{Ar}), 127.60 (C_{q}), 127.35 (C_{q}), 125.60 (CH_{Ar}), 123.18 (CH_{Ar}), 115.51 (2CH_{Ar}), 66.78 (CH₂), 58.92 (CH₂), 45.78 (2CH₃). |
| | | **HRMS (ESI):** m/z calculated for C₂₀H₂₁ClN₃O₂⁺, [M+H]⁺ : 370.1317, found 370.1328. |
| (33) | A, D, F, G | **NMR ¹H (400 MHz, MeOD)** : δ 9.49 (d, *J* = 1.0 Hz, 1H), 8.57 (d, *J* = 6.1 Hz, 1H), 8.12 - 7.96 (m, 2H), 7.90 - 7.73 (m, 2H), 7.44 (dd, *J* = 10.0, 8.3 Hz, 1H), 7.12 (d, *J* = 8.9 Hz, 2H), 4.22 (t, *J* = 5.4 Hz, 2H), 2.84 (t, *J* = 5.4 Hz, 2H), 2.38 (s, 6H). |
| | | **NMR ¹⁹F (376 MHz, MeOD):** δ -126.54. |
| | | **NMR ¹³C (101 MHz, MeOD):** δ 169.41 (CO), 163.52 (C_{q}), 158.78 (d, *J* = 255.2 Hz, CF), 146.90 (d, *J* = 5.2 Hz, CH_{Ar}), 144.34 (CH_{Ar}), 135.02 (d, *J* = 3.9 Hz, C_{q}), 130.92 (2CH_{Ar}), 130.75 (d, *J* = 4.3 Hz, C_{q}), 130.60 (d, *J* = 8.8 Hz, CH_{Ar}), 127.37 (C_{q}), 120.63 (d, *J* = 16.4 Hz, C_{q}), 117.96 (CH_{Ar}), 115.54 (2CH_{Ar}), 112.38 (d, *J* = 20.4 Hz, CH_{Ar}), 66.90 (CH₂), 58.98 (CH₂), 45.83 (2CH₃). |
| | | **HRMS (ESI):** m/z calculated for C₂₀H₂₁FN₃O₂⁺, [M+H]⁺ : 354.1612, found 354.1616. |
| (34) | D, F, G | **NMR ¹H (400 MHz, MeOD):** δ 9.52 (d, *J* = 1.0 Hz, 1H), 8.45 (d, *J* = 6.0 Hz, 1H), 8.03 (d, *J* = 8.8 Hz, 2H), 7.78 - 7.69 (m, 2H), 7.10 (dd, *J* = 8.6, 3.9 Hz, 3H), 4.21 (t, *J* = 5.4 Hz, 2H), 4.08 (s, 3H), 2.82 (t, *J* = 5.4 Hz, 2H), 2.37 (s, 6H). |
| | | **NMR ¹³C (126 MHz, MeOD):** δ 168.18 (C_{q}), 162.00 (C_{q}), 155.39 (C_{q}), 146.80 (CH_{Ar}), 142.31 (CH_{Ar}), 133.78 (C_{q}), 130.05 (C_{q}), 129.43 (2CH_{Ar}), 126.15 (CH_{Ar}), 125.08 (C_{q}), 120.82 (CH_{Ar}), 116.32 (CH_{Ar}), 114.09 (2CH_{Ar}), 105.27 (CH_{Ar}), 65.51 (CH₂), 57.59 (CH₂), 55.19 (CH₃), 44.46 (2CH₃). |
| | | **HRMS (ESI):** m/z calculated for C₂₁H₂₄N₃O₃⁺, [M+H]⁺ 366.1812, found 366.1818. |
| (35) | H | **NMR ¹H (500 MHz, MeOD)** : δ 8.91 (dd, *J* = 4.2, 1.7 Hz, 1H), 8.76 (dd, *J* = 7.5, 1.4 Hz, 1H), 8.33 (dd, *J* = 8.3, 1.7 Hz, 1H), 7.95 (d, *J* = 8.9 Hz, 2H), 7.63 (dd, *J* = 8.3, 1.5 Hz, 1H), 7.61 - 7.54 (m, 2H), 7.20 - 6.85 (m, 2H), 3.44 - 3.36 (m, 4H), 2.62 (t, *J* = 5.1 Hz, 4H), 2.36 (s, 3H). |
| | | **NMR ¹³C (126 MHz, MeOD)** : δ 167.26 (CO), 155.24 (C_{q}), 149.96 (CH_{Ar}), 140.17 (C_{q}), 137.80 (CH_{Ar}), 135.72 (C_{q}), 129.79 (2CH_{Ar}), 129.67 (C_{q}), 128.18 (CH_{Ar}), 125.17 (C_{q}), 123.16 (CH_{Ar}), 123.13 (CH_{Ar}), 117.63 (CH_{Ar}), 115.54 (2CH_{Ar}), 55.75 (2CH₂), 48.29 (2CH₂), 46.12 (CH₃). |
| | | **HRMS (ESI):** m/z calculated for C₂₁H₂₃N₄O⁺, [M+H]⁺ 347.1866, found 347.1887. |
| (36) | D, F, G | **NMR ¹H (500 MHz, MeOD):** δ 9.34 (s, 1H), 8.48 (d, *J* = 5.8 Hz, 1H), 8.43 - 8.34 (m, 2H), 7.94 - 7.87 (m, 2H), 7.84 (dd, *J* = 8.3, 7.3 Hz, 1H), 7.78 (dd, *J* = 7.4, 1.2 Hz, 1H), 7.40 (d, *J* = 8.8 Hz, 1H), 4.41 (t, *J* = 5.3 Hz, 2H), 2.97 (t, *J* = 5.3 Hz, 2H), 2.47 (s, 6H), 2.01 (s, 3H). |
| | | **NMR ¹³C (126 MHz, MeOD):** δ 167.40 (C_{q}), 164.34 (C_{q}), 149.24 (CH_{Ar}), 143.03 (CH_{Ar}), 138.42 (Cq), 135.90 (CH_{Ar}), 135.70 (C_{q}), 134.91 (CH_{Ar}), 132.25 (CH_{Ar}), 128.27 (C_{q}), 126.70 (CH_{Ar}), 126.50 (CH_{Ar}), 125.68 (C_{q}), 122.47 (CH_{Ar}), 116.59 (CN), 113.85 (CH_{Ar}), 103.15 (C_{q}), 69.01 (CH₂), 58.51 (CH₂), 46.16 (2CH₃). |
| | | **HRMS (ESI):** m/z calculated for C₂₁H₂₁N₄O₂⁺, [M+H]⁺ 361.1659, found 361.1663. |
| (37) | A, H | **NMR ¹H (500 MHz, MeOD)** : δ 9.09 - 8.83 (m, 2H), 8.75 (dd, *J* = 8.7, 4.8 Hz, 1H), 7.93 (d, *J* = 8.9 Hz, 2H), 7.60 (dd, *J* = 10.1, 8.8 Hz, 1H), 7.07 (d, *J* = 9.0 Hz, 2H), 3.53 - 3.35 (m, 4H), 2.74 - 2.56 (m, 4H), 2.37 (s, 3H). |
| | | **NMR ¹³C (126 MHz, MeOD)** : δ 167.32 (CO), 155.32 (C_{q}), 153.83 (d, *J =* 255.6 Hz, CF), 146.97 (CH_{Ar}), 145.67 (CH_{Ar}), 135.89 (C_{q}), 134.31 (d, *J* = 13.8 Hz, C_{q}), 132.70 (d, *J* = 4.5 Hz, C_{q}), 129.89 (2CH_{Ar}), 124.63 (C_{q}), 118.38 (d, *J* = 7.3 Hz, CH_{Ar}), 115.55 (d, *J* = 19.1 Hz, CH_{Ar}), 115.44 (2CH_{Ar}), 55.75 (2CH₂), 48.24 (2CH₂), 46.12 (CH₃). |
| | | **NMR ¹⁹F (376 MHz, MeOD)** : δ -133.75. |
| | | **HRMS (ESI):** m/z calculated for C₂₀H₂₁FN₅O⁺, [M+H]⁺ 366.1725; found 366.1741. |
| (38) | H | **NMR ¹H (500 MHz, MeOD)** : δ 9.32 (s, 1H), 8.46 (d, *J* = 5.9 Hz, 1H), 8.00 (d, *J* = 8.9 Hz, 2H), 7.90 - 7.84 (m, 2H), 7.81 (t, *J* = 7.8 Hz, 1H), 7.73 (dd, *J* = 7.3, 1.2 Hz, 1H), 7.07 (d, *J* = 9.0 Hz, 2H), 3.46 - 3.37 (m, 4H), 2.67 (t, *J* = 5.1 Hz, 4H), 2.40 (s, 3H). |
| | | **NMR ¹³C (126 MHz, MeOD) :** δ 169.67 (CO), 155.20 (C_{q}), 149.37 (CH_{Ar}), 142.86 (CH_{Ar}), 138.38 (C_{q}), 136.29 (C_{q}), 132.25 (CH_{Ar}), 130.52 (CH_{Ar}), 126.44 (CH_{Ar}), 126.28 (CH_{Ar}), 124.47 (C_{q}), 122.45 (CH_{Ar}), 115.36 (C_{q}), 115.21 (CH_{Ar}), 55.66 (2CH₂), 48.20 (2CH₂), 45.96 (CH₃). |
| | | **HRMS (ESI):** m/z calculated for C₂₁H₂₃N₄O⁺, [M+H]⁺ 347.1866; found 347.1879. |
| (39) | H | **NMR ¹H (400 MHz, MeOD)** : δ 9.32 - 9.22 (m, 1H), 8.45 (d, *J* = 6.0 Hz, 1H), 8.04 (dd, *J* = 8.3, 1.0 Hz, 1H), 7.99 (d, *J* = 8.9 Hz, 2H), 7.92 - 7.84 (m, 2H), 7.73 (dd, *J* = 8.3, 7.5 Hz, 1H), 7.07 (d, *J* = 9.0 Hz, 2H), 3.47 - 3.37 (m, 4H), 2.66 (t, *J* = 5.1 Hz, 4H), 2.39 (s, 3H). |
| | | **NMR ¹³C (101 MHz, MeOD) :** δ 169.50 (CO), 155.20 (C_{q}), 153.49 (CH_{Ar}), 142.92 (CH_{Ar}), 134.55 (Cq), 133.81 (Cq), 130.81 (Cq), 130.50 (2CH_{Ar}), 129.57 (CH_{Ar}), 128.74 (CH_{Ar}), 127.49 (CH_{Ar}), 124.57 (Cq), 118.05 (CH_{Ar}), 115.35 (2CH_{Ar}), 55.71 (2CH₂), 48.26 (2CH₂), 46.03 (CH₃). |
| | | **HRMS (ESI):** m/z calculated for C₂₁H₂₃N₄O⁺, [M+H]⁺ 347.1866; found 347.1876. |
| (40) | H | **NMR ¹H (400 MHz, MeOD)** : δ 8.87 (dd, *J* = 4.3, 1.7 Hz, 1H), 8.44 (dd, *J =* 8.5, 1.2 Hz, 1H), 8.05 - 7.91 (m, 3H), 7.82 (dd, *J* = 8.6, 7.4 Hz, 1H), 7.69 (dd, *J* = 7.4, 1.2 Hz, 1H), 7.56 (dd, *J* = 8.6, 4.3 Hz, 1H), 7.08 (d, *J* = 9.1 Hz, 2H), 3.40 (t, *J* = 5.1 Hz, 4H), 2.82 - 2.54 (m, 4H), 2.38 (s, 31H). |
| | | **NMR ¹³C (101 MHz, MeOD) :** δ 169.67 (CO), 155.26 (C_{q}), 151.35 (CH_{Ar}), 149.32 (C_{q}), 135.60 (C_{q}), 134.24 (CH_{Ar}), 130.69 (CH_{Ar}), 130.48 (2CH_{Ar}), 127.85 (CH_{Ar}), 126.53 (Cq), 125.68 (CH_{Ar}), 124.54 (Cq), 122.39 (CH_{Ar}), 115.35 (2CH_{Ar}), 55.76 (2CH₂), 48.33 (2CH₂), 46.09 (CH₃). |
| | | **HRMS (ESI):** m/z calculated for C₂₁H₂₃N₄O⁺, [M+H]⁺ 347.1866; found 347.1876. |
| (41) | H | **NMR ¹H (500 MHz, MeOD, CD₃CN)** : δ 8.98 (d, *J* = 1.8 Hz, 1H), 8.92 (d, *J* = 1.8 Hz, 1H), 8.83 (dd, *J* = 7.6, 1.4 Hz, 1H), 7.97 (d, *J* = 8.9 Hz, 2H), 7.91 - 7.85 (m, 1H), 7.82 (dd, *J* = 8.5, 1.4 Hz, 1H), 7.10 (d, *J* = 9.0 Hz, 2H), 3.41 (t, *J* = 5.2 Hz, 4H), 2.62 (t, *J* = 5.1 Hz, 4H), 2.37 (s, 3H). |
| | | **NMR ¹³C (126 MHz, MeOD, CD₃CN) :** δ 166.97 (CO), 155.17 (C_{q}), 147.06 (CH_{Ar}), 144.60 (CH_{Ar}), 143.80 (C_{q}), 136.16 (C_{q}), 135.03 (C_{q}), 132.08 (CH_{Ar}), 129.82 (2CH_{Ar}), 124.61 (C_{q}), 123.76 (CH_{Ar}), 118.19 (CH_{Ar}), 115.38 (2CH_{Ar}), 55.60 (2CH₂), 48.12 (2CH₂), 46.07 (CH₃). |
| | | **HRMS (ESI):** m/z calculated for C₂₀H₂₂N₅O⁺, [M+H]⁺ 348.1819; found 348.1835. |
| (42) | H | **NMR ¹H (400 MHz, MeOD)** : δ 8.91 (dt, *J* = 3.0, 1.5 Hz, 1H), 8.65 (ddd, *J =* 8.7, 5.4, 1.2 Hz, 1H), 8.44 (dt, *J* = 8.4, 1.6 Hz, 1H), 7.97 - 7.80 (m, 2H), 7.61 (ddd, *J* = 8.5, 4.3, 1.3 Hz, 1H), 7.25 (ddd, *J* = 10.1, 8.6, 1.4 Hz, 1H), 6.99 (d, *J* = 1.9 Hz, 2H), 3.35 (t, *J* = 5.1 Hz, 4H), 2.60 (t, *J* = 5.1 Hz, 4H), 2.35 (s, 3H). |
| | | **NMR ¹³C (126 MHz, MeOD)** : δ 166.93 (CO), 155.10 (C_{q}), 154.33 (d, *J =* 250.2 Hz, CF), 150.88 (CH_{Ar}), 140.32 (d, *J* = 3.2 Hz, C_{q}), 132.37 (d, *J* = 4.1 Hz, C_{q}), 130.66 (d, *J* = 3.9 Hz, CH_{Ar}), 129.70 (2CH_{Ar}), 124.91 (C_{q}), 123.34 (d, *J* = 2.6 Hz, CH_{Ar}), 120.00 (d, *J* = 18.2 Hz, C_{q}), 117.13 (d, *J* = 7.8 Hz, CH_{Ar}), 115.42 (2CH_{Ar}), 111.17 (d, *J* = 19.9 Hz, CH_{Ar}), 55.71 (2CH₂), 48.21 (2CH₂), 46.09 (CH₃). |
| | | **NMR ¹⁹F (376 MHz, MeOD)** : δ -130.96. |
| | | **HRMS (ESI):** m/z calculated for C₂₁H₂₂FN₄O⁺, [M+H]⁺ 365.1772; found 365.1790. |
| (43) | D, H | **NMR ¹H (500 MHz, MeOD):** δ 8.88 (dd, *J* = 4.2, 1.7 Hz, 1H), 8.72 (dd, *J =* 7.6, 1.4 Hz, 1H), 8.30 (dd, *J* = 8.3, 1.7 Hz, 1H), 7.89 (dd, *J* = 8.5, 2.4 Hz, 1H), 7.83 (dd, *J* = 2.5, 0.9 Hz, 1H), 7.63 (dd, *J* = 8.3, 1.3 Hz, 1H), 7.60 - 7.52 (m, 2H), 7.13 (d, *J* = 8.5 Hz, 1H), 4.53 - 4.46 (m, 2H), 3.73 - 3.67 (m, 2H), 3.06 (s, 6H), 2.36 (s, 3H). |
| | | **NMR ¹³C (126 MHz, MeOD):** δ 166.86 (C_{q}), 160.41 (C_{q}), 150.03 (CH_{Ar}), 140.08 (C_{q}), 137.80 (CH_{Ar}), 135.47 (C_{q}), 130.93 (CH_{Ar}), 129.61 (C_{q}), 128.81 (C_{q}), 128.56 (C_{q}), 128.09 (CH_{Ar}), 127.87 (CH_{Ar}), 123.43 (CH_{Ar}), 123.19 (CH_{Ar}), 117.80 (CH_{Ar}), 112.24 (CH_{Ar}), 64.07 (CH₂), 57.90 (CH₂), 47.88 (CH₃), 16.71 (2CH₃). |
| | | **HRMS (ESI):** m/z calculated for C₂₁H₂₄N₃O₂⁺, [M+H]⁺ 350.1863; found 350.1879. |
| (44) | H | **NMR ¹H (500 MHz, MeOD)** : δ 8.92 (dd, *J* = 4.3, 1.6 Hz, 1H), 8.42 (dt, *J* = 8.6, 1.6 Hz, 1H), 7.98 (d, *J* = 8.9 Hz, 2H), 7.63 (ddd, *J* = 11.2, 8.5, 4.4 Hz, 2H), 7.55 (dd, *J* = 10.4, 8.3 Hz, 1H), 7.17 - 6.96 (m, 2H), 3.40 (dd, *J* = 6.0, 4.3 Hz, 4H), 2.64 (t, *J* = 5.1 Hz, 4H), 2.38 (s, 3H). |
| | | **NMR ¹³C (126 MHz, MeOD)** : δ 169.77 (CO), 157.42 (d, *J* = 254.6 Hz, CF), 155.28 (C_{q}), 151.58 (d, *J* = 1.7 Hz, CH_{Ar}), 139.10 (d, *J* = 12.8 Hz, C_{q}), 134.39 (d, *J* = 2.4 Hz, CH_{Ar}), 131.45 (d, *J* = 4.7 Hz, C_{q}), 130.47 (2CH_{Ar}), 128.05 (d, *J* = 2.2 Hz, C_{q}), 125.95 (d, *J* = 8.0 Hz, CH_{Ar}), 124.34 (C_{q}), 123.45 (CH_{Ar}), 115.33 (2CH_{Ar}), 114.40 (d, *J* = 20.0 Hz, CH_{Ar}), 55.75 (2CH₂), 48.30 (2CH₂), 46.08 (CH₃). |
| | | **NMR ¹⁹F (376 MHz, MeOD)** : δ -128.91. |
| | | **HRMS (ESI):** m/z calculated for C₂₁H₂₂FN₄O⁺, [M+H]⁺ 365.1772; found 365.1790. |
| (45) | C, H | **NMR ¹H (500 MHz, CDCl₃)** : δ 9.27 (s, 1H, CONH), 8.55 (d, *J* = 6.0 Hz, 1H), 8.33 (d, *J* = 7.6 Hz, 1H), 7.88 (s, 1H), 7.83 (dt, *J* = 8.1, 1.0 Hz, 1H), 7.69 - 7.63 (m, 2H), 7.63 - 7.53 (m, 1H), 6.80 (d, *J* = 7.7 Hz, 2H), 3.33 (dd, *J* = 6.3, 3.9 Hz, 4H), 2.61 - 2.58 (m, 4H), 2.58 (s, 3H), 2.37 (s, 3H). |
| | | **NMR ¹³C (126 MHz, CDCl₃) :** δ 168.37 (CO), 153.31 (CH_{Ar}), 152.88 (C_{q}), 143.38 (CH_{Ar}), 139.44 (C_{q}), 132.44 (C_{q}), 129.70 (C_{q}), 129.28 (C_{q}), 128.73 (CH_{Ar}), 127.61 (CH_{Ar}), 125.57 (C_{q}), 124.92 (CH_{Ar}), 124.17 (CH_{Ar}), 117.95 (CH_{Ar}), 114.01 (CH_{Ar}), 112.26 (CH_{Ar}), 54.97 (2CH₂), 48.03 (2CH₂), 46.24 (CH₃), 21.22 (CH₃). |
| | | **HRMS (ESI):** m/z calculated for C₂₂H₂₅N₄O⁺, [M+H]⁺ 361.2023; found 361.2030. |
| (46) | H | **NMR ¹H (400 MHz, MeOD)** : δ 8.97 (dd, *J* = 4.2, 1.6 Hz, 1H), 8.46 (dd, *J =* 8.6, 1.6 Hz, 1H), 7.96 (dd, *J* = 14.5, 8.5 Hz, 3H), 7.67 - 7.52 (m, 2H), 7.09 - 6.91 (m, 2H), 3.40 (t, *J* = 5.1 Hz, 4H), 2.64 (t, *J* = 5.1 Hz, 4H), 2.38 (s, 3H). |
| | | **NMR ¹³C (126 MHz, MeOD) :** δ 169.61 (CO), 155.30 (C_{q}), 152.03 (CH_{Ar}), 145.34 (C_{q}), 134.88 (C_{q}), 134.66 (CH_{Ar}), 131.96 (C_{q}), 130.56 (CH_{Ar}), 130.51 (2CH_{Ar}), 127.76 (C_{q}), 125.64 (CH_{Ar}), 124.30 (C_{q}), 123.17 (CH_{Ar}), 115.31 (2CH_{Ar}), 55.75 (2CH₂), 48.28 (2CH₂), 46.09 (CH₃). |
| | | **HRMS (ESI):** m/z calculated for C₂₁H₂₂ClN₄O⁺, [M+H]⁺ 381.1477; found 381.1489. |
| (47) | D, H | **NMR ¹H (500 MHz, MeOD):** δ 8.88 (dd, *J* = 4.2, 1.7 Hz, 1H), 8.51 (d, *J =* 8.5 Hz, 1H), 7.98 (d, *J* = 8.4 Hz, 1H), 7.81 (t, *J* = 7.9 Hz, 1H), 7.77 (d, *J* = 7.4 Hz, 1H), 7.69 (d, *J* = 8.2 Hz, 1H), 7.58 (dd, *J* = 8.6, 4.3 Hz, 1H), 6.92 (d, *J* = 8.8 Hz, 2H), 4.18 (t, *J* = 5.4 Hz, 2H), 2.83 (t, *J* = 5.4 Hz, 2H), 2.54 (s, 3H), 2.38 (s, 6H). |
| | | **NMR ¹³C (126 MHz, MeOD):** δ 170.79 (C_{q}), 160.29 (C_{q}), 150.00, 147.89 (C_{q}), 138.84 (C_{q}), 133.72 (C_{q}), 132.49 (CH_{Ar}), 129.32 (CH_{Ar}), 129.15 (CH_{Ar}), 128.32 (C_{q}), 126.40 (CH_{Ar}), 124.54 (C_{q}), 123.72 (CH_{Ar}), 121.06 (CH_{Ar}), 116.74 (CH_{Ar}), 111.27 (CH_{Ar}), 65.21 (CH₂), 57.59 (CH₂), 44.41 (CH₃), 19.12 (2CH₃). |
| | | **HRMS (ESI):** m/z calculated for C₂₁H₂₄N₃O₂⁺, [M+H]⁺ 350.1863, found 350.1873. |
| (48) | D, H | **NMR ¹H (500 MHz, MeOD):** δ 9.27 (d, *J* = 1.0 Hz, 1H), 8.45 (d, *J* = 6.0 Hz, 1H), 8.36 (d, *J* = 2.3 Hz, 1H), 8.33 (dd, *J* = 8.7, 2.4 Hz, 1H), 8.05 (d, *J* = 8.3 Hz, 1H), 7.92 - 7.80 (m, 2H), 7.72 (dd, *J* = 8.3, 7.4 Hz, 1H), 7.36 (d, *J* = 8.7 Hz, 1H), 4.35 (t, *J* = 5.4 Hz, 2H), 2.90 (t, *J* = 5.4 Hz, 2H), 2.41 (s, 6H). |
| | | **NMR ¹³C (126 MHz, MeOD):** δ 170.43 (C_{q}), 163.32 (C_{q}), 156.00 (CH_{Ar}, d, *J* = 20.1 Hz), 145.5 (CH_{Ar}, d, *J* = 15.8 Hz), 137.48 (CH_{Ar}, d, *J* = 10.8 Hz), 136.63 (C_{q}), 136.21 (C_{q}), 133.31 (C_{q}), 132.24 (C_{q}), 131.26 (CH_{Ar}), 130.76 (CH_{Ar}, m, *J* = 14.5 Hz), 130.39 (CH_{Ar}), 129.80 (CH_{Ar}), 126.3 (CF₃, q, *J* = 272 Hz), 122.5 (C_{q}-CF₃, q, *J* = 31.3 Hz) 120.46 (CH_{Ar}), 116.77 (CH_{Ar}), 71.52 (CH₂), 61.17 (CH₂), 48.67 (2CH₃). |
| | | **NMR ¹⁹F (471 MHz, MeOD, decoupled):** δ -59.75. |
| | | **HRMS (ESI):** m/z calculated for C₂₁H₂₁F₃N₃O₂⁺, [M+H]⁺ 404.1580, found 404.1595. |
| (49) | D, H | **NMR ¹H (500 MHz, MeOD):** δ 8.89 (dd, *J* = 4.3, 1.6 Hz, 1H), 8.54 (d, *J =* 8.2 Hz, 1H), 8.00 (dd, *J =* 6.9, 2.0 Hz, 1H), 7.83 (dd, *J =* 8.2, 2.3 Hz, 3H), 7.59 (dd, *J* = 8.6, 4.3 Hz, 1H), 7.43 - 7.31 (m, 2H), 4.25 (t, *J* = 5.4 Hz, 2H), 2.85 (t, *J* = 5.3 Hz, 2H), 2.38 (s, 6H). |
| | | **NMR ¹³C (126 MHz, MeOD):** δ 168.60 (C_{q}), 159.90 (C_{q}), 150.07 (CH_{Ar}), 147.84 (C_{q}), 133.13 (C_{q}), 132.17 (CH_{Ar}), 130.33 (CH_{Ar}), 129.32 (CH_{Ar}), 128.64 (C-CF₃, q, *J* = 32.1 Hz), 127.87 (C_{q}), 126.59 (CH_{Ar}), 124.32 (C_{q}), 123.73 (CF₃, q, *J* = 272 Hz), 123.53 (CH_{Ar}), 121.12 (CH_{Ar}), 117.08 (CH_{Ar}), 113.02 (CH_{Ar}, d, *J* = 5.1 Hz), 65.86 (CH₂), 57.46 (CH₂), 44.41 (2CH₃). |
| | | **NMR ¹⁹F (471 MHz, MeOD, decoupled):** δ -60.36. |
| | | **HRMS (ESI):** m/z calculated for C₂₁H₂₁F₃N₃O₂⁺, [M+H]⁺ 404.1580, found 404.1595. |
| (50) | C, H | **NMR ¹H (400 MHz, MeOD)** : δ 8.87 (dd, *J* = 4.3, 1.7 Hz, 1H), 8.43 (ddd, *J* = 8.5, 1.7, 0.9 Hz, 1H), 8.00 (dt, *J* = 8.5, 1.0 Hz, 1H), 7.82 (dd, *J* = 8.5, 7.4 Hz, 1H), 7.74 - 7.65 (m, 2H), 7.64 (d, *J* = 2.0 Hz, 1H), 7.55 (dd, *J* = 8.6, 4.3 Hz, 1H), 7.06 (d, *J* = 8.3 Hz, 1H), 3.95 (s, 3H), 3.21 (s, 4H), 2.66 (s, 4H), 2.37 (s, 3H). |
| | | **NMR ¹³C (126 MHz, MeOD)** : δ 169.52 (CO), 153.41 (C_{q}), 151.39 (CH_{Ar}), 149.30 (C_{q}), 146.15 (C_{q}), 135.43 (C_{q}), 134.18 (CH_{Ar}), 130.68 (CH_{Ar}), 128.99 (C_{q}), 128.01 (CH_{Ar}), 126.46 (C_{q}), 125.74 (CH_{Ar}), 122.44 (CH_{Ar}), 122.36 (CH_{Ar}), 118.74 (CH_{Ar}), 112.37 (CH_{Ar}), 56.22 (CH₃), 56.04 (2CH₂), 50.81 (2CH₂), 46.13 (CH₃). |
| | | **HRMS (ESI):** m/z calculated for C₂₂H₂₅N₄O₂⁺, [M+H]⁺ 377.1972, found 377.1983. |
| (51) | H | **NMR ¹H (500 MHz, DMSO)** : δ 11.38 (s, 1H), 10.56 (s, 1H), 9.67 (s, 1H), 8.69 (d, *J* = 6.1 Hz, 1H), 8.05 (d, *J* = 8.5 Hz, 3H), 7.97 (d, *J* = 1.9 Hz, 2H), 7.14 (d, *J* = 8.8 Hz, 2H), 4.05 (d, *J* = 13.3 Hz, 2H), 3.49 (d, *J* = 12.1 Hz, 2H), 3.37 - 3.20 (m, 2H), 3.15 (td, *J* = 10.6, 9.3, 2.9 Hz, 2H), 2.80 (d, *J* = 4.2 Hz, 3H). |
| | | **NMR ¹³C (126 MHz, DMSO) :** δ 165.72 (CO), 152.07 (C_{q}), 147.56 (CH_{Ar}), 140.52 (CH_{Ar}), 133.78 (C_{q}), 133.57 (C_{q}), 129.58 (2CH_{Ar}), 129.14 (C_{q}), 128.58 (CH_{Ar}), 128.43 (C_{q}), 125.18 (C_{q}), 123.73 (C_{q}), 118.04 (CH_{Ar}), 114.28 (2CH_{Ar}), 51.72 (2CH₂), 44.33 (2CH₂), 41.88 (CH₃). |
| | | **HRMS (ESI)** : m/z calculated for C₂₁H₂₂ClN₄O⁺, [M+H]⁺ 381.1477, found 381.1486. |
| (52) | H | **NMR ¹H (500 MHz, DMSO) :** δ 11.62 (s, 1H), 10.90 (s, 1H), 9.50 (s, 1H), 9.07 (d, *J* = 2.1 Hz, 1H), 8.84 (d, *J* = 2.1 Hz, 1H), 8.60 (d, *J* = 6.1 Hz, 1H), 8.09 (d, *J* = 9.0 Hz, 2H), 7.96 - 7.77 (m, 1H), 7.13 (d, *J* = 9.0 Hz, 2H), 4.06 (d, *J* = 13.6 Hz, 2H), 3.50 -3.40 (m, 2H), 3.31 (d, *J* = 11.8 Hz, 2H), 3.14 (td, *J* = 9.1, 3.5 Hz, 2H), 2.79 (d, *J* = 4.3 Hz, 3H). |
| | | **NMR ¹³C (126 MHz, DMSO):** δ 165.52 (CO), 152.57 - 152.25 (m, CH_{Ar}), 152.11 (C_{q}), 142.57 (CH_{Ar}), 137.78 (C_{q}), 129.60 (CH_{Ar}), 129.22 (C_{q}), 126.13 (d, *J* = 283.1 Hz, CF3), 125.37 - 124.52 (m, CH_{Ar}), 123.65 (C_{q}), 122.82 - 122.57 (d, J = 31.03 Hz, C_{q}), 120.72 (C_{q}), 120.13 (CH_{Ar}), 116.36 (CH_{Ar}), 114.17 (CH_{Ar}), 51.67 (2CH₂), 44.18 (2CH₂), 41.85 (CH₃). |
| | | **NMR ¹⁹F (376 MHz, MeOD):** δ -61.61. |
| | | **HRMS (ESI):** m/z calculated for C₂₂H₂₂F₃N₄O⁺, [M+H]⁺ 415.1740, found 415.1758. |
| (53) | C, H | **NMR ¹H (500 MHz, Methanol-*d*4):** δ 9.31 (t, *J* = 1.4 Hz, 1H), 8.58 (d, *J =* 5.9 Hz, 1H), 8.01 (dd, *J* = 5.8, 1.0 Hz, 1H), 7.82 (dd, *J* = 8.5, 2.2 Hz, 1H), 7.75 (dd, *J* = 14.9, 2.1 Hz, 1H), 7.68 (dd, *J* = 8.3, 4.7 Hz, 1H), 7.56 (dd, *J* = 9.7, 8.3 Hz, 1H), 6.95 (t, *J* = 8.7 Hz, 1H), 3.52 - 3.41 (m, 4H), 2.99 (dq, *J* = 12.9, 7.0, 5.5 Hz, 4H), 2.51 - 2.46 (m, 2H), 2.40 (s, 3H). |
| | | **NMR ¹³C (126 MHz, DMSO):** δ 168.69 (CO), 157.10 (d, *J* = 252.3 Hz, C-F), 153.66 (d, *J* = 243.0 Hz C-F), 149.52 (d, *J* = 2.4 Hz, CH_{Ar}), 143.64 (d, *J* = 1.9 Hz, CH_{Ar}), 142.16 (d, *J* = 9.5 Hz, C_{q}), 132.26 (d, *J* = 4.4 Hz, C_{q}), 128.24 (d, *J* = 19.1 Hz, C_{q}), 126.92 (d, *J* = 7.8 Hz, C_{q}), 126.56 (d, *J* = 4.7 Hz, CH_{Ar}), 125.99 (d, *J* = 2.5 Hz, CH_{Ar}), 124.88 (d, *J* = 6.5 Hz, C_{q}), 118.19 (d, *J* = 4.9 Hz, CH_{Ar}), 116.77 (d, *J* = 23.6 Hz, CH_{Ar}), 115.55 (d, *J* = 20.5 Hz, CH_{Ar}), 114.92 (d, *J* = 3.8 Hz, CH_{Ar}), 63.62 (CH₃), 56.30 (d, *J* = 5.9 Hz, 2CH), 43.09 (2CH₂), 41.85 (2CH₂). |
| | | **¹⁹F NMR (471 MHz, Methanol-*d*4):** δ -124.76, -126.65. |
| | | **HRMS (ESI):** m/z calculated for C₂₃H₂₃F₂N₄O⁺, [M+H]⁺ 409.1834, found 409.1849. |
| (54) | C, H | **NMR ¹H (500 MHz, Methanol-*d*4):** δ 9.51 (d, *J* = 1.0 Hz, 1H), 8.44 (d, *J =* 6.1 Hz, 1H), 7.79 (dd, *J* = 8.4, 2.3 Hz, 1H), 7.74 - 7.67 (m, 3H), 7.09 (d, *J* = 8.3 Hz, 1H), 6.52 (d, *J* = 8.4 Hz, 1H), 4.08 (s, 3H), 3.80 (s, 4H), 2.43 (s, 4H), 2.31 (s, 3H), 2.30 (s, 3H), 1.89 (t, *J* = 5.6 Hz, 4H). |
| | | **NMR ¹³C (126 MHz, Methanol-*d*4):** δ 170.09 (CO), 156.62 (C_{q}), 154.87 (C_{q}), 148.14 (CH_{Ar}), 143.59 (CH_{Ar}), 135.26 (C_{q}), 132.28 (CH_{Ar}), 131.38 (CH_{Ar}), 127.94 (CH_{Ar}), 126.74 (C_{q}), 124.83 (C_{q}), 124.23 (C_{q}), 122.21 (C_{q}), 117.82 (CH_{Ar}), 113.09 (CH_{Ar}), 106.66 (CH_{Ar}), 64.35 (2CH₂), 56.57 (CH₃), 53.56 (2CH₂), 46.25 (CH₃), 36.26 (2CH₂), 34.31 (Cq), 19.98 (CH₃). |
| | | **HRMS (ESI):** m/z calculated for C₂₆H₃₁N₄O₂⁺, [M+H]⁺ 431.2442, found 431.2449. |
| (55) | E, H | **NMR ¹H (500 MHz, Methanol-*d*4):** δ 8.78 (dd, *J* = 4.2, 1.7 Hz, 1H), 8.66 (dd, *J* = 7.1, 1.8 Hz, 1H), 8.16 (dd, *J* = 8.3, 1.6 Hz, 1H), 7.72 - 7.68 (m, 1H), 7.65 (dd, *J* = 2.4, 0.9 Hz, 1H), 7.51 - 7.42 (m, 3H), 6.83 (d, *J* = 8.5 Hz, 1H), 3.87 - 3.77 (m, 2H), 2.79 (dd, *J* = 9.8, 8.0 Hz, 1H), 2.69 - 2.59 (m, 2H), 2.53 (ddd, *J* = 9.5, 8.0, 6.4 Hz, 1H), 2.39 (dd, *J* = 9.9, 6.5 Hz, 1H), 2.34 (s, 3H), 2.16 (s, 3H), 2.03 (dddd, *J* = 13.0, 9.9, 7.7, 6.4 Hz, 1H), 1.61 (ddt, *J* = 13.7, 8.1, 5.8 Hz, 1H). |
| | | **NMR ¹³C (126 MHz, Methanol-*d*4):** δ 166.71 (CO), 161.49 (C_{q}), 149.80 (CH_{Ar}), 139.88 (C_{q}), 137.61 (CH_{Ar}), 135.48 (C_{q}), 130.47 (CH_{Ar}), 129.44 (C_{q}), 128.07 (C_{q}), 128.04 (CH_{Ar}), 127.70 (CH_{Ar}), 127.38 (C_{q}), 123.07 (CH_{Ar}), 123.03 (CH_{Ar}), 117.45 (CH_{Ar}), 111.62 (CH_{Ar}), 71.85 (CH₂), 59.97 (CH₂), 56.74 (CH₂), 42.22 (CH₃), 38.73 (CH), 28.68 (CH₂), 16.55 (CH₃). |
| | | **HRMS (ESI):** m/z calculated for C₂₃H₂₆N₃O₂⁺, [M+H]⁺ 376.2020, found 376.2036. |
| (56) | C, F, G | **NMR ¹H (500 MHz, MeOD)** : δ 8.87 (dd, *J* = 4.3, 1.6 Hz, 1H), 8.48 (dt, *J* = 8.6, 1.2 Hz, 1H), 7.97 (dt, *J* = 8.4, 1.1 Hz, 1H), 7.87 - 7.67 (m, 3H), 7.57 (dd, *J* = 8.6, 4.3 Hz, 1H), 6.88 (dd, *J* = 8.9, 2.4 Hz, 1H), 6.80 (dd, *J* = 15.6, 2.4 Hz, 1H), 3.41 (t, *J* = 5.1 Hz, 4H), 2.67 (t, *J* = 5.1 Hz, 4H), 2.41 (s, 3H). |
| | | **NMR ¹³C (126 MHz, MeOD)** : δ 164.69 (CO), 162.06 (d, *J* = 246.7 Hz, CF), 155.06 (d, *J* = 11.4 Hz, C_{q}), 149.95 (CH_{Ar}), 147.83 (C_{q}), 133.61 (C_{q}), 132.21 (CH_{Ar}), 131.85 (d, *J* = 4.2 Hz, CH_{Ar}), 129.33 (CH_{Ar}), 126.21 (CH_{Ar}), 124.31 (C_{q}), 123.38 (CH_{Ar}), 121.08 (CH_{Ar}), 110.81 (d, *J* = 13.1 Hz, C_{q}), 110.09 (d, *J* = 1.9 Hz, CH_{Ar}), 100.75 (d, *J* = 28.5 Hz, CH_{Ar}), 54.06 (2CH₂), 46.40 (2CH₂), 44.49 (CH₃). |
| | | **NMR ¹⁹F (471 MHz, MeOD 2nd recours):** δ -112.37. |
| | | **HRMS (ESI):** m/z calculated for C₂₁H₂₂FN₄O⁺, [M+H]⁺ 365.1772, found 365.1780. |
| (57) | C, H | **NMR ¹H (500 MHz, MeOD)** : δ 9.39 (s, 1H), 8.47 (d, *J* = 5.8 Hz, 1H), 7.92 (dd, *J* = 7.2, 1.4 Hz, 1H), 7.88 - 7.79 (m, 4H), 6.89 (dd, *J* = 8.9, 2.4 Hz, 1H), 6.81 (dd, *J* = 15.6, 2.4 Hz, 1H), 3.49 - 3.37 (m, 4H), 2.85 - 2.61 (m, 5, CH + 2CH₂), 1.14 (s, 3H), 1.13 (s, 3H). |
| | | **NMR ¹³C (126 MHz, MeOD)** : δ 166.23 (CO), 163.51 (d, *J* = 246.5 Hz, C-F), 156.72 (d, *J =* 11.4 Hz, C_{q}), 148.85 (CH_{Ar}), 142.95 (CH_{Ar}), 138.34 (C_{q}), 135.76 (C_{q}), 133.21 (d, *J* = 4.2 Hz, CH_{Ar}), 132.29 (CH_{Ar}), 126.02 (CH_{Ar}), 125.53 (CH_{Ar}), 125.18 (C_{q}), 122.49 (CH_{Ar}), 111.86 (d, *J* = 13.0 Hz, C_{q}), 111.28 (d, *J* = 2.0 Hz, CH_{Ar}), 101.86 (d, *J* = 28.5 Hz, CH_{Ar}), 56.00 (CH), 49.58 (2CH₂), 48.18 (2CH₂), 18.65 (2CH₃). |
| | | **NMR ¹⁹F (471 MHz, MeOD) :** δ -112.49. |
| | | **HRMS (ESI):** m/z calculated for C₂₃H₂₆FN₄O⁺, [M+H]⁺ 393.2085, found 393.2098. |

### PAIN CONDITIONS OR DISORDERS

All the compounds disclosed in the present application are specifically considered herein for the prevention and/or treatment of pain conditions or disorders.

Different pain disorders may proceed from distinct mechanisms.

Chronic pain means that the duration of the pain exceeds 3 months whereas acute pain means that the duration of the pain is less than 3 months.

Among pain disorders a compound of formula (I) according to the present invention may prevent and/or treat, one may cite the following: acute pain, chronic pain, neuropathic pain, inflammatory pain, low back pain, post-operative pain, cancer pain, vascular headache such as migraine, fibromyalgia, hyperalgesia such as mechanical and thermal hyperalgesia, allodynia such as thermal and mechanical allodynia, peripheral sensitization of pain mechanisms and central sensitization of pain mechanisms.

Neuropathic pain, refers to a pain that occurs after nerve injury or a disease of the somatosensory system, is a debilitating chronic clinical condition, one hallmark symptom of which is tactile hypersensitivity to pain. According to the definition by The International Association for the Study of Pain (Treede et al., Neurology, 2008, 70:1630-1635), the term "neuropathic pain " indeed refers to a chronic or persisting pain disorder, arising as a direct consequence of a lesion or disease affecting the somatosensory system, which includes the nociceptive system and its ascending and descending pathways. The term lesion is commonly used when diagnostic investigations (e.g. clinical investigations imaging, neurophysiology, biopsies, lab tests) reveal an abnormality or when there was obvious trauma. The term disease is commonly used when the underlying cause of the lesion is known (e.g. stroke, vasculitis, diabetes mellitus, genetic abnormality). The symptoms include hyperalgesia, i.e. from a stimulus that normally provokes pain, and allodynia, i.e. pain due to a stimulus that does not normally provoke pain.

Neuropathic pain can be either peripheral or central neuropathic pain, based on the anatomic location of the lesion or disease. However, the distribution of pain or hyperalgesia is not necessarily identical to the innervation territory of the organ affected by the disease.

According to a particular embodiment of the present invention, neuropathic pain, which may also be called neuropathic pain syndrome, is selected from pain arising from metabolic diseases, for example painful diabetic neuropathic pain, infectious diseases, for example post-herpetic neuralgia, trigeminal neuralgia, post-traumatic neuropathic pain, lumbosacral radiculopathic pain, post-surgical neuropathic pain, iatrogenic neuropathic pain, for example cancer chemotherapy-induced neuropathic pain, and central neuropathic pain.

Neuropathic pain is distinct from inflammatory pain, which is produced by localized reaction following a damage to tissues outside the sensory system, for instance joints, ligaments, tendons, bones, muscles, blood vessels or visceral structures.

Different types of pain may coexist, and namely neuropathic pain can coexist with other types of pain, and in particular in chronic pain. By way of example, neuropathic pain may coexist with inflammatory or nociceptive pain, for instance in low back pain or cancer pain.

According to a particular embodiment of the present invention, the compound of formula (I) or any of its pharmaceutically acceptable salt, according to the present invention may be useful in the prevention and/or treatment of low-back pain, osteoarthritic pain, cancer pain and sciatica.

### PHARMACEUTICAL COMPOSITION AND USE

A pharmaceutical composition according to the present invention can contain one or more compound(s) of the invention in any form described herein.

The compounds can be administered through any mode of administration such as, for example, topical, intramuscular, intravenous, intranasal or oral route, etc.

In one embodiment, a pharmaceutical composition according to the present invention is selected from an oral composition; a topical composition; an inhalation composition; an injectable composition, in particular a sub-cutaneous composition, an intramuscular composition or an intravenous composition and an oral, injectable or surgical sustained release composition.

The inventive composition can further include one or more additives such as diluents, excipients, stabilizers and preservatives. Such additives are well known to those skilled in the art and are described notably in *"*Ullmann's Encyclopedia of Industrial Chemistry, 6th Ed." (various editors, 1989-1998, Marcel Dekker) and in *"*Pharmaceutical Dosage Forms and Drug Delivery Systems" (ANSEL et al., 1994, WILLIAMS & WILKINS).

The aforementioned excipients are selected according to the dosage form and the desired mode of administration. The said excipients are selected, in accordance with the pharmaceutical form and method of administration desired, from the customary excipients, which are known to a person skilled in the art.

Compositions of the present invention may be administered orally, parenterally, transdermally, transmucosally, topically, rectally, by inhalation spray, nasally, intranasally *via* inhalation, buccally, sublingually, vaginally or *via* an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, transdermally or intravenously. Sterile injectable forms of the compositions of this invention may be aqueous or oleaginous suspensions. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

In a particular embodiment, a compound of formula (I) according to the invention or anyone of its pharmaceutically acceptable salts is administered orally.

Immediate, delayed, modified or sustained release galenic forms may be chosen in the framework of the present invention, in particular sustained release galenic forms.

The treatment by administration of a compound of formula (I) according to the present invention may also be continuous. A "continuous treatment" means a long-term treatment, which can be implemented, including with various administration frequencies, preferably twice a day, and more preferably once a day.

The compound of formula (I) according to the present invention can alternatively be administered repeatedly over the course of several sequences or cycles according to a protocol which depends on the nature of the pain and intensity of the pain to be treated and also on the patient to be treated (age, weight, previous treatment(s), etc.). The protocol can be determined by any practitioner specializing in pain.

In a more particular embodiment, the compound of formula (I) according to the present invention is administered before predictable pain occurs. For example, it is well known that moderately or highly invasive surgical procedures, such as cardiac operations, joint replacement, tumour extraction, digestive tract partial ablation, graft or amputation elicit, during the hours and days following the procedure or even longer, pain of various intensity. In these conditions, and according to a particular embodiment, the compound of formula (I) according to the present invention may be administered before the surgical procedure subject is conscious, or during a surgical procedure, when the subject is anesthetized and before initiation of the opioid treatment, which generally takes place during the post-operative care.

For example, a compound of formula (I) or anyone of its pharmaceutically acceptable salts can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions, in doses which enable the daily administration of from 0.1 to 1000 mg of active substance, in particular varying from 0.1 to 10 mg, or for example varying from 10 to 200 mg, or for example varying from 200 to 1000 mg.

There may be particular cases in which higher or lower dosages are appropriate. According to usual practice, the dosage that is appropriate for each patient is determined by the doctor according to the mode of administration and the weight and response of the said patient.

In a further embodiment, the compound of formula (I) is as used as single agent or in combination with other agents such as opioids. The opioid may be selected from the group consisting of: alfentanil, allylprodine, alphaprodine, anileridine, benzylmorphine, bezitramide, buprenorphine, butorphanol, clonitazene, codeine, cyclazocine, desomorphine, dextromoramide, dextropropoxyphene, dezocine,diampromide, diamorphone, dihydrocodeine, dihydromorphine, dimenoxadol, dimepheptanol, dimethylthiambutene, dioxaphetylbuturate, dipipanone, eptazocine, ethoheptazine, ethylmethylthiambutene. ethylmorphine, etonitazene fentanyl, heroin, hydrocodone, hydromorphone, hydroxypethideine, isomethadone, ketobemidone, levallorphan, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propiram, propoxyphene, remifentanyl, sufentonil, tapentadol, tilidine and tramadol.

Herein is therefore further provided a pharmaceutical composition comprising at least a compound of formula (I) or anyone of its pharmaceutically acceptable salts, an opioid, and also at least one pharmaceutically acceptable excipient, wherein said opioid is as defined hereinabove.

In a further embodiment, a compound of formula (I) or anyone of its pharmaceutically acceptable salts, alone or in combination with an opioid, as defined hereinabove, is used as a medicament.

In a further embodiment, a compound of formula (I) or anyone of its pharmaceutically acceptable salts, alone or in combination with an opioid, as defined hereinabove, is used in the prevention and/or treatment of pain.

The examples provided herein are intended to be merely exemplary, and those skilled in the art will recognize, or will be able to ascertain using no more than routine experimentation, numerous eqalents of specific compounds, materials, and procedures. All such eqalents are considered to be within the scope of the invention and are encompassed by the appended claims.

### EXAMPLES

In the examples, the following terms mean:
- TMDCA: trans-N,N'-Dimethylcyclohexane-1,2-diamine
- DMSO: dimethylsulfoxide
- TLC: thin layer chromatography
- RuPhos: 2-Dicyclohexylphosphino-2',6'-diisopropoxybiphenyl
- Pd2(dba)3: Tris(dibenzylideneacetone)dipalladium(0)
- XantPhos: 4,5-Bis(diphenylphosphino)-9,9-dimethylxanthene
- DCM: dichloromethane
- r.t.: room temperature
- O/N: overnight
- DIAD: Diisopropyl azodicarboxylate
- eq.: eqalent
- EtOAc: ethyl acetate
- HPLC: High-performance liquid chromatography
- DMF: dimethylformamide
- THF: tetrahydrofuran
- EDCI: N-(3-Dimethylaminopropyl)-N'-ethylcarbodiimide
- HOBt: 1-Hydroxybenzotriazole
- Oxyma^{®}: Ethyl cyanohydroxyiminoacetate
- DIC: N,N'-Diisopropylcarbodiimide
- DIPEA: N,N-Diisopropylethylamine

### General procedures

### Method A. Amination of arylbromides (Scheme 2)

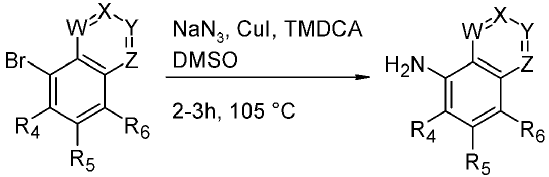

A 10 mL microwave vial was charged with an arylbromide (1 eq.), sodium azide (2 eq.), copper iodide (1 eq.) and TMDCA (1.3 eq.). The vial is flushed with argon then dry DMSO was added while flushing with argon. The reaction mixture was then capped properly and placed in a preheated oil bath at 105°C until complete conversion of the starting material (2-3 h) (TLC with EtOAc in Heptane 1:1 as eluent). The mixture was quenched with EtOAc and water, filtrated on a pad of Celite and the product was then extracted with EtOAc. The combined organic phases were washed with brine, dried over anhydrous Na₂SO₄ and the solvent was evaporated under reduced pressure. The crude was then purified by flash column chromatography.

### Method B. Suzuki-Miyaura coupling (Scheme 3)

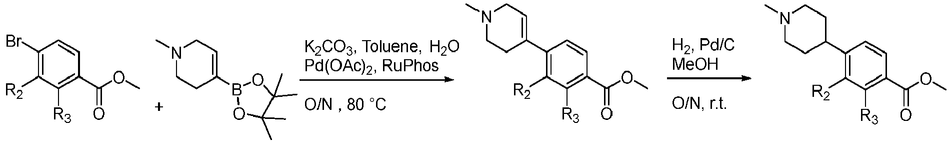

### Suzuki coupling

In a microwave vial under argon were added commercial bromo benzoate compound (1 eq.), the boronic ester or acid (1.1 eq.), K₂CO₃ (3 eq.) in toluene and water. The mixture was degassed and palladium diacetate (0.02 eq.) and commercially available RuPhos (0.04 eq.) were added. The reaction mixture was capped and heated to 80°C overnight. The flask was allowed to cool at room temperature, then was diluted in EtOAC and filtered on a Celite pad. The solvents were evaporated and the crude purified by flash column chromatography.

### Hydrogenation

To a solution of the methyl 4-(1-methyl-1,2,3,6-tetrahydropyridin-4-yl)benzoate derivative (1 eq.) in EtOH or MeOH previously degassed, was added Pd/C (0.46 eq.). The mixture was degassed again and then the flask was put under H2 atmosphere overnight. The reaction was monitored with HPLC or with TLC (10%MeOH/DCM). The mixture was filtered through celite and the solvent evaporated. The crude was purified by flash column chromatography.

### Method C. Buchwald-Hartwig coupling (Scheme 4)

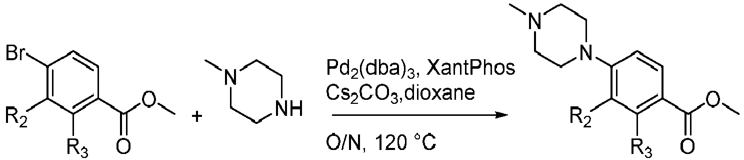

Methyl bromobenzoate derivative (1 eq.), Pd2(dba)3 (3 mol%) and Xantphos (8 mol%) was dissolved in dry 1,4-dioxane (0.53 mmol/mL) and the solution was degassed with argon for 10min. The amine (1.2 eq.) was added to the mixture followed by Cs₂CO₃ (1.4 eq.) and the reaction mixture was stirred overnight at 120°C. The reaction mixture was allowed to cool at room temperature, then was diluted in EtOAc, filtered on a Celite pad and washed with EtOAc. The solvent was removed under reduced pressure before purification by flash chromatography.

### Method D. Nucleophilic substitution (Scheme 5)

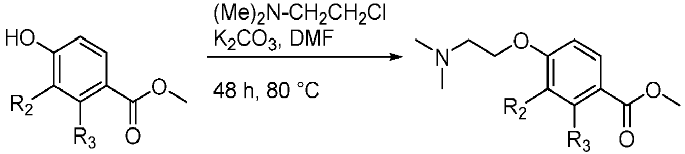

To a stirred solution of commercially available 2-dimethylaminoethyl chloride hydrochloride (1 eq.) in dry DMF was added K₂CO₃ (4 eq.) under argon atmosphere. After stirring for 10 min at room temperature, a 4-hydroxybenzoate derivative (1 eq.) was added in one portion. The vial was stirred for 48 hours at 80 °C. The reaction was quenched with sat. NaHCO₃ and extracted with Et₂O or EtOAc. The organic phase was washed with sat. NaCl before being dried over Na₂SO₄. The crude was purified by flash column chromatography.

### Method E. Mitsunobu coupling (Scheme 6)

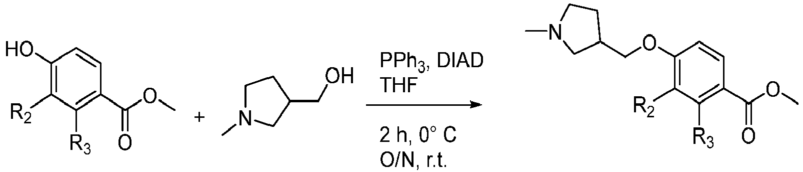

To a stirred solution, alcohol derivative (1 eq), triphenylphosphine (1.1 eq) and 4-hydroxybenzoate derivative (1.1 eq) were dissolved in dry THF under argon and the solution was cooled at 0 °C. DIAD (1 eq) was added slowly over 5 minutes. The mixture was stirred for 2h at 0 °C and then warmed at room temperature overnight. The solvent was evaporated and the obtained yellow oil was dissolved in 50 mL of EtOAc and extracted 3 times with HCl 4N. The aqueous phases were then cooled to 0°C and basified with solid NaOH until pH equals 10 at least. The basified phase was extracted 3 times with DCM, dried over MgSO₄ and filtrated. The crude was purified by flash column chromatography.

### Method F. Hydrolysis of the benzoic acid methyl ester (Scheme 7)

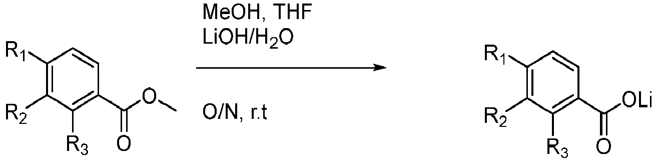

To a solution of benzoate derivative (1 eq.) in MeOH (14.94 eq.) and THF (29.55 eq.) was added a solution of lithium hydroxide monohydrate (1.05 eq.) in H₂O (33.60 eq.). The reaction was stirred overnight at room temperature. Additional LiOH in H₂O could be added and the reaction mixture could be stirred at room temperature for several days if the reaction was not completed. The reaction was evaporated under reduced pressure, taken in EtOAc and evaporated again.

### Method G. Synthesis of benzamide derivatives (Scheme 8)

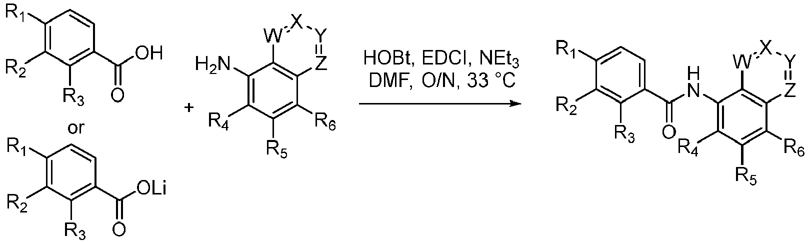

To a solution of benzoic acid or its lithium salt (1 eq.) and arylamine (1.1 eq.) in dry DMF (31.28 eq.) were added successively HOBt (1.2 eq.), EDCI (1.2 eq.), NEt₃ (2 eq.) under argon atmosphere at 0°C. The reaction was then stirred overnight at 33°C. The reaction was followed by HPLC and could be continued by adding EDCI and stirring the rection mixture at 40°C-50°C for several days if the reaction was not completed. The mixture was quenched with sat. NaHCO₃ and H₂O before being extracted with EtOAc. The combined organic phase was washed with sat. NaCl, dried over anhydrous Na₂SO₄ and evaporated under reduced pressure. The crude residue was purified by flash column chromatography.

### Method H. Synthesis of benzamide derivatives (Scheme 9)

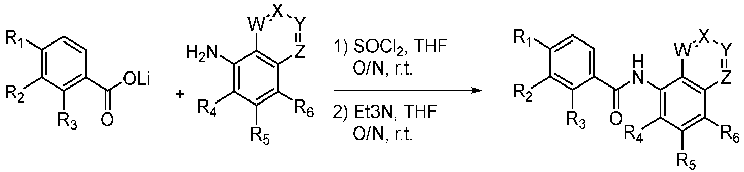

### Preparation of the benzoyl chloride

After preparation of the benzoic acid lithium salt (see general procedure F), SOCl₂ (30 eq.) was added slowly to a stirred solution of lithium salt (1 eq.) in dry DCM (3.4 mL) and the reaction mixture was heated 40°C for 1 hour. Then the excess of thionyl chloride was evaporated under reduced pressure. The crude residue was used directly in the next step without further purification.

### Amide bond formation

Benzoyl chloride derivative (1 eq.) was dissolved in anhydrous THF (0.35 mmol/mL) and the resulting solution was cooled at 0°C. Amine derivative (1.1 eq.) and NEt₃ (1.2 eq.) were added and the resulting mixture was stirred overnight at room temperature. Saturated aqueous NaHCO₃ and EtOAc were added, and the phases were separated. The aqueous phase was further extracted with EtOAc three times. Then, the combined organic phases were washed with brine, dried over MgSO₄, filtered and evaporated under reduced pressure before purification by flash chromatography

### Method I. Synthesis of benzamide derivatives (Scheme 10)

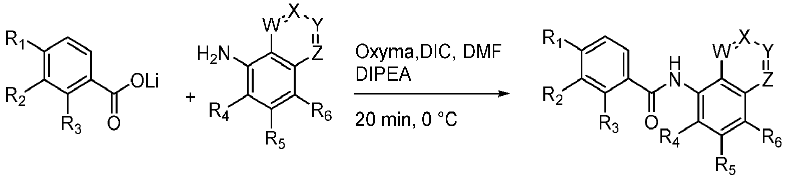

The lithium salt (1.1 eq.), Oxyma^{®} (1.1 eq.) and DIC (1.1 eq.) were mixed in dry DMF (0.18 mmol/mL) at 0 °C. The reaction mixture was stirred for 5 min at 0 °C to preactivate the carboxylate or the acid and generate the active ester. Then DIPEA (1.1 eq.) followed by amine derivative (1 eq.) were added. The reaction mixture was stirred at 0°C for 20min then at room temperature overnight. [Additional DIC could be added and the reaction mixture could be stirred at 40°C for several days if the reaction was not completed]. Saturated aqueous NaHCO₃ and EtOAc were added, and the phases were separated. The aqueous phase was further extracted with EtOAc three times. Then, the combined organic phases were washed with brine, dried over MgSO₄, filtered and evaporated under reduced pressure before purification by flash chromatography.

### Example 1: Preparation of N-(5-fluoroisoquinolin-8-yl)-4-(1-methylpiperidin-4-yl)benzamide (3)

### 5-fluoroisoquinolin-8-amine

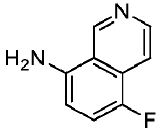

General procedure A was applied to 8-bromo-5-fluoroisoquinoline (1.079 g, 4.77 mmol). The crude residue was purified by flash chromatography on silica gel (Heptane/EtOAc 70/30 to 50/50 to 25/75) to afford 5-fluoroisoquinolin-8-amine (490 mg, 3.02 mmol, 63 %) as a brown/green solid and 98% HPLC purity.

**NMR ¹H (400 MHz, MeOD):** δ 9.39 (s, 1H), 8.40 (d, *J* = 6.0 Hz, 1H), 7.80 (bd, *J* = 6.0 Hz, 1H), 7.24 (dd, *J* = 10.1, 8.4 Hz, 1H), 6.79 (dd, *J* = 8.4, 4.1 Hz, 1H).

**NMR ¹³C (101 MHz, MeOD):** δ = 150.72 (d, *J* = 240.0 Hz, C_{q}), 148.39 (d, *J* = 2.5 Hz, CH_{Ar}), 143.74 (d, *J* = 3.0 Hz, C_{q}), 142.76 (d, *J* = 1.8 Hz, CH_{Ar}), 127.68 (d, *J* = 18.5 Hz, C_{q}), 119.81 (d, *J* = 3.9 Hz, C_{q}), 116.97 (d, *J* = 20.2 Hz, CH_{Ar}), 114.70 (d, *J* = 3.7 Hz, CH_{Ar}), 110.53 (d, *J* = 6.5 Hz, CH_{Ar}).

**NMR ¹⁹F (376 MHz, MeOD, decoupled):** δ = -141.11

### N-(5-fluoroisoquinolin-8-yl)-4-(1-methylpiperidin-4-yl)benzamide (3)

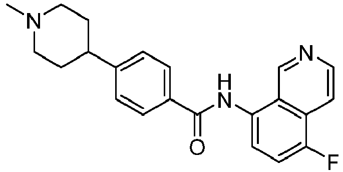

General procedure I was applied to commercial 4-(1-methylpiperidin-4-yl)benzoic acid (1 eq., 93 mg, 0.424 mmol). The crude residue was purified by flash column chromatography on silica gel (DCM/MeOH 98/2 to 85/15) to afford *N*-(5-fluoroisoquinolin-8-yl)-4-(1-methylpiperidin-4-yl)benzamide (3) (49.5 mg, 0.136 mmol, 32 %) as a light pink/purple solid.

**NMR ¹H (400 MHz, MeOD):** δ = 9.34 (bs, 1H), 8.59 (d, *J* = 5.9 Hz, 1H), 8.05 - 8.02 (m, 3H), 7.72 (dd, *J* = 8.3, 4.7 Hz, 1H), 7.58 (dd, *J* = 9.7, 8.3 Hz, 1H), 7.47 (d, *J* = 8.3 Hz, 2H), 3.07 (dt, *J* = 12.4, 3.2 Hz, 2H), 2.71 (tt, *J* = 11.7, 4.2 Hz, 1H), 2.38 (s, 3H), 2.26 (td, *J* = 11.9, 3.0 Hz, 2H), 1.95 - 1.81 (m, 4H).

**NMR ¹³C (400 MHz, MeOD):** δ = 169.9 (CO), 157.2 (d, *J* = 252.3 Hz, C_{q}), 152.0 (C_{q}), 149.5 (d, *J* = 2.1 Hz, CH_{Ar}), 143.7 (d, *J* = 1.6 Hz, CH_{Ar}), 133.1 (C_{q}), 132.2 (d, *J* = 4.5 Hz, C_{q}), 129.2 (2CH_{Ar}), 128.3 (2CH_{Ar}), 128.2 (d, *J* = 2.9 Hz, C_{q}), 127.0 (d, *J* = 7.7 Hz, CH_{Ar}), 126.5 (C_{q}), 115.6 (d, *J* = 20.5 Hz, CH_{Ar}), 115.0 (CH_{Ar}), 56.9 (2CH₂), 46.2 (CH₃), 42.7 (CH), 33.7 (2CH₂).

**NMR ¹⁹F (400 MHz, MeOD, coupled):** δ = - 126.5

**HRMS (ESI):** m/z calcd for C₂₂H₂₃FN₃O⁺, [M+H]⁺ 364.1820, found 364.1823

### Example 2: Preparation of N-(8-fluoroquinoxalin-5-yl)-4-(1-methylpiperidin-4-yl)benzamide (5)

### 5-bromo-8-fluoroquinoxaline

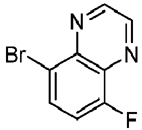

To a solution of 3-bromo-6-fluorobenzene-1,2-diamine (1 eq., 202 mg, 0.985 mmol) in EtOH (6 mL) under argon atmosphere, were added glyoxal (4.5 eq., 257 mg, 0.214 mL, 4.43 mmol) and NEt₃ (1.5 eq., 149 mg, 0.205 mL, 1.48 mmol). The reaction mixture was stirred overnight at room temperature. EtOH was evaporated under reduced pressure, then water and EtOAc were added, and the phases were separated. The aqueous phase was further extracted with EtOAc three times. Then, the combined organic phases were washed with brine, dried over MgSO₄, filtered and evaporated under reduced pressure. The crude residue was purified by flash column chromatography on silica gel (Hetpane/EtOAc 100/0 to 95/5 to 90/10) to afford 5-bromo-8-fluoroquinoxaline (198 mg, 0.872 mmol, 89 %) as a light-yellow solid.

### 8-fluoroquinoxalin-5-amine

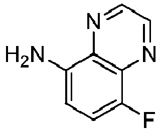

General procedure A was applied to 5-bromo-8-fluoroquinoxaline (716 mg, 3.15 mmol). The crude residue was purified by flash chromatography on silica gel (Heptane/EtOAc 9:1 to 7:3) to afford 8-fluoroquinoxalin-5-amine (302 mg, 1.85 mmol, 59 %) as a yellow/orange solid.

**NMR ¹H (400 MHz, MeOD):** δ 8.81 (d, *J* = 1.9 Hz, 1H), 8.79 (d, *J* = 1.9 Hz, 1H), 7.34 (dd, *J* = 10.4, 8.5 Hz, 1H), 6.91 (dd, *J* = 8.6, 4.4 Hz, 1H).

**NMR ¹³C (101 MHz, MeOD):** δ = 149.59 (d, *J* = 245.8 Hz, C_{q}), 146.01 (d, *J* = 2.5 Hz, CH_{Ar}), 143.96 (CH_{Ar}), 143.27 (d, *J* = 3.4 Hz, C_{q}), 134.22 (d, *J* = 13.4 Hz, C_{q}), 134.20 (C_{q}), 116.34 (d, *J* = 18.9 Hz, CH_{Ar}), 109.60 (d, *J* = 6.6 Hz, CH_{Ar}).

**NMR ¹⁹F (376 MHz, MeOD, decoupled):** δ = -144.36.

### N-(8-fluoroquinoxalin-5-yl)-4-(1-methylpiperidin-4-yl)benzamide (5)

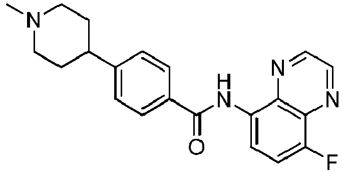

General procedure G was applied to commercial 4-(1-methylpiperidin-4-yl)benzoic acid (55.6 mg, 0.254 mmol) and 8-fluoroquinoxalin-5-amine (41.4 mg, 0.254 mmol). The crude residue was purified by flash column chromatography on silica gel (DCM/MeOH 98/2 to 85/15) then triturated with DCM/EtOAc/Et₂O to afford *N*-(8-fluoroquinoxalin-5-yl)-4-(1-methylpiperidin-4-yl)benzamide **(5)** (8 mg, 0,022 mmol, 9 %) as light yellow solid and >99% HPLC purity.

**NMR ¹H (400 MHz, MeOD):** δ 9.01 (d, *J* = 1.9 Hz, 1H), 8.99 (d, *J* = 1.9 Hz, 1H), 8.76 (dd, *J* = 8.8, 4.8 Hz, 1H), 7.98 (d, *J* = 8.3 Hz, 2H), 7.61 (dd, *J* = 10.1, 8.7 Hz, 1H), 7.47 (d, *J* = 8.3 Hz, 2H), 3.10-2.99 (m, 2H), 2.73-2.65 (m, 1H), 2.37 (s, 3H), 2.24 (td, *J* = 11.8, 3.0 Hz, 2H), 1.96-1.77 (m, 4H).

**NMR ¹³C (101 MHz, MeOD):** δ = 167.36 (CO), 154.14 (d, *J* = 256.3 Hz, C_{q}), 151.79 (C_{q}), 147.05 (d, *J* = 2.5 Hz, CH_{Ar}), 145.82 (CH_{Ar}), 136.01 (C_{q}), 133.71 (C_{q}), 132.42 (d, *J* =4.9 Hz, C_{q}), 128.68 (2CH_{Ar}), 128.55 (2CH_{Ar}), 128.12 (C_{q}), 118.86 (d, *J* = 7.3 Hz, CH_{Ar}), 115.50 (d, *J* = 19.0 Hz, CH_{Ar}), 56.76 (2CH₂), 45.97 (CH₃), 42.44 (CH), 33.44 (2CH₂).

NMR **¹⁹F (376 MHz, MeOD, decoupled):** δ = -133.0

**HRMS (ESI):** m/z calcd for C₂₁H₂₂FN₄O⁺, [M+H]⁺ 365.1772, found 365.1782

### Example 3 : Preparation of 2-fluoro-4-(1-methylpiperidin-4-yl)-N-(quinolin-8-yl)benzamide (12)

### methyl 2-fluoro-4-(1-methylpiperidin-4-yl)benzoate

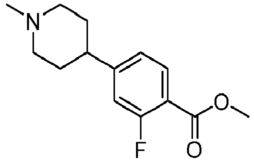

General procedure B was applied to methyl 4-bromo-2-fluorobenzoate (1 eq., 500 mg, 2.15 mmol) and 1-methyl-1,2,3,6-tetrahydropyridine-4-boronic acid pinacol ester (1.1 eq., 526.6 mg, 2.36 mmol). The crude was purified by flash column chromatography using EtOAC in heptane (0 to 100%) and then methanol in DCM (0 to 10%) as eluent to afford methyl 3-fluoro-4-(1-methyl-1,2,3,6-tetrahydropyridin-4-yl)benzoate (280.8 mg, 1.13 mmol, 53%) obtained as light orange solid. After the hydrogenation of the obtained compound (1 eq., 280.8 mg, 1.13 mmol), methyl 3-fluoro-4-(1-methylpiperidin-4-yl)benzoate (275.5 mg, 1.096 mmol, 97%) was obtained as a yellow oil without purification.

**NMR ¹H (400 MHz, MeOD)** : δ 7.80 (dd, *J* = 8.0, 1.7 Hz, 1H), 7.65 (dd, *J* = 11.0, 1.7 Hz, 1H), 7.44 (t, *J* = 7.7 Hz, 1H), 3.90 (s, 3H), 3.03 (ddt, *J* = 11.4, 3.7, 1.9 Hz, 2H), 3.00 - 2.88 (m, 1H), 2.35 (s, 3H), 2.27 - 2.15 (m, 2H), 1.90 - 1.79 (m, 4H).

**NMR ¹³C (126 MHz, MeOD) :** δ 167.30 (d, *J* = 2.7 Hz, CO), 161.75 (d, *J* = 245.2 Hz, CF), 139.20 (d, *J* = 14.5 Hz, C_{q}), 131.36 (d, *J* = 8.0 Hz, C_{q}), 129.23 (d, *J* = 4.7 Hz, CH_{Ar}), 126.64 (d, *J* = 3.3 Hz, CH_{Ar}), 117.13 (d, *J* = 25.3 Hz, CH_{Ar}), 56.84 (2CH₂), 52.79 (CH₃), 46.31 (CH₃), 36.22 (d, *J* = 1.9 Hz, CH), 32.32 (2CH₂).

**NMR ¹⁹F (376 MHz, MeOD)** : δ -120.24.

### 2-fluoro-4-(1-methylpiperidin-4-yl)-N-(quinolin-8-yl)benzamide (12)

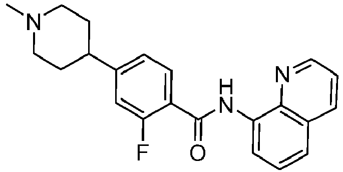

General procedure F was applied to methyl 2-fluoro-4-(1-methylpiperidin-4-yl)benzoate (1 eq., 275.5 mg, 1.096 mmol) to afford quantitatively lithio 3-fluoro-4-(1-methylpiperidin-4-yl)benzoate (316 mg) obtained as a light beige powder. According to general procedure G, the lithium salt (1 eq., 76.68 mg, 0.32 mmol) was used with 8-quinolinamine (1.1 eq., 50 mg, 0.35 mmol) to the amide bond coupling and the reaction mixture was stirred overnight at 30°C. 3 eqalents of EDCI were added and the reaction mixture was stirred for 3 days at 35°C. The crude was purified by flash column chromatography using MeOH in DCM as eluent (0 to 15%). 2-fluoro-4-(1-methylpiperidin-4-yl)-N-(quinolin-8-yl)benzamide **(12)** was obtained as a beige powder (61.9 mg, 0.17 mmol, 54%) and 99% HPLC purity.

**NMR ¹H (400 MHz, MeOD):** δ 8.89 (dd, *J* = 4.2, 1.7 Hz, 1H), 8.85 (dd, *J* = 7.6, 1.4 Hz, 1H), 8.32 (dd, *J* = 8.3, 1.7 Hz, 1H), 8.06 (t, *J* = 8.2 Hz, 1H), 7.66 (dd, *J* = 8.3, 1.4 Hz, 1H), 7.63 - 7.47 (m, 2H), 7.34 - 7.18 (m, 2H), 3.01 (dt, *J* = 12.6, 3.3 Hz, 2H), 2.68 (tt, *J* = 11.8, 3.9 Hz, 1H), 2.34 (s, 3H), 2.17 (td, *J* = 12.7, 11.9, 3.4 Hz, 2H), 1.96 - 1.87 (m, 2H), 1.80 (dtd, *J* = 13.4, 12.1, 3.8 Hz, 2H).

**NMR ¹³C (126 MHz, MeOD):** δ 163.24 (CO), 163.01 - 161.04 (d, *J* = 248.30 Hz, CF), 155.07 - 155.01 (d, *J* = 8.50 Hz, C_{q}), 150.10 (CH_{Ar}), 140.01 (C_{q}), 137.63 (CH_{Ar}), 135.77 (C_{q}), 132.78 - 132.76 (d, *J* = 2.40 Hz, CH_{Ar}), 129.58 (C_{q}), 128.12 (CH_{Ar}), 124.78 - 124.76 (d, *J* = 2.73 Hz, CH_{Ar}), 123.69 (CH_{Ar}), 123.19 (CH_{Ar}), 120.75 - 120.65 (d, *J* = 12.32 Hz, C_{q}), 118.20 (CH_{Ar}), 115.86 - 115.67 (d, *J* = 24.64 Hz, CH_{Ar}), 56.79 (2CH₂), 46.37 (CH₃), 42.58 (CH), 33.59 (2CH₂).

**NMR ¹⁹F (376 MHz, MeOD):** δ -114.04.

**HRMS (ESI):** m/z calculated for C₂₂H₂₃FN₃O⁺ [M+H]⁺: 364.1825; found 364.1827.

### Example 4: 4-(2-(dimethylamino)ethoxy)-N-(quinolin-5-yl)benzamide (15)

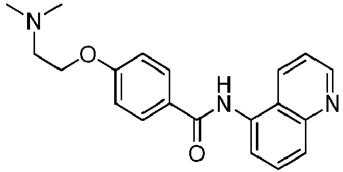

General procedure F was applied to methyl 4-[2-(dimethylamino)ethoxy]benzoate (1.3 g, 5.82 mmol) to afford quantitatively lithio 4-[2-(dimethylamino)ethoxy]benzoate (1.25g) as a beige solid. According to general procedure G, one eqalent of the lithium salt (71.3 mg, 0.332 mmol) was used with 5-quinolinamine (1 eq., 47.8 mg, 0.332 mmol) to the amide bond coupling and the reaction mixture was stirred 2 days at 30°C before the addition of 1.5 eqalents of EDCI. After 5 days of stirring at 40°C, the crude was purified by flash column chromatography using MeOH in DCM (98/2 to 85/15) as eluent. 4-[2-(dimethylamino)ethoxy]-*N*-(quinolin-5-yl)benzamide **(15)** (65 mg, 0.194 mmol, 58 %) was obtained as a light yellow solid and 93% HPLC purity.

**NMR ¹H (400 MHz, MeOD):** δ 8.86 (dd, *J* = 4.3, 1.6 Hz, 1H), 8.43 (bd, *J* = 8.5, 1H), 8.05 (d, *J* = 8.9 Hz, 2H), 7.99 (dt, *J* = 8.5, 1.1 Hz, 1H), 7.80 (dd, *J* = 8.5, 7.4 Hz, 1H), 7.68 (dd, *J* = 7.4, 1.2 Hz, 1H), 7.54 (dd, *J* = 8.6, 4.3 Hz, 1H), 7.10 (d, *J* = 8.9 Hz, 2H), 4.22 (t, *J* = 5.4 Hz, 2H), 2.88 (t, *J* = 5.4 Hz, 2H), 2.41 (s, 6H).

**NMR ¹³C (101 MHz, MeOD):** δ 169.36 (CO), 163.37 (C_{q}), 151.38 (CH_{Ar}), 149.28 (C_{q}), 135.39 (C_{q}), 134.14 (CH_{Ar}), 130.93 (2CH_{Ar}), 130.68 (CH_{Ar}), 127.98 (CH_{Ar}), 127.59 (C_{q}), 126.42 (C_{q}), 125.69 (CH_{Ar}), 122.43 (CH_{Ar}), 115.52 (2CH_{Ar}), 66.64 (CH₂), 58.87 (CH₂), 45.71 (2CH₃).

**HRMS (ESI):** m/z calculated for C₂₀H₂₂N₃O₂⁺, [M+H]⁺ 336.1707, found 336.1709.

### Example 5: Preparation of 4-(1-methylpiperidin-4-yl)-N-(quinolin-8-yl)-2-(trifluoromethyl)benzamide (18)

### methyl 4-(1-methylpiperidin-4-yl)-2-(trifluoromethyl)benzoate

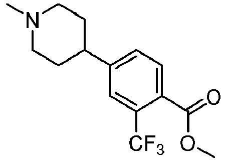

General procedure B was applied to methyl 4-bromo-2-(trifluoromethyl)benzoate (1 eq., 400 mg, 1.41 mmol) and 1-methyl-1,2,3,6-tetrahydropyridine-4-boronic acid pinacol ester (1.1 eq., 346.85 mg, 1.55 mmol). The crude was purified by flash column chromatography using EtOAc in heptane (50 to 100%) and then MeOH in DCM (0 to 10%) as eluent to afford methyl 4-(1-methyl-1,2,3,6-tetrahydropyridin-4-yl)-2-(trifluoromethyl)benzoate was obtained as an orange oil (404 mg, 1.35 mmol, 96%). After hydrogenation of the obtained compound, methyl 4-(1-methylpiperidin-4-yl)-2-(trifluoromethyl)benzoate was obtained as a yellow oil (390 mg, 1.29 mmol, 97%) and used in the next step without further purification.

**NMR ¹H (400 MHz, MeOD):** δ 7.66 (d, *J* = 8.0 Hz, 1H), 7.57 (d, *J* = 1.7 Hz, 1H), 7.50 (dd, *J* = 8.0, 1.8 Hz, 1H), 2.95 - 2.87 (m, 2H), 2.61 (tt, *J* = 12.0, 4.0 Hz, 1H), 2.24 (s, 3H), 2.08 (td, *J* = 12.0, 2.8 Hz, 2H), 1.82 - 1.75 (m, 2H), 1.69 (dtd, *J* = 13.3, 12.0, 3.7 Hz, 2H).

**NMR ¹³C (101 MHz, MeOD):** δ 168.58 (CO), 151.60 (C_{q}), 131.76 (CH_{Ar}), 131.59 (C_{q}), 130.30 (CH_{Ar}), 129.93 - 129.61 (d, *J* = 31.90 Hz, C-CF₃), 126.35 (d, *J* = 5.4 Hz, CH_{Ar}), 126.27 - 120.846 (q, *J* = 273.40 Hz, CF3), 56.77 (2CH₂), 53.19 (CH₃), 46.36 (CH₃), 42.65 (CH), 33.62 (2CH₂).

**NMR ¹⁹F (376 MHz, MeOD):** δ -60.90.

### 4-(1-methylpiperidin-4-yl)-N-(quinolin-8-yl)-2-(trifluoromethyl)benzamide (18)

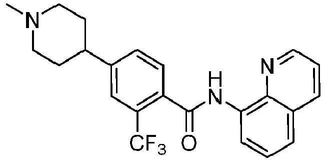

General procedure G was applied to methyl 4-(1-methylpiperidin-4-yl)-2-(trifluoromethyl)benzoate (1 eq., 390 mg, 1.29 mmol) and led to lithio 4-(1-methylpiperidin-4-yl)-2-(trifluoromethyl)benzoate obtained as a light beige powder (450 mg, 1.53 mmol, quantitative yield). The lithium salt previously synthesized was used with 8-aminoquinoline (1.1 eq., 60 mg, 0.42 mmol) to the amide bon coupling and the reaction mixture was stirred overnight at 30°C. 3 eqalents of EDCI were added and the reaction mixture was stirred for 3 days at 35 °C. The crude was purified by flash column chromatography using MeOH in DCM (0 to 15 %) as eluent. 4-(1-Methylpiperidin-4-yl)-N-(quinolin-8-yl)-2-(trifluoromethyl)benzamide **(18)** was obtained as an orange powder (76 mg, 0.18 mmol, 49%) and 97 % HPLC purity.

**NMR ¹H (400 MHz, MeOD):** δ 8.81 (dd, *J* = 4.2, 1.7 Hz, 1H), 8.74 (dd, *J* = 7.6, 1.4 Hz, 1H), 8.32 (dd, *J* = 8.3, 1.7 Hz, 1H), 7.77 - 7.72 (m, 2H), 7.72 - 7.65 (m, 2H), 7.61 (dd, *J* = 8.3, 7.6 Hz, 1H), 7.55 (dd, *J* = 8.3, 4.3 Hz, 1H), 3.05 (dq, *J* = 11.5, 2.5, 1.7 Hz, 2H), 2.76 (tt, *J* = 12.0, 4.0 Hz, 1H), 2.37 (s, 3H), 2.24 (td, *J* = 12.0, 2.7 Hz, 2H), 1.98 - 1.90 (m, 2H), 1.84 (dtd, *J* = 13.2, 12.0, 3.8 Hz, 2H).

**NMR ¹³C (101 MHz, MeOD)** : δ 168.09 (C=O), 150.38 (C_{q}), 150.16 (CH_{Ar}), 139.97 (C_{q}), 137.75 (CH_{Ar}), 135.37 (C_{q}), 135.19 (C_{q}), 135.18 (C_{q}), 132.05 (CH_{Ar}), 130.01 (CH_{Ar}), 129.63 (C_{q}), 128.78 - 128.47 (d, *J* = 31.51 Hz, C-CF₃) , 128.03 (CH_{Ar}), 126.58 - 123.86 (d, *J* = 272.89 Hz, CF₃), 126.30 - 126.26 (d, *J* = 4.79 Hz, CH_{Ar}), 124.15 (CH_{Ar}), 123.20 (CH_{Ar}), 118.37 (CH_{Ar}), 56.76 (2CH₂), 46.26 (CH₃), 42.51 (CH), 33.62 (2CH₂).

**NMR ¹⁹F (376 MHz, MeOD):** δ -60.08.

**HRMS (ESI):** m/z calculated for C₂₃H₂₂F₃N₃NaO⁺ [M+Na]⁺: 436.4613; found 436.1623.

### Example 6: Preparation of 3-cyano-N-(isoquinolin-5-yl)-4-(1-methylpiperidin-4-yl)benzamide (21)

### methyl 3-cyano-4-(1-methylpiperidin-4-yl)benzoate

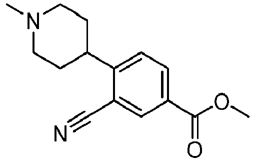

General procedure B was applied to methyl 4-bromo-3-cyanobenzoate (1 eq., 300 mg, 1.25 mmol) and 1-methyl-4-(4,4,5,5-tetramethyl-1,3,2-dioxaborolan-2-yl)-1,2,3,6-tetrahydropyridine (1.063 eq., 296.37 mg, 1.33 mmol). The crude was purified by flash column chromatography using EtOAc in heptane (50 to 100%) and then methanol in DCM (0 to 10%) as eluent to afford quantitatively methyl 3-cyano-4-(1-methyl-1,2,3,6-tetrahydropyridin-4-yl)benzoate (384.37 mg) obtained as light orange oil. After hydrogenation of the obtained compound, the crude was purified by flash column chromatography using MeOH in DCM (0 to 10%) as eluent and led to methyl 3-cyano-4-(1-methylpiperidin-4-yl)benzoate (177.2 mg, 0.69 mmol, 55%) obtained as a yellow solid.

**NMR ¹H (400 MHz, MeOD):** δ 8.28 (dd, *J* = 1.9, 0.5 Hz, 1H), 8.23 (dd, *J* = 8.3, 1.9 Hz, 1H), 7.64 (d, *J* = 8.3 Hz, 1H), 3.93 (s, 3H), 3.09 - 2.99 (m, 3H, CH2+CH), 2.35 (s, 3H), 2.21 (td, *J* = 11.7, 3.2 Hz, 2H), 1.94 - 1.79 (m, 4H, 2CH₂)

**NMR ¹³C (101 MHz, MeOD):** δ 166.48 (CO), 155.27 (C_{q}), 135.07 (CH_{Ar}), 135.04 (CH_{Ar}), 130.59 (C_{q}), 128.32 (CH_{Ar}), 117.92 (CN), 113.72 (C_{q}), 56.71 (2CH₂), 53.02 (CH₃), 46.34 (CH₃), 41.72 (CH), 32.98 (2CH₂).

### 3-cyano-N-(isoquinolin-5-yl)-4-(1-methylpiperidin-4-yl)benzamide (21)

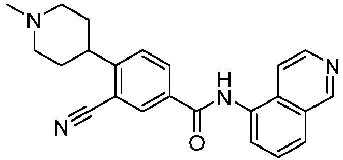

General procedure F was applied to methyl 3-cyano-4-(1-methylpiperidin-4-yl)benzoate (1 eq., 177.2 mg, 0.69 mmol) to afford quantitatively lithio 3-cyano-4-(1-methylpiperidin-4-yl)benzoate (190.3 mg) as a light yellow powder. According to general procedure I, one eqalent of the lithium salt previously synthesized (58.2 mg, 0.233 mmol) was used with 5-isoquinolinamine (1 eq., 33.5 mg, 0.233 mmol) according to general procedure G. The crude residue was purified by flash column chromatography on silica gel (DCM/MeOH 98/2 to 85/15) to afford 3-cyano-*N*-(isoquinolin-5-yl)-4-(1-methylpiperidin-4-yl)benzamide **(21)** (24 mg, 0.0648 mmol, 28 %) as a light orange solid and 97% HPLC purity.

**NMR ¹H (400 MHz, MeOD):** δ 9.20 (s, 1H), 8.38 (d, *J* = 6.0 Hz, 1H), 8.32 (s, 1H), 8.24 (dd, *J* = 8.3, 2.0 Hz, 1H), 8.00 (dd, *J* = 8.2, 1.1 Hz, 1H), 7.86 (dd, *J* = 7.5, 1.3 Hz, 1H), 7.81 (d, *J* = 6.0 Hz, 1H), 7.68-7.62 (m, 2H), 3.17-3.00 (m, 3H), 2.40 (s, 3H), 2.39-2.30 (m, 2H), 1.98-1.77 (m, 4H).

**NMR ¹³C (101 MHz, MeOD):** δ 167.34 (CO), 153.72 (C_{q}), 153.60 (CH_{Ar}), 143.19 (CH_{Ar}), 134.64 (C_{q}), 133.82 (C_{q}), 133.80 (CH_{Ar}), 133.76 (CH_{Ar}), 133.55 (C_{q}), 130.80 (C_{q}), 129.64 (CH_{Ar}), 128.75 (CH_{Ar}), 128.38 (CH_{Ar}), 128.02 (CH_{Ar}), 118.10 (C_{q}), 117.83 (C_{q}), 113.75 (CH_{Ar}), 56.46 (2CH₂), 45.81 (CH₃), 41.12 (CH), 32.54 (2CH₂).

**HRMS (ESI):** m/z calculated for C₂₃H₂₃N₄O⁺, [M+H]⁺ 371.1866, found 371.1864.

### Example 7: Preparation of N-(isoquinolin-5-yl)-2-methanesulfonyl-4-(1-methylpiperidin-4-yl)benzamide (25)

### methyl 2-methanesulfonyl-4-(1-methylpiperidin-4-yl)benzoate

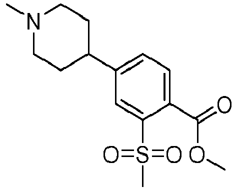

General procedure B was applied to methyl 4-bromo-2-(methylsulfonyl)benzoate (1 eq., 400 mg, 1.36 mmol) and 1-methyl-4-(4,4,5,5-tetramethyl-1,3,2-dioxaborolan-2-yl)-1,2,3,6-tetrahydropyridine (1.1 eq., 334.91 mg, 1.501 mmol). The crude was purified by flash column chromatography using EtOAc in heptane (50 to 100%) and then MeOH in DCM (0 to 10%) as eluent to afford methanesulfonyl-4-(1-methyl-1,2,3,6-tetrahydropyridin-4-yl)benzoate (410.5 mg, 1.33 mmol, 97%) obtained as an orange oil. After the hydrogenation of the obtained compound (1 eq., 330 mg, 1.067 mmol), the crude was purified by flash column chromatography using MeOH in DCM (0 to 20%) as eluent and led to methyl 2-methanesulfonyl-4-(1-methylpiperidin-4-yl)benzoate obtained as a yellow oil (124.9 mg, 0.401 mmol, 37%).

**NMR ¹H (400 MHz, MeOD):** δ 7.97 (d, *J* = 1.5 Hz, 1H), 7.74 - 7.61 (m, 2H), 3.92 (s, 3H), 3.09 - 2.92 (m, 2H), 2.72 (ddt, *J* = 11.9, 7.8, 4.0 Hz, 1H), 2.33 (s, 3H), 2.18 (td, *J* = 11.8, 2.6 Hz, 2H), 1.98 - 1.85 (m, 2H), 1.85 - 1.63 (m, 2H).

**NMR ¹³C (126 MHz, MeOD):** δ 168.95 (CO), 151.43, 140.46, 133.24, 132.27, 131.17, 129.31, 56.62, 53.52, 46.25, 45.01, 42.41, 33.46.

### N-(isoquinolin-5-yl)-2-methanesulfonyl-4-(1-methylpiperidin-4-yl)benzamide (25)

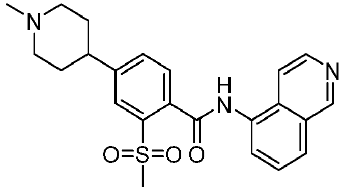

General procedure F was applied to methyl 2-methanesulfonyl-4-(1-methylpiperidin-4-yl)benzoate (1 eq., 124 mg, 0.4 mmol) to afford quantitatively lithio 2-methanesulfonyl-4-(1-methylpiperidin-4-yl)benzoate (130 mg) as beige powder. According to general procedure G, the lithium salt (1 eq., 50 mg, 0.16 mmol) was used with 5-aminoisoquinoline (1.1 eq., 26.14 mg, 0.18 mmol) for the amide bond coupling and the reaction mixture was stirred overnight at 48 hours at 35°C before adding another eqalent of EDCI (89.8 mg). The reaction was kept stirring for 48 hours at 35°C. The crude was purified by flash column chromatography using MeOH in DCM as eluent (0 to 25%). N-(isoquinolin-5-yl)-2-methanesulfonyl-4-(1-methylpiperidin-4-yl)benzamide (25) was obtained as a light-yellow powder (26.6 mg, 0.063 mmol, 38%) and 98% HPLC purity.

**NMR ¹H (400 MHz, CDCl₃)** : δ 9.43 (s, 1H), 9.19 (s, 1H), 8.49 (d, *J* = 6.0 Hz, 1H), 8.14 (d, *J* = 7.4 Hz, 1H), 7.94 - 7.88 (m, 2H), 7.85 (d, *J* = 8.2 Hz, 1H), 7.73 (d, *J* = 7.7 Hz, 1H), 7.62 (t, *J* = 7.9 Hz, 1H), 7.53 (dd, *J* = 7.8, 1.8 Hz, 1H), 3.33 (s, 3H), 2.98 (d, *J* = 11.3 Hz, 2H), 2.58 (tt, *J* = 12.1, 4.2 Hz, 1H), 2.27 (s, 3H), 2.09 (td, *J* = 11.8, 2.7 Hz, 2H), 1.79 - 1.71 (m, 2H), 1.71 - 1.58 (m, 2H).

**NMR ¹³C (126 MHz, MeOD):** δ 170.16 (CO), 153.39 (CH_{Ar}), 150.18 (C_{q}), 143.08 (CH_{Ar}), 139.82 (C_{q}), 136.59 (C_{q}), 133.56 (C_{q}), 133.45 (C_{q}), 133.43 (CH_{Ar}), 130.73 (C_{q}), 130.37 (CH_{Ar}), 129.38 (CH_{Ar}), 129.29 (CH_{Ar}), 128.73 (CH_{Ar}), 127.81 (CH_{Ar}), 118.06 (CH_{Ar}), 56.59 (2CH₂), 45.95 (CH₃), 45.73 (CH₃), 42.18 (CH), 33.33 (2CH₂).

**HRMS (ESI):** m/z calculated for C₂₃H₂₆N₃O₃S⁺ [M+H]⁺: 424.1689; found 424.1686.

### Example 8: Preparation of 4-(1-methylpiperidin-4-yl)-N-(quinolin-5-yl)-3-(trifluoromethyl)benzamide (27)

### methyl 4-(1-methylpiperidin-4-yl)-3-(trifluoromethyl)benzoate

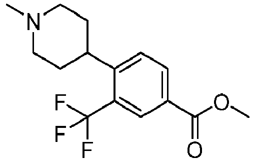

General procedure B was applied to methyl 4-bromo-3-(trifluoromethyl)benzoate (1 eq., 400 mg, 1.41 mmol) and 1-methyl-1,2,3,6-tetrahydropyridine-4-boronic acid pinacol ester (1.1 eq., 346.85 mg, 1.55 mmol). The crude was purified by flash column chromatography using EtOAc in heptane (50 to 100%) and then methanol in DCM (0 to 10%) as eluent to afford methyl 4-(1-methyl-1,2,3,6-tetrahydropyridin-4-yl)-3-(trifluoromethyl)benzoate (330 mg, 1.103 mmol, 78%) obtained as an green oil. After hydrogenation of the obtained compound (1 eq., 330 mg, 1.103 mmol), the crude was purified by flash column chromatography using MeOH in DCM as eluent (0 to 10%) and led to methyl 4-(1-methylpiperidin-4-yl)-3-(trifluoromethyl)benzoate (87.1 mg, 0.29 mmol, 26%) obtained as a yellow oil.

**NMR ¹H (400 MHz, MeOD):** δ 8.25 (d, *J* = 1.8 Hz, 1H), 8.21 (dd, *J* = 8.2, 1.9 Hz, 1H), 7.72 (d, *J* = 8.2 Hz, 1H), 3.93 (s, 3H), 3.09 - 3.01 (m, 2H), 2.98 (m, 1H), 2.36 (s, 3H), 2.17 (td, *J* = 12.0, 2.7 Hz, 2H), 1.90 (qd, *J* = 12.4, 3.8 Hz, 2H), 1.83 - 1.74 (m, 2H).

**NMR ¹³C (126 MHz, MeOD):** δ 167.00 (C=O), 151.41, 134.21, 130.08, 129.87, 129.78 - 128.71 (m, *J* = 30.26 Hz, C_{q}-CF3), 127.85 (q, *J* = 6.1 Hz), 125.62 (d, *J* = 273.3 Hz, CF3), 56.87 (2CH₂), 52.93 (CH₃), 46.34 (CH₃), 39.25 (d, *J* = 2.2 Hz, CH), 33.89 (2CH₂).

**NMR ¹⁹F (376 MHz, MeOD):** δ -60.63.

### 4-(1-methylpiperidin-4-yl)-N-(quinolin-5-yl)-3-(trifluoromethyl)benzamide(27)

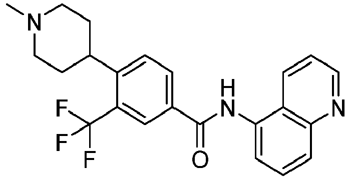

General procedure F was applied to methyl 4-(1-methylpiperidin-4-yl)-3-(trifluoromethyl)benzoate (1 eq., 87 mg, 0.29 mmol) to afford quantitatively lithio 4-(1-methylpiperidin-4-yl)-3-(trifluoromethyl)benzoate (96.8 mg) as a beige powder. According to general procedure G, the lithium salt (1 eq., 40 mg, 0.14 mmol) was used with 5-quinolinamine (1.1 eq., 21.64 mg, 0.15 mmol) to the amide bond coupling and the reaction mixture was stirred overnight at 30°C. 34,5 mg and 37.5 mg of EDCI were successively added every 24 hours and the reaction was stirred overnight at 35°C each time. 73.5 mg of EDCI, 40.3 mg of lithio 4-(1-methylpiperidin-4-yl)-3-(trifluoromethyl)benzoate and 1 eqalent of Et₃N were added and the reaction was stirred 48h at 35°C. After extraction, the crude was purified by flash column chromatography using MeOH in EtOAc (0 to 15%) as eluent. 4-(1-Methylpiperidin-4-yl)-N-(quinolin-5-yl)-3-(trifluoromethyl)benzamide (27) (30 mg, 0.073 mmol, 53%) was obtained as a yellow powder and 98% HPLC purity.

**NMR ¹H (500 MHz, MeOD)** : δ 8.87 (dd, *J* = 4.3, 1.6 Hz, 1H), 8.44 (dt, *J* = 8.5, 1.3 Hz, 1H), 8.38 (d, *J* = 1.9 Hz, 1H), 8.30 (dd, *J* = 8.2, 2.0 Hz, 1H), 8.01 (dt, *J* = 8.5, 1.1 Hz, 1H), 7.80 (td, *J* = 8.7, 7.6 Hz, 2H), 7.72 (dd, *J* = 7.5, 1.2 Hz, 1H), 7.55 (dd, *J* = 8.6, 4.3 Hz, 1H), 3.12 - 2.97 (m, 3H, CH2+CH), 2.38 (s, 3H), 2.31 - 2.15 (m, 2H), 1.95 (qd, *J* = 12.5, 3.7 Hz, 2H), 1.88 - 1.73 (m, 2H).

**NMR ¹³C (126 MHz, MeOD)** : δ 168.06 (CO), 151.46 (CH_{Ar}), 150.33 (C_{q}), 149.27 (C_{q}), 134.92 (C_{q}), 134.01 (CH_{Ar}), 133.68 (C_{q}), 132.67 (CH_{Ar}), 130.65 (CH_{Ar}), 130.06 (CH_{Ar}), 129.47 (q, *J* = 29.8 Hz, C_{q}-CF), 128.27 (CH_{Ar}), 129.11 - 124.48 (q, *J* = 274.46 Hz, CF3), 126.65 (q, *J* = 6.0 Hz, CH_{Ar}), 126.25 (CH_{Ar}), 125.72 (C_{q}), 122.54 (CH_{Ar}), 56.89 (2CH₂), 46.34 (CH₃), 39.14 (CH), 33.95 (2CH₂).

**NMR ¹⁹F (376 MHz, MeOD):** δ -60.32.

**HRMS (ESI):** m/z calculated for C₂₃H₂₃F₃N₃O⁺ [M+H]⁺: 414.1788; found 414.1785.

### Example 9: preparation of 3-methyl-4-(4-methylpiperazin-1-yl)-N-(quinolin-5-yl)benzamide (29)

### methyl 3-methyl-4-(4-methylpiperazin-1-yl)benzoate

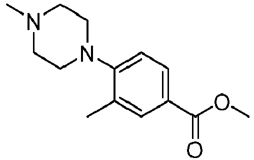

General procedure C was applied to methyl 4-bromo-3-methylbenzoate (232 mg, 1.01 mmol). The crude residue was purified by flash column chromatography using EtOAc in heptane (90/10 to 70/30) then MeOH in DCM (98/2 to 90/10) as eluent to afford methyl 3-methyl-4-(4-methylpiperazin-1-yl)benzoate (160 mg, 0.646 mmol, 64 %) as an orange oil.

**NMR ¹H (400 MHz, CDCl₃)** : δ 7.86-7.76 (m, 2H), 6.97 (d, *J* = 8.1 Hz, 1H), 3.85 (s, 3H), 2.98 (t, *J* = 4.7 Hz, 4H), 2.57 (bs, 4H), 2.35 (s, 3H), 2.30 (s, 3H).

**NMR ¹³C (101 MHz, CDCl₃) :** δ 167.26 (CO), 155.84 (C_{q}), 132.69 (CH_{Ar}), 131.67 (C_{q}), 128.51 (CH_{Ar}), 124.10 (C_{q}), 118.29 (CH_{Ar}), 55.44 (2CH₂), 51.88 (CH₃), 51.20 (2CH₂), 46.20 (CH₃), 18.39 (CH₃).

### 3-methyl-4-(4-methylpiperazin-1-yl)-N-(quinolin-5-yl)benzamide (29)

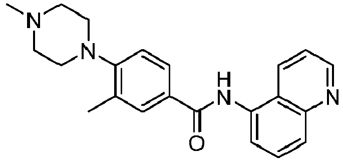

General procedure F was applied to methyl 3-methyl-4-(4-methylpiperazin-1-yl)benzoate (158 mg, 0.639 mmol) to afford quantitatively lithio 3-methyl-4-(4-methylpiperazin-1-yl)benzoate (153.4 mg) as a light orange solid. According to general procedure I, the lithium salt (1.1 eq.,72 mg, 0.3 mmol) was used with 5-quinolinamine (1 eq., 39.3 mg, 0.272 mmol) to the amide bon coupling. The crude residue was purified by flash column chromatography using MeOH in DCM (98/2 to 85/15) as eluent to afford 3-methyl-4-(4-methylpiperazin-1-yl)-N-(quinolin-5-yl)benzamide **(29)** (30.5 mg, 0.0846 mmol, 31 %) as a light orange solid and 92% HPLC purity.

**NMR ¹H (400 MHz, MeOD):** δ 8.87 (dd, *J* = 4.3, 1.6 Hz, 1H), 8.43 (ddd, *J* = 8.6, 1.6, 0.9 Hz, 1H), 8.00 (dt, *J* = 8.6, 1.0 Hz, 1H), 7.94-7.87 (m, 2H), 7.82 (dd, *J* = 8.5, 7.4 Hz, 1H), 7.69 (dd, *J* = 7.4, 1.1 Hz, 1H), 7.56 (dd, *J* = 8.6, 4.3 Hz, 1H), 7.17 (d, *J* = 8.1 Hz, 1H), 3.06 (bt, *J* = 4.7 Hz, 4H), 2.69 (s, 4H), 2.40 (s, 6H).

**NMR ¹³C (101 MHz, MeOD):** δ 169.78 (CO), 156.38 (C_{q}), 151.39 (CH_{Ar}), 149.31 (C_{q}), 135.41 (C_{q}), 134.16 (CH_{Ar}), 133.53 (CH_{Ar}), 131.94 (C_{q}), 130.69 (CH_{Ar}), 129.33 (C_{q}), 127.98 (CH_{Ar}), 127.75 (CH_{Ar}), 126.44 (C_{q}), 125.71 (CH_{Ar}), 122.44 (CH_{Ar}), 119.74 (CH_{Ar}), 56.32 (2CH₂), 51.97 (2CH₂), 46.10 (CH₃), 18.51 (CH₃).

**HRMS (ESI):** m/z calculated for C₂₂H₂₅N₄O⁺, [M+H]⁺ 361.2023, found 361.2023.

### Example 10: Preparation of 4-[2-(dimethylamino)ethoxyl-N-(8-methoxyisoquinolin-5-yl)benzamide (34)

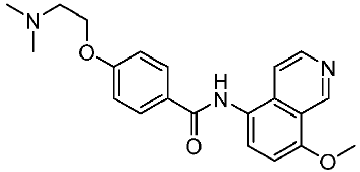

Following general method G and starting from lithio 4-[2-(dimethylamino)ethoxy]benzoate (1 eq., 50 mg, 0.23 mmol) and 8-methoxyisoquinolin-5-amine (1.1 eq., 45 mg, 0.26 mmol), the reaction mixture was stirred overnight at 35°C. 3 equivalents of EDCI were added and the reaction mixture was stirred for 2 days at 35°C. The crude was purified by flash column chromatography using MeOH in DCM as eluent (0 to 15%). The titled compound was obtained as a grey powder (38 mg, 0.23 mmol, 46%).

**NMR ¹H (400 MHz, MeOD):** δ 9.52 (d, *J* = 1.0 Hz, 1H), 8.45 (d, *J* = 6.0 Hz, 1H), 8.03 (d, *J* = 8.8 Hz, 2H), 7.78 - 7.69 (m, 2H), 7.10 (dd, *J* = 8.6, 3.9 Hz, 3H), 4.21 (t, *J* = 5.4 Hz, 2H), 4.08 (s, 3H), 2.82 (t, *J* = 5.4 Hz, 2H), 2.37 (s, 6H).

**NMR ¹³C (126 MHz, MeOD):** δ 168.18 (C_{q}), 162.00 (C_{q}), 155.39 (C_{q}), 146.80 (CH_{Ar}), 142.31 (CH_{Ar}), 133.78 (C_{q}), 130.05 (C_{q}), 129.43 (2CH_{Ar}), 126.15 (CH_{Ar}), 125.08 (C_{q}), 120.82 (CH_{Ar}), 116.32 (CH_{Ar}), 114.09 (2CH_{Ar}), 105.27 (CH_{Ar}), 65.51 (CH₂), 57.59 (CH₂), 55.19 (CH₃), 44.46 (2CH₃).

**HRMS (ESI):** m/z calculated for C₂₁H₂₄N₃O₃⁺, [M+H]+ 336.1802, found 366.1818.

### Example 11: Preparation of 4-(4-methylpiperazin-1-yl)-N-(quinolin-8-yl)benzamide (35)

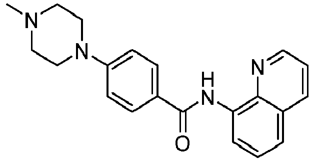

General procedure H was applied to 4-(4-methylpiperazin-1-yl)benzoic acid (1 eq., 250 mg, 1.13 mmol) to obtain 4-(4-methylpiperazin-1-yl)benzoyl chloride (270.94 mg, 1.13 mmol, 100%). One eqalent (50 mg, 0.209 mmol) of benzoyl chloride was then used with 8-aminoquinoline (1.1 eq., 33.22 mg, 0.23 mmol) to the amide bond coupling and the crude residue was purified by flash column chromatography using a solution of 10% MeOH/DCM in pure DCM as eluent (0 to 100%) to led to 4-(4-methylpiperazin-1-yl)-N-(quinolin-8-yl)benzamide (35) (60 mg, 0.17 mmol, 83%) obtained as an orange powder and 97 % HPLC purity.

**NMR ¹H (500 MHz, MeOD)** : δ 8.91 (dd, *J* = 4.2, 1.7 Hz, 1H), 8.76 (dd, *J* = 7.5, 1.4 Hz, 1H), 8.33 (dd, *J* = 8.3, 1.7 Hz, 1H), 7.95 (d, *J* = 8.9 Hz, 2H), 7.63 (dd, *J* = 8.3, 1.5 Hz, 1H), 7.61 - 7.54 (m, 2H), 7.20 - 6.85 (m, 2H), 3.44 - 3.36 (m, 4H), 2.62 (t, *J* = 5.1 Hz, 4H), 2.36 (s, 3H).

**NMR ¹³C (126 MHz, MeOD):** δ 167.26 (CO), 155.24 (C_{q}), 149.96 (CH_{Ar}), 140.17 (C_{q}), 137.80 (CH_{Ar}), 135.72 (C_{q}), 129.79 (2CH_{Ar}), 129.67 (C_{q}), 128.18 (CH_{Ar}), 125.17 (C_{q}), 123.16 (CH_{Ar}), 123.13 (CH_{Ar}), 117.63 (CH_{Ar}), 115.54 (2CH_{Ar}), 55.75 (2CH₂), 48.29 (2CH₂), 46.12 (CH₃).

**HRMS (ESI):** m/z calculated for C₂₁H₂₃N₄O⁺, [M+H]+ 347.1866, found 347.1887.

### Example 12: Preparation of 3-cyano-4-[2-(dimethylamino)ethoxy]-N-(isoquinolin-8-yl)benzamide (36)

### methyl 3-cyano-4-[2-(dimethylamino)ethoxy]benzoate

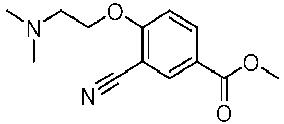

General procedure D was applied to commercially available methyl 3-cyano-4-hydroxybenzoate (1 eq., 205 mg, 1.16 mmol) in acetone (8 mL). The mixture was stirred for 22 hours at 60°C and after extraction with EtOAc and H₂O, methyl 3-cyano-4-[2-(dimethylamino)ethoxy]benzoate was obtained as a light brown oil (119 mg, 0.48 mmol, 41%) without further purification.

**NMR ¹H (400 MHz, MeOD):** δ 8.20 - 8.12 (m, 2H), 7.24 (d, *J* = 8.8 Hz, 1H), 4.33 (t, *J =* 5.3 Hz, 2H), 3.89 (s, 3H), 2.91 (t, *J* = 5.3 Hz, 2H), 2.42 (s, 6H).

### 3-cyano-4-[2-(dimethylamino)ethoxyl-N-(isoquinolin-8-yl)benzamide (36)

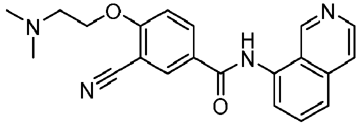

General procedure F was applied to methyl 3-cyano-4-[2-(dimethylamino)ethoxy]benzoate (1 eq., 119 mg, 0.43 mmol) and lithio 3-cyano-4-[2-(dimethylamino)ethoxy]benzoate was obtained as a light beige powder (73 mg, 0.304 mmol, 70 %). According to general procedure G, a solution of lithio 3-cyano-4-[2-(dimethylamino)ethoxy]benzoate (1 eq., 50 mg, 0.208 mmol) and commercially available 8-aminoisoquinoline (1.1 eq., 33 mg, 0.23 mmol), the reaction mixture was stirred 24h at 35°C. The crude was purified by flash column chromatography using MeOH in DCM (0 to 15%) as eluent and 3-cyano-4-[2-(dimethylamino)ethoxy]-N-(isoquinolin-8-yl)benzamide **(36)** (8 mg, 0.022 mmol, 11%) was obtained as a beige powder and 98% HPLC purity.

**NMR ¹H (500 MHz, MeOD):** δ 9.34 (s, 1H), 8.48 (d, *J* = 5.8 Hz, 1H), 8.43 - 8.34 (m, 2H), 7.94 - 7.87 (m, 2H), 7.84 (dd, *J* = 8.3, 7.3 Hz, 1H), 7.78 (dd, *J* = 7.4, 1.2 Hz, 1H), 7.40 (d, *J* = 8.8 Hz, 1H), 4.41 (t, *J* = 5.3 Hz, 2H), 2.97 (t, *J* = 5.3 Hz, 2H), 2.47 (s, 6H), 2.01 (s, 3H).

**NMR ¹³C (126 MHz, MeOD):** δ 167.40 (C_{q}), 164.34 (C_{q}), 149.24 (CH_{Ar}), 143.03 (CH_{Ar}), 138.42 (C_{q}), 135.90 (CH_{Ar}), 135.70 (C_{q}), 134.91 (CH_{Ar}), 132.25 (CH_{Ar}), 128.27 (C_{q}), 126.70 (CH_{Ar}), 126.50 (CH_{Ar}), 125.68 (C_{q}), 122.47 (CH_{Ar}), 116.59 (CN), 113.85 (CH_{Ar}), 103.15 (C_{q}), 69.01 (CH₂), 58.51 (CH₂), 46.16 (2CH₃).

**HRMS (ESI):** m/z calculated for C₂₁H₂₁N₄O₂⁺, [M+H]+ 361.1659, found 361.1663.

### Example 13: Preparation of 4-[2-(dimethylamino)ethoxyl-3-methyl-N-(quinolin-8-yl)benzamide (43)

### methyl 4-[2-(dimethylamino)ethoxy]-3-methylbenzoate

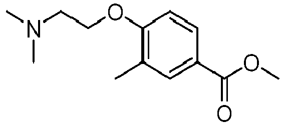

General procedure D was applied to methyl 4-hydroxy-3-methylbenzoate (1 eq., 200 mg, 1.204 mmol) in acetone (3.5 mL). (2-Chloroethyl)dimethylamine hydrochloride (1.5 eq., 260 mg, 1.805 mmol) was added and the reaction was stirred overnight (21h) at 60°C. Methyl 4-[2-(dimethylamino)ethoxy]-3-methylbenzoate (108 mg, 0.46 mmol, 37%) was obtained as a light brown oil without further purification.

**NMR ¹H (400 MHz, MeOD):** δ 7.81 (dd, *J* = 8.6, 2.4 Hz, 2H), 7.78 - 7.72 (m, 2H), 6.91 (d, *J* = 8.6 Hz, 1H), 4.13 (t, *J* = 5.4 Hz, 2H), 3.83 (s, 2H), 2.80 (t, *J* = 5.4 Hz, 2H), 2.35 (s, 5H), 2.21 (s, 3H).

### 4-[2-(dimethylamino)ethoxyl-3-methyl-N-(quinolin-8-yl)benzamide (43)

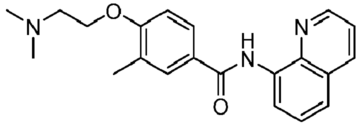

General procedure F was applied to methyl 4-[2-(dimethylamino)ethoxy]-3-methylbenzoate (1 eq., 108 mg, 0.46 mmol) and lithio 4-[2-(dimethylamino)ethoxy]-3-methylbenzoate was obtained quantitatively as a white powder (142 mg). General procedure G was applied to the lithium salt (1 eq., 92 mg, 0.401 mmol) and 4-[2-(dimethylamino)ethoxy]-3-methylbenzoyl chloride was obtained as beige powder (97 mg, 0.401 mmol). The benzoyl chloride was then used with commercially available 8-aminoquinoline (1.1 eq., 60 mg, 0.42 mmol). The mixture was stirred overnight at room temperature and the crude was purified by flash column chromatography using MeOH in DCM as eluent (0 to 10%). 4-[2-(Dimethylamino)ethoxy]-3-methyl-N-(quinolin-8-yl)benzamide **(43)** (30 mg, 0.086 mmol, 22%) was obtained as a yellow powder and 98% HPLC purity.

**NMR ¹H (500 MHz, MeOD):** δ 8.88 (dd, *J* = 4.2, 1.7 Hz, 1H), 8.72 (dd, *J* = 7.6, 1.4 Hz, 1H), 8.30 (dd, *J* = 8.3, 1.7 Hz, 1H), 7.89 (dd, *J* = 8.5, 2.4 Hz, 1H), 7.83 (dd, *J* = 2.5, 0.9 Hz, 1H), 7.63 (dd, *J* = 8.3, 1.3 Hz, 1H), 7.60 - 7.52 (m, 2H), 7.13 (d, *J* = 8.5 Hz, 1H), 4.53 - 4.46 (m, 2H), 3.73 - 3.67 (m, 2H), 3.06 (s, 6H), 2.36 (s, 3H).

**NMR ¹³C (126 MHz, MeOD):** δ 166.86 (C_{q}), 160.41 (C_{q}), 150.03 (CH_{Ar}), 140.08 (C_{q}), 137.80 (CH_{Ar}), 135.47 (C_{q}), 130.93 (CH_{Ar}), 129.61 (C_{q}), 128.81 (C_{q}), 128.56 (C_{q}), 128.09 (CH_{Ar}), 127.87 (CH_{Ar}), 123.43 (CH_{Ar}), 123.19 (CH_{Ar}), 117.80 (CH_{Ar}), 112.24 (CH_{Ar}), 64.07 (CH₂), 57.90 (CH₂), 47.88 (CH₃), 16.71 (CH₃).

**HRMS (ESI):** m/z calculated for C₂₁H₂₄N₃O₂⁺, [M+H]+ 350.1863, found 350.1879.

### Example 14: Preparation of N-(isoquinolin-5-yl)-2-methyl-4-(4-methylpiperazin-1-yl)benzamide (45)

### methyl 2-methyl-4-(4-methylpiperazin-1-yl)benzoate

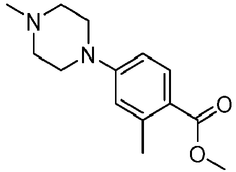

General procedure C was applied to methyl 4-bromo-2-methylbenzoate (1 eq., 204 mg, 0.893 mmol). The residue was purified with by flash column chromatography using EtOAc in heptane (90/10 to 70/30) then MeOH in DCM (98/2 to 90/10) to afford methyl 2-methyl-4-(4-methylpiperazin-1-yl)benzoate (211 mg, 0.853 mmol, 95 %) as an orange oil.

**NMR ¹H (500 MHz, CDCl₃)** : δ 7.85 (d, *J* = 8.6 Hz, 1H), 6.67 (d, *J* = 2.7 Hz, 1H), 6.64 (d, *J* = 3.2 Hz, 2H), 3.79 (s, 3H), 3.36 - 3.19 (m, 4H), 2.56 (s, 3H), 2.49 (t, *J* = 5.1 Hz, 4H), 2.30 (s, 3H).

**NMR ¹³C (126 MHz, CDCl₃) :** δ 167.57 (CO), 153.31 (C_{q}), 142.49 (C_{q}), 132.66 (CH_{Ar}), 118.83 (C_{q}), 116.96 (CH_{Ar}), 111.42 (CH_{Ar}), 54.80 (2CH₂), 51.25 (CH₃), 47.40 (2CH₂), 46.11 (CH₃), 22.74 (CH₃).

### N-(isoquinolin-5-yl)-2-methyl-4-(4-methylpiperazin-1-yl)benzamide ( 45)

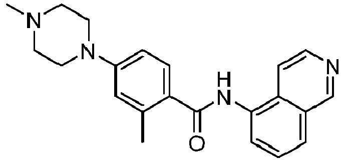

General procedure F was applied to methyl 3-methyl-4-(4-methylpiperazin-1-yl)benzoate (1 eq., 158 mg, 0.639 mmol) to afford quantitatively lithio 3-methyl-4-(4-methylpiperazin-1-yl)benzoate (181 mg). General procedure G was applied to the lithium salt (1 eq., 100 mg, 0.42 mmol) to obtained 2-methyl-4-(4-methylpiperazin-1-yl)benzoyl chloride (105 mg, 0.42 mmol, 100%). The benzoyl chloride (1.1 eq., 50 mg, 0.2 mmol) was used with 5-isoquinolinamine (1 eq., 25.93 mg, 0.18 mmol) to the amide coupling and the crude was purified by flash column chromatography using a solution of 10% MeOH/DCM in pure DCM (0 to 100%) as eluent. The expected N-(5-fluoroisoquinolin-8-yl)-4-(4-methylpiperazin-1-yl)benzamide **(45)** (8 mg, 0.022 mmol, 12%) was obtained as a yellow powder and 95% HPLC purity.

**NMR ¹H (500 MHz, CDCl₃)** : δ 9.27 (s, 1H, CONH), 8.55 (d, *J* = 6.0 Hz, 1H), 8.33 (d, *J* = 7.6 Hz, 1H), 7.88 (s, 1H), 7.83 (dt, *J* = 8.1, 1.0 Hz, 1H), 7.69 - 7.63 (m, 2H), 7.63 - 7.53 (m, 1H), 6.80 (d, *J* = 7.7 Hz, 2H), 3.33 (dd, *J* = 6.3, 3.9 Hz, 4H), 2.61 - 2.58 (m, 4H), 2.58 (s, 3H), 2.37 (s, 3H).

**NMR ¹³C (126 MHz, CDCl₃)** : δ 168.37 (CO), 153.31 (CH_{Ar}), 152.88 (C_{q}), 143.38 (CH_{Ar}), 139.44 (C_{q}), 132.44 (C_{q}), 129.70 (C_{q}), 129.28 (C_{q}), 128.73 (CH_{Ar}), 127.61 (CH_{Ar}), 125.57 (C_{q}), 124.92 (CH_{Ar}), 124.17 (CH_{Ar}), 117.95 (CH_{Ar}), 114.01 (CH_{Ar}), 112.26 (CH_{Ar}), 54.97 (2CH₂), 48.03 (2CH₂), 46.24 (CH₃), 21.22 (CH₃).

**HRMS (ESI):** m/z calculated for C₂₂H₂₅N₄O⁺, [M+H]⁺ 361.2023; found 361.2030.

### Example 15: Preparation of 4-[2-(dimethylamino)ethoxy]-2-methyl-N-(quinolin-5-yl)benzamide (47)

### methyl 4-[2-(dimethylamino)ethoxy]-2-methylbenzoate

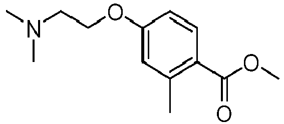

General procedure D was applied to methyl 4-hydroxy-2-methylbenzoate (1 eq., 200 mg, 1.204 mmol) and (2-chloroethyl)dimethylamine hydrochloride (1.5 eq., 260 mg, 1.805 mmol). The reaction was stirred overnight (21h) at 60°C and the crude was purified by flash column chromatography on silica gel using EtOAc in heptane (25/75) then MeOH in DCM (98/2 to 85/15) as eluent to afford methyl 4-[2-(dimethylamino)ethoxy]-2-methylbenzoate (198 mg, 0.83 mmol, 69%) as an oil.

**NMR ¹H (400 MHz, MeOD):** δ 7.77 (d, *J* = 8.4 Hz, 1H), 6.74 - 6.66 (m, 2H), 4.01 (t, *J* = 5.5 Hz, 2H), 3.71 (s, 3H), 2.64 (t, *J* = 5.5 Hz, 2H), 2.44 (s, 3H), 2.22 (s, 6H).

**NMR ¹³C (101 MHz, MeOD):** δ 132.63, 117.08, 111.22, 65.31, 57.56, 57.56, 50.61, 48.50, 44.48, 21,04

### 4-[2-(dimethylamino)ethoxyl-2-methyl-N-(quinolin-5-yl)benzamide (47)

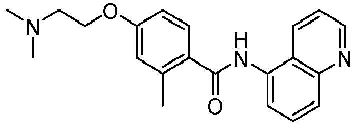

General procedure F was applied to methyl 4-[2-(dimethylamino)ethoxy]-2-methylbenzoate (1 eq., 198 mg, 0.83 mmol) and lithio 4-[2-(dimethylamino)ethoxy]-2-methylbenzoate was obtained quantitatively as a white powder (296 mg). General procedure G was applied to the lithium salt (1 eq., 296 mg, 1.29 mmol) and 4-[2-(dimethylamino)ethoxy]-2-methylbenzoyl chloride was obtained as a white powder (312.17 mg, 1.29 mmol). The benzoyl chloride (1 eq., 298 mg, 1.23 mmol) was then used with commercially available 5-aminoquinoline (1 eq., 177 mg, 1.23 mmol) and the mixture was stirred overnight at room temperature. The crude was purified on silica gel using MeOH in DCM as eluent (0 to 10%). The obtained fraction was triturated in ether and led to 4-[2-(dimethylamino)ethoxy]-2-methyl-N-(quinolin-5-yl)benzamide **(47)** (30 mg, 0.086 mmol, 7%) obtained as a yellow powder and 93.5% HPLC purity.

**NMR ¹H (500 MHz, MeOD):** δ 8.88 (dd, *J* = 4.2, 1.7 Hz, 1H), 8.51 (d, *J* = 8.5 Hz, 1H), 7.98 (d, *J* = 8.4 Hz, 1H), 7.81 (t, *J* = 7.9 Hz, 1H), 7.77 (d, *J* = 7.4 Hz, 1H), 7.69 (d, *J* = 8.2 Hz, 1H), 7.58 (dd, *J* = 8.6, 4.3 Hz, 1H), 6.92 (d, *J* = 8.8 Hz, 2H), 4.18 (t, *J* = 5.4 Hz, 2H), 2.83 (t, *J =* 5.4 Hz, 2H), 2.54 (s, 3H), 2.38 (s, 6H).

**NMR ¹³C (126 MHz, MeOD):** δ 170.79 (C_{q}), 160.29 (C_{q}), 150.00, 147.89 (C_{q}), 138.84 (C_{q}), 133.72 (C_{q}), 132.49 (CH_{Ar}), 129.32 (CH_{Ar}), 129.15 (CH_{Ar}), 128.32 (C_{q}), 126.40 (CH_{Ar}), 124.54 (C_{q}), 123.72 (CH_{Ar}), 121.06 (CH_{Ar}), 116.74 (CH_{Ar}), 111.27 (CH_{Ar}), 65.21 (CH₂), 57.59 (CH₂), 44.41 (CH₃), 19.12 (CH₃).

**HRMS (ESI):** m/z calculated for C₂₁H₂₄N₃O₂⁺, [M+H]+ 350.1863, found 350.1873.

### Example 16: Preparation of 4-[2-(dimethylamino)ethoxy]-N-(isoquinolin-5-yl)-3-(trifluoromethyl) benzamide (48)

### methyl 4-[2-(dimethylamino)ethoxyl-3-(trifluoromethyl)benzoate

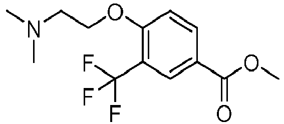

General procedure D was applied to methyl 4-hydroxy-3-(trifluoromethyl)benzoate (1 eq., 200 mg, 0.908 mmol) and 2-dimethylaminoethyl chloride hydrochloride (1.5 eq., 196 mg, 1.36 mmol). The reaction was stirred overnight (21h) at 60°C and methyl 4-[2-(dimethylamino)ethoxy]-3-(trifluoromethyl)benzoate (233 mg, 0.8 mmol, 88 %) was obtained as a light brown oil whithout further purification and 96% HPLC purity.

**NMR ¹H (400 MHz, MeOD):** δ 8.57 (d, *J* = 2.3 Hz, 1H), 8.11 (dd, *J* = 8.8, 2.3 Hz, 1H), 7.23 (d, *J* = 8.6 Hz, 1H), 4.33 (t, *J* = 5.3 Hz, 2H), 3.89 (s, 3H), 2.82 (t, *J* = 5.3 Hz, 2H), 2.33 (s, 6H).

**NMR ¹³C (101 MHz, MeOD):** δ 171.41, 165.54, 135.09, 122.09, 112.73, 67.60, 60.08, 57.19, 51.36, 44.75, 19.51, 13.12.

**NMR ¹⁹F (376 MHz, MeOD, decoupled):** δ -63.85.

### 4-[2-(Dimethylamino)ethoxyl-N-(isoquinolin-5-yl)-3-(trifluoromethyl) benzamide (48)

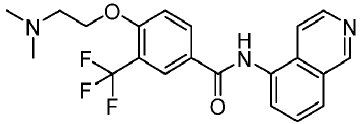

General method F (preparation of a lithium salt) was applied to methyl 4-[2-(dimethylamino)ethoxy]-3-(trifluoromethyl)benzoate (1 eq., 233 mg, 0.8 mmol) and lithium 4-[2-(dimethylamino)ethoxy]-3-(trifluoromethyl)benzoate was obtained quantitatively as a white powder (233 mg). General procedure G was applied to the lithium salt (1 eq., 296 mg, 1.29 mmol) and 4-[2-(dimethylamino)ethoxy]-3-(trifluoromethyl)benzoyl chloride ( 243.29 mg, 0.82 mmol) was obtained a white solid. The benzoyl chloride was then used with commercially available 5-isoquinolinamine (1.2 eq., 141 mg, 0.98 mmol) and the mixture was stirred overnight at room temperature. The crude was purified by flash column chromatography using MeOH in DCM as eluent (0 to 10%) to afford 4-[2-(dimethylamino)ethoxy]-N-(isoquinolin-5-yl)-3-(trifluoromethyl)benzamide **(48)** (42 mg, 0.104 mmol, 11%) obtained as a yellow powder and 99% HPLC purity.

**NMR ¹H (500 MHz, MeOD):** δ 9.27 (d, *J* = 1.0 Hz, 1H), 8.45 (d, *J* = 6.0 Hz, 1H), 8.36 (d, *J* = 2.3 Hz, 1H), 8.33 (dd, *J* = 8.7, 2.4 Hz, 1H), 8.05 (d, *J* = 8.3 Hz, 1H), 7.92 - 7.80 (m, 2H), 7.72 (dd, *J* = 8.3, 7.4 Hz, 1H), 7.36 (d, *J* = 8.7 Hz, 1H), 4.35 (t, *J* = 5.4 Hz, 2H), 2.90 (t, *J* = 5.4 Hz, 2H), 2.41 (s, 6H).

**NMR ¹³C (126 MHz, MeOD):** δ 170.43 (C_{q}), 163.32 (C_{q}), 156.00 (CH_{Ar}, d, J = 20.1 Hz), 145.5 (CH_{Ar}, d, J = 15.8 Hz), 137.48 (CH_{Ar}, d, J = 10.8 Hz), 136.63 (C_{q}), 136.21 (C_{q}), 133.31 (C_{q}), 132.24 (C_{q}), 131.26 (CH_{Ar}), 130.76 (CH_{Ar}, m, J = 14.5 Hz), 130.39 (CH_{Ar}), 129.80 (CH_{Ar}),126.3(CF₄, q, J = 272 Hz), 122.5 (C_{q}-CF₃, q, J = 31.3 Hz) 120.46 (CH_{Ar}), 116.77 (CH_{Ar}), 71.52 (CH₂), 61.17 (CH₂), 48.67 (CH₃).

### NMR ¹⁹F (471 MHz, MeOD, decoupled): δ -59.75.

**HRMS (ESI):** m/z calculated for C₂₁H₂₁F₃N₃O₂⁺, [M+H]+ 404.1580, found 404.1595.

### Example 17: Preparation of 4-[2-(dimethylamino)ethoxy]-N-(quinolin-5-yl)-2-(trifluoromethyl) benzamide (49)

### methyl 4-[2-(dimethylamino)ethoxyl-2-(trifluoromethyl)benzoate

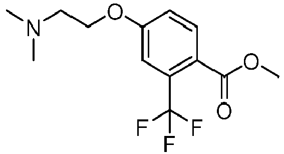

General procedure D was applied to methyl 4-hydroxy-2-(trifluoromethyl)benzoate (1 eq., 200 mg, 0.908 mmol) and (2-chloroethyl)dimethylamine hydrochloride (1.5 eq., 196 mg, 1.36 mmol). The reaction was stirred overnight (21h) at 60°C and methyl 4-[2-(dimethylamino)ethoxy]-2-(trifluoromethyl)benzoate (144 mg, 0.49 mmol, 54%) was obtained without further purification and 96% of purity.

**NMR ¹H (500 MHz, MeOD):** δ 7.89 - 7.81 (m, 1H), 7.34 (d, *J* = 2.6 Hz, 1H), 7.24 (dd, *J* = 8.7, 2.6 Hz, 1H), 4.21 (t, *J* = 5.4 Hz, 2H), 3.88 (s, 3H), 2.80 (t, *J* = 5.4 Hz, 2H), 2.35 (s, 6H)

**NMR ¹³C (126 MHz, MeOD):** δ 166.43, 161.06, 132.93, 116.25, 113.72, 113.67, 65.93, 57.39, 51.62, 44.44.

**NMR ¹⁹F (376 MHz, MeOD, decoupled):** δ -60.96.

### 4-[2-(Dimethylamino)ethoxyl-N-(quinolin-5-yl)-2-(trifluoromethyl)benzamide (49)

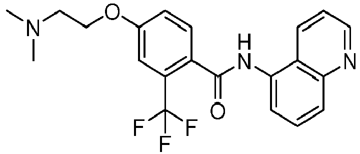

General procedure F was applied to methyl 4-[2-(dimethylamino)ethoxy]-2-(trifluoromethyl)benzoate (1 eq., 144 mg, 0.49 mmol) and lithio 4-[2-(dimethylamino)ethoxy]-2-(trifluoromethyl)benzoate was obtained quantitatively as a white powder (160 mg). General procedure G was applied to the lithium salt (1 eq., 160 mg, 0.57 mmol) and 4-[2-(dimethylamino)ethoxy]-2-(trifluoromethyl)benzoyl chloride (167 mg, 0.57 mmol) was obtained as a white powder. The benzoyl chloride (1 eq., 160 mg, 0.54 mmol) was then used with commercially available 5-isoquinolinamine (1 eq., 141 mg, 0.98 mmol). The mixture was stirred overnight at room temperature and the crude was purified by flash column chromatography using MeOH in DCM as solvent (0 to 10%). 4-[2-(Dimethylamino)ethoxy]-N-(quinolin-5-yl)-2-(trifluoromethyl)benzamide **(49)** (22 mg, 0.055 mmol, 10%) was obtained as a yellow powder and 99% HPLC purity.

**NMR ¹H (500 MHz, MeOD):** δ 8.89 (dd, *J* = 4.3, 1.6 Hz, 1H), 8.54 (d, *J* = 8.2 Hz, 1H), 8.00 (dd, *J* = 6.9, 2.0 Hz, 1H), 7.83 (dd, *J* = 8.2, 2.3 Hz, 3H), 7.59 (dd, *J* = 8.6, 4.3 Hz, 1H), 7.43 - 7.31 (m, 2H), 4.25 (t, *J* = 5.4 Hz, 2H), 2.85 (t, *J* = 5.3 Hz, 2H), 2.38 (s, 6H).

**NMR ¹³C (126 MHz, MeOD):** δ 168.60 (C_{q}), 159.90 (C_{q}), 150.07 (CH_{Ar}), 147.84 (C_{q}), 133.13 (C_{q}), 132.17 (CH_{Ar}), 130.33 (CH_{Ar}), 129.32 (CH_{Ar}), 128.64 (C-CF3, q, J = 32.1 Hz), 127.87 (C_{q}), 126.59(CH_{Ar}), 124.32 (C_{q}), 123.73 (CF_{3,} q, J = 272 Hz), 123.53 (CH_{Ar}), 121.12 (CH_{Ar}), 117.08 (CH_{Ar}), 113.02 (CH_{Ar}, d, J = 5.1 Hz), 65.86 (CH₂), 57.46 (CH₂), 44.41 (CH₃).

### NMR ¹⁹F (471 MHz, MeOD, decoupled): δ -60.36.

**HRMS (ESI):** m/z calculated for C₂₁H₂₁F₃N₃O₂⁺, [M+H]⁺ 404.1580, found 404.1595.

### Example 18: Preparation of 3-fluoro-N-(5-fluoroisoquinolin-8-yl)-4-{5-methyl-octahydropyrrolo[3,4-c]pyrrol-2-yl}benzamide (53)

### methyl 3-fluoro-4-{5-methyl-octahydropyrrolo[3,4-c]pyrrol-2-yl}benzoate

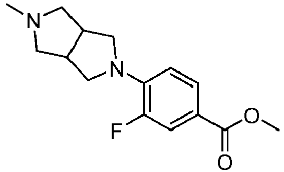

General procedure C was applied to methyl 4-bromo-3-fluorobenzoate (1 eq., 200 mg, 0.86 mmol) and 2-methyl-octahydropyrrolo[3,4-c]pyrrole dihydrochloride (1.2 eq., 1.03 mmol, 205 mg). The reaction was allowed to cool at room temperature. The mixture was diluted in EtOAc, filtered on a Celite pad and washed with EtOAc. The solvent was removed under reduced pressure. The residue was purified by flash column chromatography on silica gel (Heptane/EtOAc 90/10 to 70/30 then DCM/MeOH 98/2 to 90/10) to afford methyl 3-fluoro-4-{5-methyl-octahydropyrrolo[3,4-c]pyrrol-2-yl}benzoate (111 mg, 0.4 mmol, 46.47%) as an orange oil.

**¹H NMR (400 MHz, Methanol-*d*4):** δ 7.61 - 7.37 (m, 2H), 6.64 (t, *J* = 8.7 Hz, 1H), 3.72 (s, 3H), 2.81 (dt, *J* = 9.1, 3.4 Hz, 2H), 2.78 - 2.72 (m, 2H), 2.29 - 2.22 (m, 2H), 2.21 (s, 3H).

**¹³C NMR (101 MHz, Methanol-*d*4):** δ 166.39, 166.37, 152.94, 150.53, 141.31, 141.22, 126.44, 126.42, 119.75, 119.68, 116.71, 116.48, 116.40, 116.35, 62.26, 54.92, 54.86, 51.02, 41.74, 41.73, 40.57.

**¹⁹F NMR (376 MHz, Methanol-*d*4):** δ -125.33.

### 3-fluoro-N-(5-fluoroisoquinolin-8-yl)-4-{5-methyl-octahydropyrrolo[3,4-c]pyrrol-2-yl}benzamide (53)

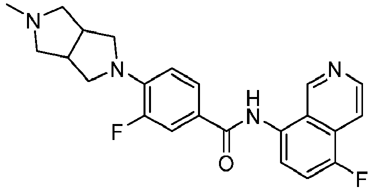

General procedure F was applied to methyl 3-fluoro-4-{5-methyl-octahydropyrrolo[3,4-c]pyrrol-2-yl}benzoate (1 eq., 111 mg, 0.4 mmol) to afford lithio 3-fluoro-4-{5-methyl-octahydropyrrolo[3,4-c]pyrrol-2-yl}benzoate (107.77 mg, 0.4 mmol, 100%) as a yellow powder.

General procedure H than then applied to the lithium salt (1 eq., 100 mg, 0.37 mmol) to yield 4-[2-(dimethylamino)ethoxy]-2-(trifluoromethyl)benzoyl chloride (100 mg, 0.35 mmol, 94. 5%) as a white powder that was used directly in the next step without further purification with 5-fluoroisoquinolin-8-amine (0.52 eq., 30 mg, 0.18 mmol). The crude was purified on silica gel using methanol in DCM as solvent (0 to 10%). The expected 3-fluoro-N-(5-fluoroisoquinolin-8-yl)-4-{5-methyl-octahydropyrrolo[3,4-c]pyrrol-2-yl}benzamide (53) was obtained as a yellow powder (13.5 mg, 0.033 mmol, 18%) and 95% HPLC purity.

**NMR ¹H (500 MHz, Methanol-*d*4):** δ 9.31 (t, *J* = 1.4 Hz, 1H), 8.58 (d, *J* = 5.9 Hz, 1H), 8.01 (dd, *J* = 5.8, 1.0 Hz, 1H), 7.82 (dd, *J* = 8.5, 2.2 Hz, 1H), 7.75 (dd, *J* = 14.9, 2.1 Hz, 1H), 7.68 (dd, *J* = 8.3, 4.7 Hz, 1H), 7.56 (dd, *J* = 9.7, 8.3 Hz, 1H), 6.95 (t, *J* = 8.7 Hz, 1H), 3.52 - 3.41 (m, 4H), 2.99 (dq, *J* = 12.9, 7.0, 5.5 Hz, 4H), 2.51 - 2.46 (m, 2H), 2.40 (s, 3H).

**NMR ¹³C (126 MHz, DMSO):** δ 168.69 (CO), 157.10 (d, *J* = 252.3 Hz, C-F), 153.66 (d, *J* = 243.0 Hz C-F), 149.52 (d, *J* = 2.4 Hz, CH_{Ar}), 143.64 (d, *J* = 1.9 Hz, CH_{Ar}), 142.16 (d, *J* = 9.5 Hz, C_{q}), 132.26 (d, *J* = 4.4 Hz, C_{q}), 128.24 (d, *J* = 19.1 Hz, C_{q}), 126.92 (d, *J* = 7.8 Hz, C_{q}), 126.56 (d, *J* = 4.7 Hz, CH_{Ar}), 125.99 (d, *J* = 2.5 Hz, CH_{Ar}), 124.88 (d, *J* = 6.5 Hz, C_{q}), 118.19 (d, *J* = 4.9 Hz, CH_{Ar}), 116.77 (d, *J* = 23.6 Hz, CH_{Ar}), 115.55 (d, *J* = 20.5 Hz, CH_{Ar}), 114.92 (d, *J* = 3.8 Hz, CH_{Ar}), 63.62 (CH₃), 56.30 (d, *J* = 5.9 Hz, 2CH), 43.09 (2CH₂), 41.85 (2CH₂).

**¹⁹F NMR (471 MHz, Methanol-*d*4):** δ -124.76, -126.65.

**HRMS (ESI):** m/z calculated for C₂₃H₂₃F₂N₄O⁺, [M+H]⁺ 409.1834, found 409.1849.

### Example 19: N-(8-methoxyisoquinolin-5-yl)-3-methyl-4-{7-methyl-2,7-diazaspiro[3.5]nonan-2-yl}benzamide (54)

### methyl 3-methyl-4-{7-methyl-2,7-diazaspiro[3.5]nonan-2-yl}benzoate

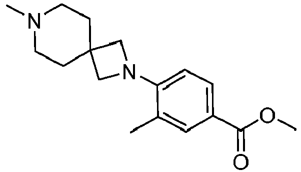

General procedure C was applied to methyl methyl 4-bromo-3-methylbenzoate (1 eq., 200 mg, 0.87 mmol) and 7-methyl-2,7-diazaspiro[3.5]nonane dihydrochloride (1.2 eq., 223 mg, 1.048 mmol). The reaction was allowed to cool at room temperature. The mixture was diluted in EtOAc, filtered on a Celite pad and washed with EtOAc. The solvent was removed under reduced pressure. The residue was purified by flash column chromatography on silica gel (Heptane/EtOAc 90/10 to 70/30 then DCM/MeOH 98/2 to 90/10) to afford methyl 3-methyl-4-{7-methyl-2,7-diazaspiro[3.5]nonan-2-yl}benzoate (114 mg, 0.4 mmol, 45.28%) as an yellow oil.

**¹H NMR (400 MHz, Methanol-*d*4):** δ 7.68 (dd, *J* = 8.5, 2.1 Hz, 1H), 7.62 - 7.57 (m, 1H), 6.37 (d, *J* = 8.5 Hz, 1H), 3.80 (s, 3H), 3.74 (s, 4H), 2.74 - 2.30 (m, 4H), 2.27 (s, 3H), 2.22 (s, 3H), 1.83 (t, *J* = 5.6 Hz, 4H).

### N-(8-methoxyisoquinolin-5-yl)-3-methyl-4-{7-methyl-2,7-diazaspiro[3.5]nonan-2-yl}benzamide (54)

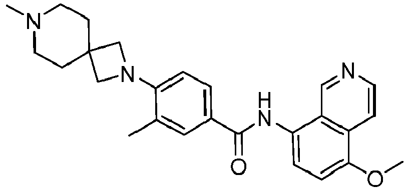

General procedure F was applied to methyl methyl 3-methyl-4-{7-methyl-2,7-diazaspiro[3.5]nonan-2-yl}benzoate (1 eq., 161.5 mg, 0.56 mmol) to afford lithio 3-methyl-4-{7-methyl-2,7-diazaspiro[3.5]nonan-2-yl}benzoate (165 mg, 0.56 mmol, 100%) as a yellow powder.

General procedure H than then applied to the lithium salt (1.28 eq., 114 mg, 0.41 mmol) to quantitatively yield 3-methyl-4-(7-methyl-2,7-diazaspiro[3.5]nonan-2-yl)benzoyl chloride (100 mg, 0.34 mmol, 83%) as a white powder that was used directly in the next step without further purification with 8-methoxyisoquinolin-5-amine (1 eq., 56.66 mg, 0.33 mmol). The crude was purified on silica gel using methanol in DCM as solvent (0 to 10%). The expected N-(8-methoxyisoquinolin-5-yl)-3-methyl-4-{7-methyl-2,7-diazaspiro[3.5]nonan-2-yl}benzamide **(54)** (18 mg, 0.042 mmol, 12.85%) was obtained as a light brown powder.

**NMR ¹H (500 MHz, Methanol-*d*4):** δ 9.51 (d, *J* = 1.0 Hz, 1H), 8.44 (d, *J* = 6.1 Hz, 1H), 7.79 (dd, *J* = 8.4, 2.3 Hz, 1H), 7.74 - 7.67 (m, 3H), 7.09 (d, *J* = 8.3 Hz, 1H), 6.52 (d, *J* = 8.4 Hz, 1H), 4.08 (s, 3H), 3.80 (s, 4H), 2.43 (s, 4H), 2.31 (s, 3H), 2.30 (s, 3H), 1.89 (t, *J* = 5.6 Hz, 4H).

**NMR ¹³C (126 MHz, Methanol-*d*4):** δ 170.09 (CO), 156.62 (C_{q}), 154.87 (C_{q}), 148.14 (CH_{Ar}), 143.59 (CH_{Ar}), 135.26 (C_{q}), 132.28 (CH_{Ar}), 131.38 (CH_{Ar}), 127.94 (CH_{Ar}), 126.74 (C_{q}), 124.83 (C_{q}), 124.23 (C_{q}), 122.21 (C_{q}), 117.82 (CH_{Ar}), 113.09 (CH_{Ar}), 106.66 (CH_{Ar}), 64.35 (2CH₂), 56.57 (CH₃), 53.56 (2CH₂), 46.25 (CH₃), 36.26 (2CH₂), 34.31 (C_{q}), 19.98 (CH₃).

**HRMS (ESI):** m/z calculated for C₂₆H₃₁N₄O₂⁺, [M+H]+ 431.2442, found 431.2449.

### Example 20: 3-methyl-4-[(1-methylpyrrolidin-3-yl)methoxy]-N-(quinolin-8-yl)benzamide (55)

### 3-methyl-4-[(1-methylpyrrolidin-3-yl)methoxy]benzoic acid

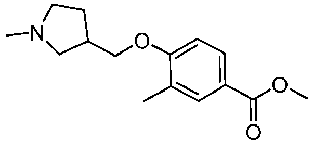

General procedure E was applied to (1-methylpyrrolidin-3-yl)methanol (1 eq., 200 mg, 0.205 mL, 1.74 mmol) , triphenylphosphine (1.1 eq., 501.009 mg, 1.91 mmol) and methyl 4-hydroxy-3-methylbenzoate (1.1 eq., 317.42 mg, 1.91 mmol). The crude was purified on silica using MeOH in DCM as eluent (0 -> 10%) to give methyl 3-methyl-4-[(1-methylpyrrolidin-3-yl)methoxy]benzoate (150 mg, 0.57 mmol, 32.803%) as light yellow oil.

**¹H NMR (400 MHz, Methanol-*d*4):** δ 7.87 - 7.68 (m, 2H), 6.89 (d, *J* = 8.5 Hz, 1H), 4.08 - 3.87 (m, 2H), 3.83 (s, 3H), 2.86 (dd, *J* = 9.8, 7.9 Hz, 1H), 2.79 - 2.66 (m, 2H), 2.62 (ddd, *J* = 9.6, 8.1, 6.3 Hz, 1H), 2.49 (dd, *J* = 9.8, 6.3 Hz, 1H), 2.40 (d, *J* = 4.0 Hz, 3H), 2.18 (s, 3H), 2.13 - 2.01 (m, 1H), 1.67 (ddt, *J* = 13.7, 8.1, 5.9 Hz, 1H).

**¹³C NMR (101 MHz, Methanol-*d*4):** δ 167.10, 160.94, 131.42, 129.11, 126.31, 121.75, 110.07, 70.54, 58.62, 55.43, 50.92, 40.89, 37.40, 27.32, 15.05.

### 3-methyl-4-[(1-methylpyrrolidin-3-yl)methoxyl-N-(quinolin-8-yl)benzamide (55)

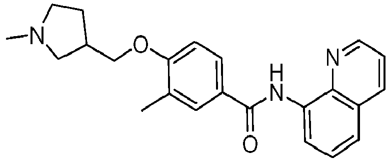

General procedure F was applied to methyl 3-methyl-4-[(1-methylpyrrolidin-3-yl)methoxy]benzoate (1 eq., 150 mg, 0.57 mmol) to yield lithio 3-methyl-4-[(1-methylpyrrolidin-3-yl)methoxy]benzoate (100 mg, 73%) was obtained as a yellow powder.

General procedure H was then applied to lithio({3-methyl-4-[(1-methylpyrrolidin-3-yl)methoxy]phenyl})methanone (1.31 eq., 100 mg, 0.42 mmol). The crude benzoylchloride (white solid) was used directly in the next step without further purification, to react with 8-quinolinamine (1 eq., 51.28 mg, 0.36 mmol) to afford 3-methyl-4-[(1-methylpyrrolidin-3-yl)methoxy]-N-(quinolin-8-yl)benzamide **(55)** (81 mg, 0.22 mmol, 60.65%) as a yellow powder.

**NMR ¹H (500 MHz, Methanol-*d*4):** δ 8.78 (dd, *J* = 4.2, 1.7 Hz, 1H), 8.66 (dd, *J* = 7.1, 1.8 Hz, 1H), 8.16 (dd, *J* = 8.3, 1.6 Hz, 1H), 7.72 - 7.68 (m, 1H), 7.65 (dd, *J* = 2.4, 0.9 Hz, 1H), 7.51 - 7.42 (m, 3H), 6.83 (d, *J* = 8.5 Hz, 1H), 3.87 - 3.77 (m, 2H), 2.79 (dd, *J* = 9.8, 8.0 Hz, 1H), 2.69 - 2.59 (m, 2H), 2.53 (ddd, *J* = 9.5, 8.0, 6.4 Hz, 1H), 2.39 (dd, *J* = 9.9, 6.5 Hz, 1H), 2.34 (s, 3H), 2.16 (s, 3H), 2.03 (dddd, *J* = 13.0, 9.9, 7.7, 6.4 Hz, 1H), 1.61 (ddt, *J* = 13.7, 8.1, 5.8 Hz, 1H).

**NMR ¹³C (126 MHz, Methanol-*d*4):** δ 166.71 (CO), 161.49 (C_{q}), 149.80 (CH_{Ar}), 139.88 (C_{q}), 137.61 (CH_{Ar}), 135.48 (C_{q}), 130.47 (CH_{Ar}), 129.44 (C_{q}), 128.07 (C_{q}), 128.04 (CH_{Ar}), 127.70 (CH_{Ar}), 127.38 (C_{q}), 123.07 (CH_{Ar}), 123.03 (CH_{Ar}), 117.45 (CH_{Ar}), 111.62 (CH_{Ar}), 71.85 (CH₂), 59.97 (CH₂), 56.74 (CH₂), 42.22 (CH₃), 38.73 (CH), 28.68 (CH₂), 16.55 (CH₃).

**HRMS (ESI):** m/z calculated for C₂₃H₂₆N₃O₂⁺, [M+H]⁺ 376.2020, found 376.2036.

### Example 21: 2-Fluoro-N-(isoquinolin-8-yl)-4-[4-(propan-2-yl)piperazin-1-yl]benzamide (57)

### Methyl 2-fluoro-4-[4-(propan-2-yl)piperazin-1-yl]benzoate

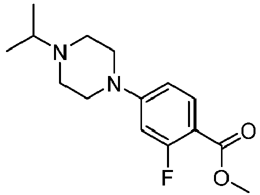

General procedure C was applied to methyl 4-bromo-2-fluorobenzoate (1 eq., 300 mg, 1.29 mmol) and 1-isopropylpiperazine (1.2 eq., 198.076 mg, 0.22 mL, 1.54 mmol). The reaction was allowed to cool at room temperature and the mixture was diluted in EtOAc, filtered on a Celite pad and washed with EtOAc. The solvant was removed under reduced pressure. The residue was purify by flash column chromatography using a solution of 10% MeOH in DCM in pure DCM (0 to 100%) to afford methyl 2-fluoro-4-[4-(propan-2-yl)piperazin-1-yl]benzoate as an orange solid (193.4 mg, 0.69 mmol, 53.59%).

**¹H NMR (400 MHz, MeOD):** δ 7.77 (t, *J* = 8.8 Hz, 1H), 6.74 (dd, *J* = 9.0, 2.5 Hz, 1H), 6.64 (dd, *J* = 15.1, 2.5 Hz, 1H), 3.83 (s, 3H), 3.46 - 3.22 (m, 4H), 2.72 (dd, *J* = 13.2, 6.7 Hz, 1H), 2.67 (dd, *J* = 6.1, 4.2 Hz, 4H), 1.12 (s, 3H), 1.10 (s, 3H).

**¹⁹F NMR (376 MHz, MeOD):** δ -109.05.

**¹³C NMR (126 MHz, MeOD):** δ 166.50 (d, *J* = 4.2 Hz, CO), 165.23 (d, *J* = 256.5 Hz, CF), 157.25 (d, *J* = 11.4 Hz, C_{q}), 134.19 (d, *J* = 2.8 Hz, CH_{Ar}), 110.35 (d, *J* = 2.3 Hz, CH_{Ar}), 107.72 (d, *J* = 10.1 Hz, C_{q}), 102.03 (d, *J* = 27.2 Hz, CH_{Ar}), 55.93 (CH), 52.12 (CH₃), 49.53 (2CH₂), 47.94 (2CH₂), 18.64 (2CH₃).

### Lithio 2-fluoro-4-[4-(propan-2-yl)piperazin-1-yl]benzoate

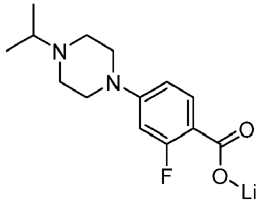

General procedure F was applied to methyl 2-fluoro-4-[4-(propan-2-yl)piperazin-1-yl]benzoate (1 eq., 190 mg, 0.68 mmol) to afford quantitatively lithio 2-fluoro-4-[4-(propan-2-yl)piperazin-1-yl]benzoate (202.1 mg, 0.74 mmol) as a beige powder.

**¹H NMR (500 MHz, MeOD):** δ 7.64 (t, *J* = 8.8 Hz, 1H), 6.69 (dd, *J* = 8.7, 2.5 Hz, 1H), 6.58 (dd, *J* = 14.2, 2.4 Hz, 1H), 3.29 - 3.23 (m, 4H), 2.80 - 2.61 (m, 5H, 2CH₂ + CH), 1.12 (s, 3H), 1.11 (s, 3H).

**¹⁹F NMR (376 MHz, MeOD):** δ -113.00.

**¹³C NMR (126 MHz, MeOD):** δ 173.22 (d, *J* = 2.7 Hz, CO), 163.72 (d, *J* = 249.4 Hz, CF), 155.09 (d, *J* = 10.4 Hz, C_{q}), 133.40 (d, *J* = 4.4 Hz, CH_{Ar}), 118.03 (d, *J* = 12.4 Hz, C_{q}), 110.78 (d, *J* = 2.7 Hz, CH_{Ar}), 102.99 (d, *J* = 27.7 Hz, CH_{Ar}), 55.95 (CH), 49.72 (2CH₂), 48.96 (2CH₂), 18.68 (2CH₃).

### 2-Fluoro-N-(isoquinolin-8-yl)-4-[4-(propan-2-yl)piperazin-1-yl]benzamide

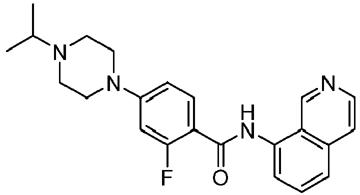

General procedure H was applied to lithio 2-fluoro-4-[4-(propan-2-yl)piperazin-1-yl]benzoate (1.03 eq., 51.5 mg, 0.19 mmol), the crude was purified by flash column chromatography using methanol in DCM as solvent (0 to 10%) The expected 2-fluoro-N-(isoquinolin-8-yl)-4-[4-(propan-2-yl)piperazin-1-yl]benzamide **(57)** (35 mg, 0.089 mmol, 51.73%) was obtained as a pink powder and with an HPLC purity of 99%.

**NMR ¹H (500 MHz, MeOD)** : δ 9.39 (s, 1H), 8.47 (d, *J* = 5.8 Hz, 1H), 7.92 (dd, *J* = 7.2, 1.4 Hz, 1H), 7.88 - 7.79 (m, 4H), 6.89 (dd, *J* = 8.9, 2.4 Hz, 1H), 6.81 (dd, *J* = 15.6, 2.4 Hz, 1H), 3.49 - 3.37 (m, 4H), 2.85 - 2.61 (m, 5, CH + 2CH₂), 1.14 (s, 3H), 1.13 (s, 3H).

**NMR ¹³C (126 MHz, MeOD)** : δ 166.23 (CO), 163.51 (d, *J* = 246.5 Hz, C-F), 156.72 (d, *J* = 11.4 Hz, C_{q}), 148.85 (CH_{Ar}), 142.95 (CH_{Ar}), 138.34 (C_{q}), 135.76 (C_{q}), 133.21 (d, *J* = 4.2 Hz, CH_{Ar}), 132.29 (CH_{Ar}), 126.02 (CH_{Ar}), 125.53 (CH_{Ar}), 125.18 (C_{q}), 122.49 (CH_{Ar}), 111.86 (d, *J* = 13.0 Hz, C_{q}), 111.28 (d, *J* = 2.0 Hz, CH_{Ar}), 101.86 (d, *J* = 28.5 Hz, CH_{Ar}), 56.00 (CH), 49.58 (2CH₂), 48.18 (2CH₂), 18.65 (2CH₃).

**NMR ¹⁹F (471 MHz, MeOD):** δ -112.49.

**HRMS (ESI):** m/z calculated for C23H26FN4O+, [M+H]+ 393.2085, found 393.2098.

### Pharmacological data

The compounds of the invention have been the subject of pharmacological tests which have demonstrated their relevance as active substances in therapy and in particular for treating pain. They have been evaluated for their capacity to inhibit the binding of FL to FLT3 (Example 10), since the relationship between this capacity and the ability to treat pain has been established (WO2016/016370).

### Example 22: Binding assay

### A. Principle

The binding of FL to FLT3 was measured by a Homologous Time-Resolved Fluorescence (HTRF) assay (Degorce et al., Current Chemical Genomics, 2009, 3, 22-32), which involves resonance energy transfer between a donor, Lumi4-Tb-donor-derivatized benzylguanine substrate (SNAP-Lumi4-Tb, excitation: 337 nm, emission: 630 nm, Cisbio, France), transfected in HEK cells, and an acceptor, red fluorescent FL (FL-d2, excitation: 620 nm, emission: 660 nm, Cisbio, France). When FL-d2 binds to the SNAP-Lumi4-Tb-tagged FLT3 receptor, the proximity of both fluorophores allows the time-resolved Förster resonance energy transfer (TR-FRET) signal to occur and the binding is measured as the ratio between the fluorescent emissions at 660 to 620 nm.

### B. Material & Methods

The detailed method is described in Rivat et al. (Nature Communications, 2018, 9 :1042). Briefly, HEK293T cells were transfected with a pCDNA3.1 SNAP-FLT3 plasmid by using Lipofectamine (Invitrogen), and were than labelled with SNAP-Lumi4-Tb. Plates containing attached transfected HEK293T cells were incubated with compounds of formula (I) for 1 h at room temperature. A series of 12 dilutions in triplicates was used for each compound and the experiment was performed twice. Then FL-d2 (0.5 nM) was added and the TR-FRET signal was read after 20 h incubation at room temperature with a HTRF-compatible reader (Envision, Perkin Elmer) allowing a donor excitation at 337 nm and a signal collection both at 615 nm and 665 nm (20 flashes, delay 50 µs, integration time 400 µs). Then HTRF ratio, obtained by dividing the acceptor signal (665 nm) by the donor signal (615 nm), was calculated at each concentration of test compounds. Non-specific binding was measured in the presence 50 µM of BDT001 (Rivat et al., *ibid*.)*.* Concentration giving 50 % inhibition (IC₅₀) were calculated by non-linear regression analysis for a one-site model, with variable slope (Prism^{®}, Graphpad software, USA).

### C. Results

All tested compounds inhibited FL binding to FLT3, yet with variable potency **(Table III).** Thus, experimental evidence shows that the compounds of formula (I) according to the invention can be used to treat pain disorders.

**Table III**

| **Compound number** | **Inhibition of FLT3 binding IC50, binding (µM)^{a}** |
|---|---|
| (1) | 1.2 |
| (2) | 1.4 |
| (3) | 1.8 |
| (4) | 5.3 |
| (5) | 0.4 |
| (6) | 1.3 |
| (7) | 0.8 |
| (8) | 0.8 |
| (9) | 1.5 |
| (10) | 0.8 |
| (11) | 2.9 |
| (12) | 1.8 |
| (13) | 1.4 |
| (14) | 0.9 |
| (15) | 1.7 |
| (16) | 0.6 |
| (17) | 0.9 |
| (18) | 1.7 |
| (19) | 25.0 |
| (20) | 6.2 |
| (21) | 2.0 |
| (22) | 1.9 |
| (23) | 0.5 |
| (24) | 1.1 |
| (25) | 2.6 |
| (26) | 1.4 |
| (27) | 3.6 |
| (28) | 0.4 |
| (29) | 1.2 |
| (30) | 1.9 |
| (31) | 0.7 |
| (32) | 2.4 |
| (33) | 2.3 |
| (34) | 0.5 |
| (35) | 1.7 |
| (36) | 2.5 |
| (37) | 0.7 |
| (38) | 0.6 |
| (39) | 0.8 |
| (40) | 1.7 |
| (41) | 0.6 |
| (42) | 3.1 |
| (43) | 2.1 |
| (44) | 3.8 |
| (45) | 1.0 |
| (46) | 1.0 |
| (47) | 1.7 |
| (48) | 1.3 |
| (49) | 2.4 |
| (50) | 2.3 |
| (51) | 1.3 |
| (52) | 7.5 |

## Claims

1. A compound of formula (I) or any of its acceptable salts (I) 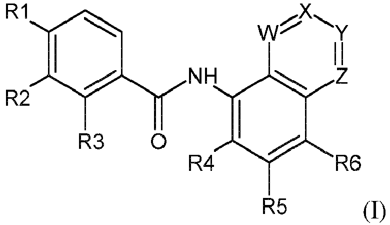 wherein
W, X, Y and Z each independently represents =CH- or -N-, provided that at most two of W, X, Y and Z both represent a -N- group,
R1 represents a linear or cyclic nitrogen containing (C₄-C₈)alkyl group, said group being a non-aromatic (C₄-C₈)alkyl group that can be linear or cyclic comprising 4 to 8 carbon atoms that contains at least one nitrogen atom, that interrupts said alkyl chain, said group being optionally interrupted by one or two oxygen atoms and optionally substituted by a (C₁-C₄)alkyl group or by a (C3-C₆)cycloalkyl group, wherein R1 comprises at least one primary, secondary or tertiary amine, in particular one or two secondary or tertiary amine,
R2 represents a hydrogen atom, a halogen atom, a (C₁-C₄)alkyl group, a (C₁-C₄)alkoxy group, a (C₁-C₄)fluoroalkyl group, a -CO-(C₁-C₄)alkyl group, a -CONH2 group, a SO₂-NH₂ group or a cyano group,
R3 represents a hydrogen atom, a halogen atom, a (C₁-C₄)alkyl group, a (C₁-C₄)alkoxy group, a (C₁-C₄)fluoroalkyl group, a cyano group, a (C₁-C₄)alkylsulfonyl group or a SO₂-NH₂ group, and
R4, R5 and R6 independently represent a hydrogen atom, a halogen atom, a -COOH group, a (C₁-C₄)fluoroalkyl group, a (C₁-C₄)alkylsulfonyl group or a (C₁-C₄)alkoxy group.

2. The compound of formula (I) according to claim 1, wherein W is a - N= and X, Y and Z are =CH- or Y is a -N= and W, X and Z are =CH- or X is a -N= and W, Y and Z are =CH- or Z is a -N= and W, X and Y are =CH- or W and Z are - N= and X and Y are =CH- or X and Y are =-N and W and Z are =CH-.

3. The compound of formula (I) according to claim 1 or 2, wherein R1 is selected from, 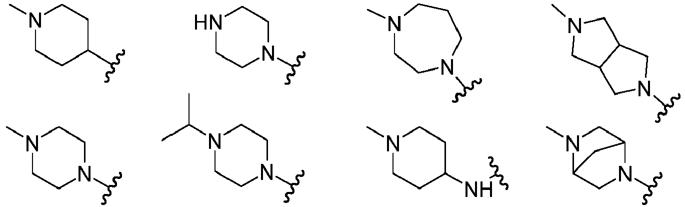 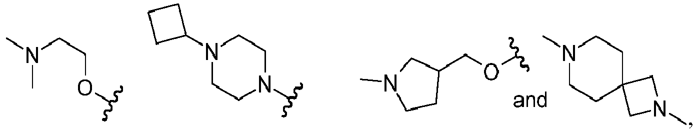 and is particular selected from 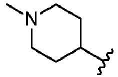 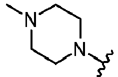 and

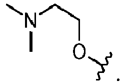

4. The compound of formula (I) according to any of claims 1 to 3, wherein R2 represents a hydrogen atom, a halogen atom, a (C₁-C₄)alkyl group, a (C₁-C₄)alkoxy group, a (C₁-C₄)fluoroalkyl group or a cyano group, and in particular a hydrogen atom, a fluorine, a methyl group, a methoxy group, a trifluoro methyl group or a cyano group.

5. The compound of formula (I) according to any of claims 1 to 4, wherein R3 represents a hydrogen atom, a halogen atom, a (C₁-C₄)alkyl group, a (C₁-C₄)alkoxy group, a (C₁-C₄)fluoroalkyl group or a (C₁-C₄)alkylsulfonyl group, and in particular a hydrogen atom, a fluorine, a methyl group, a methoxy group, a trifluoromethyl group or a SO₂-CH₃ group.

6. The compound of formula (I) according to any of claims 1 to 5, wherein R4, R5 and R6 independently represent a hydrogen atom, a halogen atom, a (C₁-C₄)fluoroalkyl group or a (C₁-C₄)alkoxy group, and in particular a hydrogen atom, a fluorine or chlorine atom, a trifluoromethyl group or a methoxy group.

7. The compound of formula (I) according to any of claims 1 to 3, wherein
R2 represents a hydrogen atom, a halogen atom, a (C₁-C₄)alkyl group, a (C₁-C₄)alkoxy group, a (C₁-C₄)fluoroalkyl group, a -CO-(C₁-C₄)alkyl group or a cyano group, in particular a hydrogen atom, a halogen atom, a (C₁-C₄)alkyl group, a (C₁-C₄)alkoxy group, a (C₁-C₄)fluoroalkyl group or a cyano group, R3 represents a hydrogen atom, a halogen atom, a (C₁-C₄)alkyl group, a (C₁-C₄)alkoxy group, a (C₁-C₄)fluoroalkyl group, a cyano group or a (C₁-C₄)alkylsulfonyl group, in particular a hydrogen atom, a halogen atom, a (C₁-C₄)alkyl group, a (C₁-C₄)alkoxy group, a (C₁-C₄)fluoroalkyl group or a (C₁-C₄)alkylsulfonyl group, and
R4, R5 and R6 independently represent a hydrogen atom, a halogen atom, a (C₁-C₄)fluoroalkyl group, a -COOH group, a (C₁-C₄)alkylsulfonyl group or a (C₁-C₄)alkoxy group, in particular a hydrogen atom, a halogen atom, a (C₁-C₄)fluoroalkyl group or a (C₁-C₄)alkoxy group.

8. The compound of formula (I) according to any of claims 1 to 7 or a pharmaceutically acceptable salt thereof, in particular hydrochloride salt thereof, **characterized in that** said compound is selected from the following compounds:
- (1) N-(5-fluoroquinolin-8-yl)-4-(1-methylpiperidin-4-yl)benzamide,
- (2) N-(8-fluoroisoquinolin-5-yl)-4-(1-methylpiperidin-4-yl)benzamide,
- (3) N-(5-fluoroisoquinolin-8-yl)-4-(1-methylpiperidin-4-yl)benzamide,
- (4) N-(8-fluoroquinolin-5-yl)-4-(1-methylpiperidin-4-yl)benzamide,
- (5) N-(8-fluoroquinoxalin-5-yl)-4-(1-methylpiperidin-4-yl)benzamide,
- (6) 4-(1-methylpiperidin-4-yl)-N-(quinolin-5-yl)benzamide,
- (7) N-(isoquinolin-5-yl)-4-(1-methylpiperidin-4-yl)benzamide,
- (8) N-(isoquinolin-8-yl)-4-(1-methylpiperidin-4-yl)benzamide,
- (9) 4-(1-methylpiperidin-4-yl)-N-(quinolin-8-yl)benzamide,
- (10) 4-[2-(dimethylamino)ethoxy]-N-(isoquinolin-8-yl)benzamide,
- (11) 4-[2-(dimethylamino)ethoxy]-N-(quinolin-8-yl)benzamide,
- (12) 2-fluoro-4-(1-methylpiperidin-4-yl)-N-(quinolin-8-yl)benzamide,
- (13) 3-fluoro-N-(isoquinolin-5-yl)-4-(1-methylpiperidin-4-yl)benzamide,
- (14) 4-(2-(dimethylamino)ethoxy)-N-(isoquinolin-5-yl)benzamide,
- (15) 4-(2-(dimethylamino)ethoxy)-N-(quinolin-5-yl)benzamide,
- (16) 2-methoxy-4-(1-methylpiperidin-4-yl)-N-(quinolin-8-yl)benzamide,
- (17) N-(isoquinolin-5-yl)-3-methoxy-4-(1-methylpiperidin-4-yl)benzamide,
- (18) 4-(1-methylpiperidin-4-yl)-N-(quinolin-8-yl)-2-(trifluoromethyl)benzamide,
- (19) 4-[2-(dimethylamino)ethoxy]-N-(phthalazin-5-yl)benzamide,
- (20) 4-(2-(dimethylamino)ethoxy)-N-(8-fluoroquinolin-5-yl)benzamide,
- (21) 3-cyano-N-(isoquinolin-5-yl)-4-(1-methylpiperidin-4-yl)benzamide,
- (22) 4-(2-(dimethylamino)ethoxy)-N-(5-fluoroisoquinolin-8-yl)benzamide,
- (23) 4-(2-(dimethylamino)ethoxy)-N-(8-fluoroquinoxalin-5-yl)benzamide,
- (24) 3-fluoro-N-(isoquinolin-5-yl)-4-(4-methylpiperazin-1-yl)benzamide,
- (25) N-(isoquinolin-5-yl)-2-methanesulfonyl-4-(1-methylpiperidin-4-yl)benzamide,
- (26) 4-[2-(dimethylamino)ethoxy]-N-(5-fluoroquinolin-8-yl)benzamide,
- (27) 4-(1-methylpiperidin-4-yl)-N-(quinolin-5-yl)-3-(trifluoromethyl)benzamide,
- (28) 4-(1-methylpiperidin-4-yl)-N-(quinoxalin-5-yl)benzamide,
- (29) 3-methyl-4-(4-methylpiperazin-1-yl)-N-(quinolin-5-yl)benzamide,
- (30) N-(8-chloroquinolin-5-yl)-4-(1-methylpiperidin-4-yl)benzamide,
- (31) 4-[2-(dimethylamino)ethoxy]-N-(quinoxalin-5-yl)benzamide,
- (32) N-(8-chloroquinolin-5-yl)-4-[2-(dimethylamino)ethoxy]benzamide,
- (33) 4-[2-(dimethylamino)ethoxy]-N-(8-fluoroisoquinolin-5-yl)benzamide,
- (34) 4-[2-(dimethylamino)ethoxy]-N-(7-methoxyisoquinolin-5-yl)benzamide,
- (35) 4-(4-methylpiperazin-1-yl)-N-(quinolin-8-yl)benzamide,
- (36) 3-cyano-4-(2-(dimethylamino)ethoxy)-N-(isoquinolin-8-yl)benzamide,
- (37) N-(8-fluoroquinoxalin-5-yl)-4-(4-methylpiperazin-1-yl)benzamide,
- (38) N-(isoquinolin-8-yl)-4-(4-methylpiperazin-1-yl)benzamide,
- (39) N-(isoquinolin-5-yl)-4-(4-methylpiperazin-1-yl)benzamide,
- (40) 4-(4-methylpiperazin-1-yl)-N-(quinolin-5-yl)benzamide,
- (41) 4-(4-methylpiperazin-1-yl)-N-(quinoxalin-5-yl)benzamide,
- (42) N-(5-fluoroquinolin-8-yl)-4-(4-methylpiperazin-1-yl)benzamide,
- (43) 4-(2-(dimethylamino)ethoxy)-3-methyl-N-(quinolin-8-yl)benzamide,
- (44) N-(8-fluoroquinolin-5-yl)-4-(4-methylpiperazin-1-yl)benzamide,
- (45) N-(isoquinolin-5-yl)-2-methyl-4-(4-methylpiperazin-1-yl)benzamide,
- (46) N-(8-chloroquinolin-5-yl)-4-(4-methylpiperazin-1-yl)benzamide,
- (47) 4-(2-(dimethylamino)ethoxy)-2-methyl-N-(quinolin-5-yl)benzamide,
- (48) 4-(2-(dimethylamino)ethoxy)-N-(isoquinolin-5-yl)-3-(trifluoromethyl)benzamide,
- (49) 4-(2-(dimethylamino)ethoxy)-N-(quinolin-5-yl)-2-(trifluoromethyl)benzamide,
- (50) 3-methoxy-4-(4-methylpiperazin-1-yl)-N-(quinolin-5-yl)benzamide,
- (51) N-(8-chloroisoquinolin-5-yl)-4-(4-methylpiperazin-1-yl)benzamide hydrochloride,
- (52) 4-(4-methylpiperazin-1-yl)-N-[5-(trifluoromethyl)isoquinolin-8-yl]benzamide hydrochloride,
- (53) 3-fluoro-N-(5-fluoroisoquinolin-8-yl)-4-{5-methyl-octahydropyrrolo[3,4-c]pyrrol-2-yl}benzamide,
- (54) N-(8-methoxyisoquinolin-5-yl)-3-methyl-4-{7-methyl-2,7-diazaspiro[3.5]nonan-2-yl}benzamide, and
- (55) 3-methyl-4-[(1-methylpyrrolidin-3-yl)methoxyl-N-(quinolin-8-yl)benzamide,
- (56) 2-fluoro-4-(4-methylpiperazin-1-yl)-N-(quinolin-5-yl)benzamide, and
- (57) 2-fluoro-N-(isoquinolin-8-yl)-4-[4-(propan-2-yl)piperazin-1-yl]benzamide.

9. A process for preparing a compound of formula (I) as defined in anyone of claims 1 to 8 or pharmaceutically acceptable salts thereof, comprising at least the following step:
- reacting a compound of formula (II): 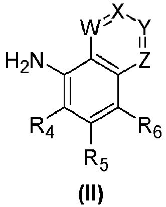 wherein R4, R5, R6, W, X, Y and Z are as defined in any of claims 1 to 7,
with a compound of formula (VII): 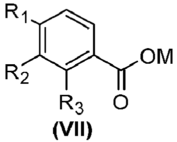 wherein R1, R2, R3 are as defined in any of claims 1 to 7 and M represents an alkali metal or a hydrogen, in order to obtain a compound of formula (I),
(i) by a peptide synthesis using a carboxyl activating agent, in particular N-(3-Dimethylaminopropyl)-N'-ethylcarbodiimide (EDCI) or N,N'-Diisopropylcarbodiimide (DIC) with a racemization suppressor agent, in particular 1-Hydroxybenzotriazole (HOBt) or ethyl cyanohydroxyiminoacetate, and with an organic base in an aprotic polar solvent; or
(ii) first, by treating a compound of formula (VII) separately in presence of a chlorinating agent, in particular SOCl₂, and optionally an organic base in an aprotic solvent in order to form the corresponding acyl chloride derivative and then, by reacting said acyl chloride derivative with a compound of formula (II) by nucleophilic substitution using an organic base in an aprotic solvent.

10. A pharmaceutical composition comprising at least one compound as defined in anyone of claims 1 to 8 or anyone of its pharmaceutically acceptable salts, and also at least one pharmaceutically acceptable excipient.

11. A pharmaceutical composition comprising at least one compound as defined in anyone of claims 1 to 8 or anyone of its pharmaceutically acceptable salts, an opioid, and also at least one pharmaceutically acceptable excipient, wherein said opioid may be selected among alfentanil, allylprodine, alphaprodine, anileridine, benzylmorphine, bezitramide, buprenorphine, butorphanol, clonitazene, codeine, cyclazocine, desomorphine, dextromoramide, dextropropoxyphene, dezocine,diampromide, diamorphone, dihydrocodeine, dihydromorphine, dimenoxadol, dimepheptanol, dimethylthiambutene, dioxaphetylbuturate, dipipanone, eptazocine, ethoheptazine, ethylmethylthiambutene. ethylmorphine, etonitazene fentanyl, heroin, hydrocodone, hydromorphone, hydroxypethideine, isomethadone, ketobemidone, levallorphan, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propiram, propoxyphene, remifentanyl, sufentonil, tapentadol, tilidine and tramadol.

12. A compound according to anyone of claims 1 to 8, alone or in combination with an opioid, in particular as defined in claim 11, for use as a medicament.

13. A compound according to anyone of claims 1 to 8, alone or in combination with an opioid, in particular as defined in claim 11, for use in the prevention and/or treatment of pain.
